# EUROPEAN PATENT SPECIFICATION

(11) **EP 2 903 619 B1**
(45) Date of publication and mention of the grant of the patent: **05.06.2019**
(21) Application number: 13843665.4
(22) Date of filing: 03.10.2013
(51) Int. Cl.: A61K 31/522, A61P 27/00, A61K 9/00, A61K 45/06, A61K 31/4015, A61K 31/404, A61K 31/437, A61K 31/4709, A61K 31/53, A61K 31/352, A61K 31/4375, A61K 31/44, A61K 31/4985, A61K 31/506, A61K 31/519, A61K 31/52

(54) **PHOSPHODIESTERASE INHIBITORS FOR TREATING TASTE AND SMELL DISORDERS**
PHOSPHODIESTERASEHEMMER ZUR BEHANDLUNG VON GESCHMACKS- UND GERUCHSSTÖRUNGEN
INHIBITEURS DE LA PHOSPHODIESTÉRASE DESTINÉS À TRAITER DES TROUBLES DU GOÛT ET DE L'ODORAT

(30) Priority: 05.10.2012 US 201261710674 P; 15.03.2013 US 201361802074 P
(43) Date of publication of application: 12.08.2015
(73) Proprietor: Henkin, Robert I., Bethesda, MD 20817 (US)
(72) Inventor: Henkin, Robert I., Bethesda, MD 20817 (US)
(74) Representative: Avidity IP
(86) International application number: PCT/US2013/063331
(87) International publication number: WO 2014/055801

(56) References cited:
- EP-A1- 0 967 214
- WO-A1-01/82931
- WO-A1-2006/085102
- WO-A1-2010/147981
- WO-A1-2010/147981
- WO-A2-2007/044375
- US-A- 5 169 849
- US-A1- 2010 022 563
- US-A1- 2010 022 563
- US-A1- 2011 023 870
- H KUBLIK ET AL: "Nasal delivery systems and their effect on deposition and absorption", ADVANCED DRUG DELIVERY REVIEWS, vol. 29, no. 1-2, 1 January 1998 (1998-01-01), pages 157-177, XP055248105, AMSTERDAM, NL ISSN: 0169-409X, DOI: 10.1016/S0169-409X(97)00067-7
- R. I. Henkin Et Al.: "Intranasal Theophylline Treatment of Hyposmia and Hypogeusia A Pilot Study", Arch Otolaryngol Head Neck Surg., 1 November 2012 (2012-11-01), pages 1064-1070, XP055247582, Retrieved from the Internet: URL:http://archotol.jamanetwork.com/articl e.aspx?articleid=1392510 [retrieved on 2016-02-04]

## Description

The present invention relates to a multi-dose nasal spray device for delivery of theophylline to a subject's nasal epithelium for use in the treatment of a taste or smell disorder, that delivers a dosage unit in a plume upon actuation, as defined in the claims.

### BACKGROUND OF THE INVENTION

Phosphodiesterases (PDE) are a diverse family of enzymes that hydrolyze cyclic nucleotides resulting in the modulation of intracellular levels of the second messengers cAMP and cGMP, and hence, cell function. Numerous diseases and conditions result from low levels of cyclic nucleotides. The use of PDE inhibitors to raise cellular levels of cyclic nucleotides offers the ability to prevent, treat, or ameliorate diseases, conditions or their symptoms, however, systemic administration may not achieve therapeutically effective concentrations due to unacceptable side effects or to inability to obtain therapeutic levels in clinically responsive tissues. US5,169,849 discloses a nasal liquid composition containing dihydroergotamine and xanthine, which increases the ciliary function. WO0182931 discloses a nasal composition containing (+) naloxone and an adrenergic agonist e.g. theophylline. US2010022563 discloses compositions comprising phosphodiesterase inhibitors formulated for intranasal and pulmonary administration.
There is a need for suitable compositions and methods of delivery to achieve medically relevant concentrations of PDE inhibitors without unacceptable side effects. The present invention addresses these unmet needs.

### SUMMARY

1. The invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human or animal body by therapy.Disclosed herein is a multi-dose nasal spray device for delivery of theophylline to a subject's nasal epithelium for use in the treatment of a taste or smell disorder in said subject, that delivers a dosage unit in a plume upon actuation, wherein the dosage unit comprises from 10 µg to 200 µg of theophylline in a pharmaceutically acceptable carrier comprising one or more excipients; and wherein the plume is characterized by:
   (a) a total volume of from 25 µL to 200 µL; and
   (b) a droplet size distribution characterized by:
      (i) less than 5% of the droplets in the plume having a size of less than 10 µm,
      (ii) a D₁₀ of greater than 12.5 µm, wherein 10% of the droplets in the plume have a size less than the D₁₀,
      (iii) a D₅₀ of from 30 to 70 µm, wherein 50% of the droplets in the plume have a size less than the D₅₀,
      (iv) a D₉₀ of less than 200 µm, wherein 90% of the droplets in the plume have a size less than the D₉₀, and
      (v) a span of from 1 to 6, wherein the span is calculated according to: (D₉₀ - D₁₀)/D₅₀.

In some cases, the dosage unit comprises from 20 µg to 100 µg of theophylline. In some cases, the dosage unit comprises about 100 µg theophylline. In some cases, the dosage unit comprises about 75 µg theophylline. In some cases, the dosage unit comprises about 50 µg of theophylline. In some cases, the dosage unit comprises about 20 µg of theophylline.

Also disclosed are multi-dose nasal spray devices for delivery of theophylline to a human's nasal epithelium that delivers a dosage unit in a plume upon actuation, wherein the dosage unit comprises, for example, from 0.17 µg/kg to 3.4 µg/kg of theophylline in a pharmaceutically acceptable carrier comprising one or more excipients; and wherein the plume is characterized by: (a) a total volume, for example, of from 25 µL to 200 µL; and (b) a droplet size distribution characterized by one or more of the following: (i) less than 5% of the droplets in the plume having a size of less than 10 µm, (ii) a D₁₀ of greater than 12.5 µm, wherein 10% of the droplets in the plume have a size less than the D₁₀, (iii) a D₅₀ of from 30 to 70 µm, wherein 50% of the droplets in the plume have a size less than the D₅₀, (iv) a D₉₀ of less than 200 µm, wherein 90% of the droplets in the plume have a size less than the D₉₀, and (v) a span of from 1 to 6, wherein the span is calculated according to: (D₉₀ - D₁₀)/D₅₀.

In some cases, the dosage unit comprises from 0.25 µg/kg to 2.6 µg/kg of theophylline. In some cases, the dosage unit comprises from 0.33 µg/kg to 1.7 µg/kg of theophylline. In some cases, the dosage unit comprises about 1.7 µg/kg theophylline. In some cases, the dosage unit comprises about 1.3 µg/kg theophylline. In some cases, the dosage unit comprises about 0.85 µg/kg of theophylline. In some cases, the dosage unit comprises about 0.33 µg/kg of theophylline.

In some cases, the dosage unit is a steroid-free dosage unit.

In some cases, the pharmaceutically acceptable carrier has a pH that is greater than 7.0. In some cases, the pharmaceutically acceptable carrier has a pH that is from 7.1 to 8.5. In some cases, the pharmaceutically acceptable carrier has a pH that is from 7.1 to 7.4. In some cases, the pharmaceutically acceptable carrier has a pH that is about: 7.1, 7.2, 7.3, 7.4, 7.5, 7.6, 7.7, 7.8, 7.9, 8, 8.1, 8.2, 8.3, 8.4, or 8.5. In some cases, the pharmaceutically acceptable carrier has a pH that is at least 7.1, 7.2, 7.3, 7.4, 7.5, 7.6, 7.7, 7.8, 7.9, 8, 8.1, 8.2, 8.3, 8.4, or 8.5.

In some cases, the plume is characterized by the total volume of the plume that is from 50 µL to 150 µL. In some cases, the plume is characterized by the total volume of the plume that is from 75 µL to 125 µL. In some cases, the plume is characterized by the total volume of the plume that is from 90 µL to 110 µL. In some cases, the plume is characterized by the total volume of the plume that is about: 25 µL, 30 µL, 35 µL, 40 µL, 45 µL, 50 µL, 55 µL, 60 µL, 65 µL, 70 µL, 75 µL, 80 µL, 85 µL, 90 µL, 100 µL, 110 µL, 120 µL, 130 µL, 140 µL, 150 µL, 160 µL, 170 µL, 180 µL, 190 µL, or 200 µL. In some cases, the plume is characterized by the total volume of the plume that is about 50 µL. In some cases, the plume is characterized by the total volume of the plume that is about 100 µL. In some cases, the plume is characterized by the total volume of the plume that is about 140 µL.

In some cases, the plume is characterized by less than 4% of the droplets in the plume having a size of less than 10 µm. In some cases, the plume is characterized by less than 3% of the droplets in the plume having a size of less than 10 µm. In some cases, the plume is characterized by less than 2% of the droplets in the plume having a size of less than 10 µm. In some cases, the plume is characterized by the D₁₀ that is greater than 15 µm. In some cases, the plume is characterized by the D₁₀ that is greater than 17.5 µm. In some cases, the plume is characterized by the D₁₀ that is from 12.5 µm to 30 µm. In some cases, the plume is characterized by the D₁₀ that is from 15 µm to 25 µm.

In some cases, the plume is characterized by the D₅₀ that is from 40 µm to 60 µm. In some cases, the plume is characterized by the D₅₀ that is from 30 µm to 60 µm. In some cases, the plume is characterized by the D₅₀ that is from 30 µm to 50 µm. In some cases, the plume is characterized by the D₅₀ that is from 30 µm to 40 µm. In some cases, the plume is characterized by the D₅₀ that is about: 30 µm, 32.5 µm, 35 µm, 37.5 µm, 40 µm, 42.5 µm, 45 µm, 50 µm, 55 µm, 60 µm, 65 µm, or 70 µm.

In some cases, the plume is characterized by the D₉₀ that is less than 175 µm. In some cases, the plume is characterized by the D₉₀ that is less than 150 µm. In some cases, the plume is characterized by the D₉₀ that is less than 125 µm. In some cases, the plume is characterized by the D₉₀ that is less than 100 µm. In some cases, the plume is characterized by the D₉₀ that is less than 90 µm. In some cases, the plume is characterized by the D₉₀ that is from 75 µm to 199 µm. In some cases, the plume is characterized by the D₉₀ that is from 75 µm to 175 µm. In some cases, the plume is characterized by the D₉₀ that is from 75 µm to 150 µm. In some cases, the plume is characterized by the D₉₀ that is from 75 µm to 125 µm. In some cases, the plume is characterized by the D₉₀ that is from 75 µm to 100 µm. In some cases, the plume is characterized by the D₉₀ that is about: 75 µm, 80 µm, 85 µm, 90 µm, 100 µm, 110 µm, 120 µm, 130 µm, 140 µm, 150 µm, 160 µm, 170 µm, 180 µm, or 190µm.

In some cases, the plume is characterized by the span that is from 1 to 5. In some cases, the plume is characterized by the span that is from 1 to 4. In some cases, the plume is characterized by the span that is from 1 to 3. In some cases, the plume is characterized by the span that is from 1 to 2. In some cases, the plume is characterized by the span that is about: 1, 1.1, 1.2, 1.3, 1.4, 1.5, 1.6, 1.7, 1.8, 1.9, 2, 2.1, 2.2, 2.3, 2.4, 2.5, 2.6, 2.7, 2.7, 2.9, 3, 3.25, 3.5, 3.75, 4, 4.5, 5, 5.5, or 6.

In some cases, the plume is further characterized by having an ovality of, for example, from 0.7 to 1. In some cases, the plume is further characterized by having an ovality of from 0.8 to 1. In some cases, the plume is further characterized by having an ovality of from 0.9 to 1. In some cases, the plume is further characterized by having an ovality of about 1. In some cases, the plume is further characterized by having a geometry of from 30° to 90°. In some cases, the plume is further characterized by having a geometry of from 45° to 75°. In some cases, the plume is further characterized by having a geometry of about: 30°, 35°, 40°, 45°, 50°, 55°, 60°, 65°, 70°, 75°, 80°, 85°, or 90°.

In some cases, the pharmaceutically acceptable carrier comprises water, aminoboronic acid and its derivatives, amastatin, surfactants, bile salts, gelified insulin, bioadhesive microspheres, phospholipids, chitosan nanoparticles, alkyl glycerides, or a combination thereof. In some cases, the pharmaceutically acceptable carrier comprises water.

In some cases, the one or more excipients comprise a buffering agent, a flavoring agent, a humectant, a penetration enhancer, a pH adjusting agent, a preservative, a solvent or co-solvent, a surfactant, a tonicity adjusting agent, a viscosity adjusting agent, or a combination thereof. Some cases comprise the buffering agent that is potassium phosphate, sodium acetate, sodium citrate, sodium phosphate, trisodium citrate, or a combination thereof. Some cases comprise the flavoring agent that is menthol, saccharin sodium, sorbitol, or a combination thereof. Some cases comprise the humectant that is glycerin, propylene glycol, hexylene glycol, butylenes glycol, glyceryl triacetate, vinyl alcohol, neoagarobiose, glycerol, sorbitol, xylitol, maltitol, polydextrose, quillaia, lactic acid, urea, or aloe vera. Some cases comprise the humectant that is propylene glycol. Some cases comprise the penetration enhancer that is oleic acid. Some cases comprise the pH adjusting agent that is acetic acid, citric acid, hydrochloric acid, sodium hydroxide, sulfuric acid, or a combination thereof. Some cases comprise the preservative that is benzalkonium chloride, benzethonium chloride, benzyl alcohol, butylated hydroxy toluene, butylated hydroxyanisole, chlorobutanol, edetate disodium, methylparaben, phenylethyl alcohol, phenylmercuric acetate, propylene paraben, propylparaben, thimerosal, or a combination thereof. Some cases comprise the preservative that is methylparaben, propylparaben, or a combination thereof. Some cases comprise the solvent or co-solvent that is ethanol, glycerol, glyceryl dioleate, glycine, polyethylene glycol (PEG), PEG 400, propylene glycol, triglycerides, or a combination thereof. Some cases comprise the solvent or co-solvent that is propylene glycol. Some cases comprise the surfactant that is glyceryl monoleate, lecithin, PEG 3500, PEG 400, polyoxyl 400 stearate, polysorbate 20, polysorbate 80, propylene glycol, triglycerides, or a combination thereof. Some cases comprise the tonicity adjusting agent that is dextrose, potassium chloride, sodium chloride, or a combination thereof. Some cases comprise the tonicity adjusting agent that is sodium chloride. Some cases comprise the viscosity adjusting agent that is carboxymethyl cellulose (CMC), Me-OH-Pr cellulose, microcrystalline cellulose (MCC), sodium carboxymethyl cellulose (Na CMC), or a combination thereof.

In some cases, the one or more excipients comprise from about 0.01% to about 0.5% w/w acetic acid. In some cases, the one or more excipients comprise from about 0.001% to about 0.119% w/w benzalkonium chloride. In some cases, the one or more excipients comprise from about 0.001% to about 0.0366% w/w benzyl alcohol. In some cases, the one or more excipients comprise from about 0.001% to about 0.01% w/w butylated hydroxy toluene. In some cases, the one or more excipients comprise from about 0.00001% to about 0.0002% w/w butylated hydroxyanisole. In some cases, the one or more excipients comprise from about 0.1% to about 2% w/w carboxymethyl cellulose. In some cases, the one or more excipients comprise from about 0.01% to about 0.5% w/w chlorobutanol. In some cases, the one or more excipients comprise from about 0.01% to about 0.5% w/w citric acid. In some cases, the one or more excipients comprise from about 0.01% to about 0.5% w/w dextrose. In some cases, the one or more excipients comprise from about 0.01% to about 0.5% w/w edetate disodium. In some cases, the one or more excipients comprise from about 0.1% to about 2% w/w ethanol. In some cases, the one or more excipients comprise from about 0.01% to about 0.233% w/w glycerin. In some cases, the one or more excipients comprise from about 0.1% to about 5% w/w glycerol. In some cases, the one or more excipients comprise from about 1% to about 15% w/w glyceryl dioleate. In some cases, the one or more excipients comprise from about 1% to about 10% w/w glyceryl monoleate. In some cases, the one or more excipients comprise from about 0.1% to about 10% w/w lecithin. In some cases, the one or more excipients comprise from about 0.1% to about 5% w/w Me-OH-Pr cellulose. In some cases, the one or more excipients comprise from about 0.001% to about 0.7% w/w methylparaben. In some cases, the one or more excipients comprise from about 0.1% to about 2% w/w microcrystalline cellulose. In some cases, the one or more excipients comprise from about 0.01% to about 0.132% w/w oleic acid. In some cases, the one or more excipients comprise from about 0.1% to about 1.5% w/w PEG 3500. In some cases, the one or more excipients comprise from about 0.1% to about 20% w/w PEG 400. In some cases, the one or more excipients comprise from about 0.01% to about 0.254% w/w phenylethyl alcohol. In some cases, the one or more excipients comprise from about 0.1% to about 5% w/w polyethylene glycol (PEG). In some cases, the one or more excipients comprise from about 0.1% to about 15% w/w polyoxyl 400 stearate. In some cases, the one or more excipients comprise from about 0.01% to about 2.5% w/w polysorbate 20. In some cases, the one or more excipients comprise from about 0.1% to about 10% w/w polysorbate 80. In some cases, the one or more excipients comprise from about 0.1% to about 1.9% w/w potassium chloride. In some cases, the one or more excipients comprise from about 0.1% to about 20% w/w propylene glycol. In some cases, the one or more excipients comprise from about 0.001% to about 0.1% w/w propylene paraben. In some cases, the one or more excipients comprise from about 0.01% to about 0.3% w/w propylparaben. In some cases, the one or more excipients comprise from about 0.1% to about 5% w/w sodium carboxymethyl cellulose (Na CMC). In some cases, the one or more excipients comprise from about 0.1% to about 1.9% w/w sodium chloride. In some cases, the one or more excipients comprise from about 0.5% to about 10% w/w sorbitol. In some cases, the one or more excipients comprise from about 0.01% to about 0.4% w/w sulfuric acid. In some cases, the one or more excipients comprise from about 0.1% to about 5% w/w triglycerides. In some cases, the one or more excipients comprise from about 0.00001% to about 0.0006% w/w trisodium citrate.

Also disclosed are methods of treating anosmia, hyposmia, dysosmia, ageusia, hypogeusia, or dysgeusia in a subject in need thereof comprising intranasal administration of theophylline with any of the multi-dose nasal spray devices disclosed herein. In some cases, the theophylline is administered once daily to each naris. In some cases, the theophylline is administered twice daily to each naris.

In some cases, the theophylline is administered each day for from about 7 days to about 365 days. In some cases, the theophylline is administered each day for from about 7 days to about 6 months. In some cases, the theophylline is administered each day for from about 7 days to about 4 months. In some cases, the theophylline is administered each day for from about 1 month to about 12 months.

In some cases, the subject experiences a decrease in a detection threshold (DT) score, a decrease in a recognition threshold (RT) score, an increase in a magnitude estimation (ME) score, and/or a change in a hedonic (H) score as measured with a forced-choice, three-stimuli, stepwise-staircase technique using one or more odorants after administering the theophylline to the subject. In some cases, the one or more odorants comprise pyridine, nitrobenzene, thiophene, amyl acetate, or a combination thereof.

In some cases, the subject experiences a decrease in an taste detection threshold (DT) score, a decrease in a recognition threshold (RT) score, an increase in a magnitude estimation (ME) score, and/or a change in a hedonic (H) score as measured with a forced-choice, three-stimuli, stepwise-staircase technique using one or more tastants testing compounds after administering the theophylline to the subject. In some cases, the one or more tastants comprise sodium chloride (NaCl), sucrose, hydrogen chloride (HCl), urea, or a combination thereof.

In some cases, the subject experiences a clinically detectable improvement in taste or smell function within 1-4 weeks of starting treatment.

Also disclosed are kits for the treatment of anosmia, hyposmia, dysosmia, ageusia, hypogeusia, or dysgeusia comprising: (a) any of the multi-dose nasal spray devices for delivery of theophylline to a human's nasal epithelium disclosed herein; and instructions for use.

Also disclosed herein are pharmaceutical dosage units for intranasal administration comprising an amount of theophylline effective for treating anosmia, hyposmia, dysosmia, ageusia, hypogeusia, or dysgeusia in a human in need thereof, wherein the steroid-free dosage unit comprises less than 1 mg of the theophylline in a pharmaceutically acceptable liquid carrier that has a pH that is greater than 7.0. Some cases comprise less than 500 µg, less than 250 µg, or less than 100 µg of the theophylline. Some cases comprise from about 10 µg to about 500 µg of the theophylline. Some cases comprise from about 20 µg to about 100 µg of the theophylline. Some cases comprise about 20 µg of the theophylline. Some cases comprise about 100 µg of the theophylline.

Also disclosed herein are pharmaceutical dosage unit for intranasal administration comprising an amount of theophylline effective for treating anosmia, hyposmia, dysosmia, ageusia, hypogeusia, or dysgeusia in a human in need thereof, wherein the steroid-free dosage unit comprises theophylline at a dosage level that is less than about 16.7 µg/kg in a pharmaceutically acceptable liquid carrier that has a pH that is greater than 7.0. In some cases, the dosage level is less than about 8.3 µg/kg, less than about 4.2 µg/kg, or less than about 1.7 µg/kg. In some cases, the dosage level is from about 0.33 µg/kg to about 1.7 µg/kg. In some cases, the dosage level is about 0.33 µg/kg. In some cases, the dosage level is about 1.7 µg/kg.

In some cases, the dosage unit is a steroid-free dosage unit.

In some cases, the pH is from 7.1 to 8.5. In some cases, the pH is from 7.1 to 7.4. In some cases, the pH is 7.1, 7.2, 7.3, 7.4, 7.5, 7.6, 7.7, 7.8, 7.9, 8, 8.1, 8.2, 8.3, 8.4, or 8.5. In some cases, the pH is at least 7.1, 7.2, 7.3, 7.4, 7.5, 7.6, 7.7, 7.8, 7.9, 8, 8.1, 8.2, 8.3, 8.4, or 8.5.

In some cases, a volume of the dosage unit is, for example, from about 50 µL to about 750 µL. In some cases, a volume of the dosage unit is from about 100 µL to about 400 µL. In some cases, a volume of the dosage unit is about: 50 µL, 75 µL, 100 µL, 125 µL, 150 µL, 175 µL, 200 µL, 225 µL, 250 µL, 275 µL, 300 µL, 325 µL, 350 µL, 375 µL, 400 µL, 425 µL, 450 µL, 475 µL, 500 µL, 525 µL, 550 µL, 575 µL, 600 µL, 625 µL, 650 µL, 675 µL, 700 µL, 725 µL or 750 µL.

In some cases, the dosage unit is in a plume that has a droplet size distribution characterized by one or more of the following: (a) less than 5% of the droplets in the plume having a size of less than 10 µm, (b) a D₁₀ of greater than 12.5 µm, wherein 10% of the droplets in the plume have a size less than the D₁₀, (c) a D₅₀ of from 30 to 70 µm, wherein 50% of the droplets in the plume have a size less than the D₅₀, (d) a D₉₀ of less than 200 µm, wherein 90% of the droplets in the plume have a size less than the D₉₀, and (e) a span of from 1 to 6, wherein the span is calculated according to: (D₉₀ - D₁₀)/D₅₀.

In some cases, the plume is characterized by less than 4% of the droplets in the plume having a size of less than 10 µm. In some cases, the plume is characterized by less than 3% of the droplets in the plume having a size of less than 10 µm. In some cases, the plume is characterized by less than 2% of the droplets in the plume having a size of less than 10 µm.

In some cases, the plume is characterized by the D₁₀ that is greater than 15 µm. In some cases, the plume is characterized by the D₁₀ that is greater than 17.5 µm. In some cases, the plume is characterized by the D₁₀ that is from 12.5 µm to 30 µm. In some cases, the plume is characterized by the D₁₀ that is from 15 µm to 25 µm.

In some cases, the plume is characterized by the D₅₀ that is from 40 µm to 60 µm. In some cases, the plume is characterized by the D₅₀ that is from 30 µm to 60 µm. In some cases, the plume is characterized by the D₅₀ that is from 30 µm to 50 µm. In some cases, the plume is characterized by the D₅₀ that is from 30 µm to 40 µm. In some cases, the plume is characterized by the D₅₀ that is about: 30 µm, 32.5 µm, 35 µm, 37.5 µm, 40 µm, 42.5 µm, 45 µm, 50 µm, 55 µm, 60 µm, 65 µm, or 70 µm.

In some cases, the plume is characterized by the D₉₀ that is less than 175 µm. In some cases, the plume is characterized by the D₉₀ that is less than 150 µm. In some cases, the plume is characterized by the D₉₀ that is less than 125 µm. In some cases, the plume is characterized by the D₉₀ that is less than 100 µm. In some cases, the plume is characterized by the D₉₀ that is less than 90 µm. In some cases, the plume is characterized by the D₉₀ that is from 75 µm to 199 µm. In some cases, the plume is characterized by the D₉₀ that is from 75 µm to 175 µm. In some cases, the plume is characterized by the D₉₀ that is from 75 µm to 150 µm. In some cases, the plume is characterized by the D₉₀ that is from 75 µm to 125 µm. In some cases, the plume is characterized by the D₉₀ that is from 75 µm to 100 µm. In some cases, the plume is characterized by the D₉₀ that is about: 75 µm, 80 µm, 85 µm, 90 µm, 100 µm, 110 µm, 120 µm, 130 µm, 140 µm, 150 µm, 160 µm, 170 µm, 180 µm, or 190µm.

In some cases, the plume is characterized by the span that is from 1 to 5. In some cases, the plume is characterized by the span that is from 1 to 4. In some cases, the plume is characterized by the span that is from 1 to 3. In some cases, the plume is characterized by the span that is from 1 to 2. In some cases, the plume is characterized by the span that is about: 1, 1.1, 1.2, 1.3, 1.4, 1.5, 1.6, 1.7, 1.8, 1.9, 2, 2.1, 2.2, 2.3, 2.4, 2.5, 2.6, 2.7, 2.7, 2.9, 3, 3.25, 3.5, 3.75, 4, 4.5, 5, 5.5, or 6.

In some cases, the plume is further characterized by having an ovality of from 0.7 to 1. In some cases, the plume is further characterized by having an ovality of from 0.8 to 1. In some cases, the plume is further characterized by having an ovality of from 0.9 to 1. In some cases, the plume is further characterized by having an ovality of about 1.

In some cases, the plume is further characterized by having a geometry of from 30° to 90°. In some cases, the plume is further characterized by having a geometry of from 45° to 75°. In some cases, the plume is further characterized by having a geometry of about: 30°, 35°, 40°, 45°, 50°, 55°, 60°, 65°, 70°, 75°, 80°, 85°, or 90°.

In some cases, the dosage unit is provided as a nasal spray or aerosol. In some cases, about 50% of droplets in the nasal spray or aerosol have a size of from about 15 µm to about 150 µm, from about 20 µm to about 100 µm, or from about 30 µm to about 70 µm. In some cases, less than about 20%, 15%, 10%, 9%, 8%, 7%, 6%, 5%, 4%, 3%, 2%, or 1% of droplets in the nasal spray or aerosol have a size of less than about 10 µm. In some cases, at least about 60%, 70%, 80%, 85%, 90%, or 95% of droplets in the nasal spray or aerosol have a size of less than about 200 µm. In some cases, at least about 50%, 60%, 70%, 80%, 90%, 95%, or 99% of droplets in the nasal spray or aerosol have a size of from about 10 µm to about 200 µm.

In some cases, the pharmaceutically acceptable liquid carrier comprises aminoboronic acid and its derivatives, amastatin, surfactants, bile salts, gelified insulin, bioadhesive microspheres, phospholipids, chitosan nanoparticles, alkyl glycerides, or a combination thereof.

In some cases, the pharmaceutically acceptable liquid carrier comprises water, a buffering agent, a flavoring agent, a humectant, a penetration enhancer, a pH adjusting agent, a preservative, a solvent or co-solvent, a surfactant, a tonicity adjusting agent, a viscosity adjusting agent, or a combination thereof. In some cases, the pharmaceutically acceptable liquid carrier comprises water.

In some cases, the pharmaceutically acceptable liquid carrier comprises the buffering agent that is potassium phosphate, sodium acetate, sodium citrate, sodium phosphate, trisodium citrate, or a combination thereof. In some cases, the pharmaceutically acceptable liquid carrier comprises the flavoring agent that is menthol, saccharin sodium, sorbitol, or a combination thereof. In some cases, the pharmaceutically acceptable liquid carrier comprises the humectant that is glycerin, propylene glycol, hexylene glycol, butylenes glycol, glyceryl triacetate, vinyl alcohol, neoagarobiose, glycerol, sorbitol, xylitol, maltitol, polydextrose, quillaia, lactic acid, urea, or aloe vera. In some cases, the pharmaceutically acceptable liquid carrier comprises the penetration enhancer that is oleic acid. In some cases, the pharmaceutically acceptable liquid carrier comprises the pH adjusting agent that is acetic acid, citric acid, hydrochloric acid, sodium hydroxide, sulfuric acid, or a combination thereof. In some cases, the pharmaceutically acceptable liquid carrier comprises the preservative that is benzalkonium chloride, benzethonium chloride, benzyl alcohol, butylated hydroxy toluene, butylated hydroxyanisole, chlorobutanol, edetate disodium, methylparaben, phenylethyl alcohol, phenylmercuric acetate, propylene paraben, propylparaben, thimerosal, or a combination thereof. In some cases, the pharmaceutically acceptable liquid carrier comprises the solvent or co-solvent that is ethanol, glycerol, glyceryl dioleate, glycine, polyethylene glycol (PEG), PEG 400, propylene glycol, triglycerides, or a combination thereof. In some cases, the pharmaceutically acceptable liquid carrier comprises the surfactant that is glyceryl monoleate, lecithin, PEG 3500, PEG 400, polyoxyl 400 stearate, polysorbate 20, polysorbate 80, propylene glycol, triglycerides, or a combination thereof. In some cases, the pharmaceutically acceptable liquid carrier comprises the tonicity adjusting agent that is dextrose, potassium chloride, sodium chloride, or a combination thereof. In some cases, the pharmaceutically acceptable liquid carrier comprises the viscosity adjusting agent that is carboxymethyl cellulose (CMC), Me-OH-Pr cellulose, microcrystalline cellulose (MCC), sodium carboxymethyl cellulose (Na CMC), or a combination thereof.

In some cases, the pharmaceutically acceptable liquid carrier comprises from about 0.01% to about 0.5% w/w acetic acid. In some cases, the pharmaceutically acceptable liquid carrier comprises from about 0.001% to about 0.119% w/w benzalkonium chloride. In some cases, the pharmaceutically acceptable liquid carrier comprises from about 0.001% to about 0.0366% w/w benzyl alcohol. In some cases, the pharmaceutically acceptable liquid carrier comprises from about 0.001% to about 0.01% w/w butylated hydroxy toluene. In some cases, the pharmaceutically acceptable liquid carrier comprises from about 0.00001% to about 0.0002% w/w butylated hydroxyanisole. In some cases, the pharmaceutically acceptable liquid carrier comprises from about 0.1% to about 2% w/w carboxymethyl cellulose. In some cases, the pharmaceutically acceptable liquid carrier comprises from about 0.01% to about 0.5% w/w chlorobutanol. In some cases, the pharmaceutically acceptable liquid carrier comprises from about 0.01% to about 0.5% w/w citric acid. In some cases, the pharmaceutically acceptable liquid carrier comprises from about 0.01% to about 0.5% w/w dextrose. In some cases, the pharmaceutically acceptable liquid carrier comprises from about 0.01% to about 0.5% w/w edetate disodium. In some cases, the pharmaceutically acceptable liquid carrier comprises from about 0.1% to about 2% w/w ethanol. In some cases, the pharmaceutically acceptable liquid carrier comprises from about 0.01% to about 0.233% w/w glycerin. In some cases, the pharmaceutically acceptable liquid carrier comprises from about 0.1% to about 5% w/w glycerol. In some cases, the pharmaceutically acceptable liquid carrier comprises from about 1% to about 15% w/w glyceryl dioleate. In some cases, the pharmaceutically acceptable liquid carrier comprises from about 1% to about 10% w/w glyceryl monoleate. In some cases, the pharmaceutically acceptable liquid carrier comprises from about 0.1% to about 10% w/w lecithin. In some cases, the pharmaceutically acceptable liquid carrier comprises from about 0.1% to about 5% w/w Me-OH-Pr cellulose. In some cases, the pharmaceutically acceptable liquid carrier comprises from about 0.001% to about 0.7% w/w methylparaben. In some cases, the pharmaceutically acceptable liquid carrier comprises from about 0.1% to about 2% w/w microcrystalline cellulose. In some cases, the pharmaceutically acceptable liquid carrier comprises from about 0.01% to about 0.132% w/w oleic acid. In some cases, the pharmaceutically acceptable liquid carrier comprises from about 0.1% to about 1.5% w/w PEG 3500. In some cases, the pharmaceutically acceptable liquid carrier comprises from about 0.1% to about 20% w/w PEG 400. In some cases, the pharmaceutically acceptable liquid carrier comprises from about 0.01% to about 0.254% w/w phenylethyl alcohol. In some cases, the pharmaceutically acceptable liquid carrier comprises from about 0.1% to about 5% w/w polyethylene glycol (PEG). In some cases, the pharmaceutically acceptable liquid carrier comprises from about 0.1% to about 15% w/w polyoxyl 400 stearate. In some cases, the pharmaceutically acceptable liquid carrier comprises from about 0.01% to about 2.5% w/w polysorbate 20. In some cases, the pharmaceutically acceptable liquid carrier comprises from about 0.1% to about 10% w/w polysorbate 80. In some cases, the pharmaceutically acceptable liquid carrier comprises from about 0.1% to about 1.9% w/w potassium chloride. In some cases, the pharmaceutically acceptable liquid carrier comprises from about 0.1% to about 20% w/w propylene glycol. In some cases, the pharmaceutically acceptable liquid carrier comprises from about 0.001% to about 0.1% w/w propylene paraben. In some cases, the pharmaceutically acceptable liquid carrier comprises from about 0.01% to about 0.3% w/w propylparaben. In some cases, the pharmaceutically acceptable liquid carrier comprises from about 0.1% to about 5% w/w sodium carboxymethyl cellulose (Na CMC). In some cases, the pharmaceutically acceptable liquid carrier comprises from about 0.1% to about 1.9% w/w sodium chloride. In some cases, the pharmaceutically acceptable liquid carrier comprises from about 0.5% to about 10% w/w sorbitol. In some cases, the pharmaceutically acceptable liquid carrier comprises from about 0.01% to about 0.4% w/w sulfuric acid. In some cases, the pharmaceutically acceptable liquid carrier comprises from about 0.1% to about 5% w/w triglycerides. In some cases, the pharmaceutically acceptable liquid carrier comprises from about 0.00001% to about 0.0006% w/w trisodium citrate.

Also disclosed are methods of treating anosmia, hyposmia, dysosmia, ageusia, hypogeusia, or dysgeusia in a subject in need thereof comprising administering to the subject in need thereof any of the pharmaceutical dosage units disclosed herein. In some cases, the pharmaceutical dosage unit is administered once daily to each naris. In some cases, the pharmaceutical dosage unit is administered twice daily to each naris.

In some cases, the pharmaceutical dosage unit is administered each day for from about 7 days to about 365 days. In some cases, the pharmaceutical dosage unit is administered each day for from about 7 days to about 6 months. In some cases, the pharmaceutical dosage unit is administered each day for from about 7 days to about 4 months. In some cases, the pharmaceutical dosage unit is administered each day for from about 1 month to about 12 months.

In some cases, the subject experiences a decrease in a detection threshold (DT) score, a decrease in a recognition threshold (RT) score, an increase in a magnitude estimation (ME) score, and/or a change in a hedonic (H) score as measured with a forced-choice, three-stimuli, stepwise-staircase technique using one or more odorants after administering the pharmaceutical dosage unit to the subject. In some cases, the one or more odorants comprise pyridine, nitrobenzene, thiophene, amyl acetate, or a combination thereof.

In some cases, the subject experiences a decrease in an taste detection threshold (DT) score, a decrease in a recognition threshold (RT) score, an increase in a magnitude estimation (ME) score, and/or a change in a hedonic (H) score as measured with a forced-choice, three-stimuli, stepwise-staircase technique using one or more tastants testing compounds after administering the pharmaceutical dosage unit to the subject. In some cases, the one or more tastants comprise sodium chloride (NaCl), sucrose, hydrogen chloride (HCl), urea, or a combination thereof.

Also disclosed are kits for the treatment of anosmia, hyposmia, dysosmia, ageusia, hypogeusia, or dysgeusia comprising: (a) a multi-dose nasal spray device that delivers any of the pharmaceutical dosage units disclosed herein; and (b) instructions for use.

Also disclosed are combination pharmaceutical dosage units for intranasal administration comprising: (a) a first amount of a non-selective PDE inhibitor; (b) a second amount of a second PDE inhibitor that is a PDE 1 inhibitor, a PDE2 inhibitor, a PDE3 inhibitor, a PDE 4 inhibitor, a PDE 5 inhibitor, or a PDE 10 inhibitor; and (c) a pharmaceutically acceptable liquid carrier; wherein the first amount and the second amount are effective in combination to treat anosmia, hyposmia, dysosmia, ageusia, hypogeusia, or dysgeusia in a subject in need thereof, wherein the subject is resistant to treatment with either the non-selective PDE inhibitor or the second PDE inhibitor alone.

In some cases, the non-selective PDE inhibitor is caffeine, aminophylline, paraxanthine, pentoxifylline, theobromine, theophylline, or oxphylline. In some cases, the non-selective PDE inhibitor is theophylline.

In some cases, the second PDE inhibitor is the PDE 1 inhibitor that is vinpocetine. In some cases, the second PDE inhibitor is the PDE 2 inhibitor that is EHNA. In some cases, the second PDE inhibitor is the PDE 3 inhibitor that is inamrinone, anagrelide, or cilostazol. In some cases, the second PDE inhibitor is the PDE 4 inhibitor that is mesembrine, rolipram, ibudilast, pclamilast, luteolin, drotaverine, or roflumilast. In some cases, the second PDE inhibitor is the PDE 5 inhibitor that is sildenafil, tadalafil, vardenafil, udenafil, avanafil, or dipyridamole. In some cases, the second PDE inhibitor is the PDE 10 inhibitor that is papaverine.

In some cases, the non-selective PDE inhibitor is caffeine and the first amount is from about 20 µg to about 100 µg per naris. In some cases, the non-selective PDE inhibitor is caffeine and the first amount is from about 0.33 µg/kg to about 1.7 µg/kg per naris.

In some cases, the non-selective PDE inhibitor is aminophylline and the first amount is from about 20 µg to about 100 µg per naris. In some cases, the non-selective PDE inhibitor is aminophylline and the first amount is from about 0.33 µg/kg to about 1.7 µg/kg per naris.

In some cases, the non-selective PDE inhibitor is paraxanthine and the first amount is from about 10 µg to about 100 µg per naris. In some cases, the non-selective PDE inhibitor is paraxanthine and the first amount is from about 0.17 µg/kg to about 1.7 µg/kg per naris.

In some cases, the non-selective PDE inhibitor is pentoxifylline and the first amount is from about 20 µg to about 100 µg per naris. In some cases, the non-selective PDE inhibitor is pentoxifylline and the first amount is from about 0.33 µg/kg to about 1.7 µg/kg per naris.

In some cases, the non-selective PDE inhibitor is theobromine and the first amount is from about 30 µg to about 150 µg per naris. In some cases, the non-selective PDE inhibitor is theobromine and the first amount is from about 0.5 µg/kg to about 2.5 µg/kg per naris.

In some cases, the non-selective PDE inhibitor is theophylline and the first amount is from about 20 µg to about 100 µg per naris. In some cases, the non-selective PDE inhibitor is theophylline and the first amount is from about 0.33 µg/kg to about 1.7 µg/kg per naris.

In some cases, the non-selective PDE inhibitor is oxphylline and the first amount is from about 100 µg to about 100 µg per naris. In some cases, the non-selective PDE inhibitor is oxphylline and the first amount is from about 0.17 µg/kg to about 1.7 µg/kg per naris.

In some cases, the second PDE inhibitor is inamrinone and the second amount is from about 2 µg to about 40 µg per naris. In some cases, the second PDE inhibitor is inamrinone and the second amount is from about 0.03 µg/kg to about 0.67 µg/kg per naris.

In some cases, the second PDE inhibitor is anagrelide and the second amount is from about 2 µg to about 40 µg per naris. In some cases, the second PDE inhibitor is anagrelide and the second amount is from about 0.03 µg/kg to about 0.67 µg/kg per naris.

In some cases, the second PDE inhibitor is cilostazol and the second amount is from about 2 µg to about 40 µg per naris. In some cases, the second PDE inhibitor is cilostazol and the second amount is from about 0.03 µg/kg to about 0.67 µg/kg per naris.

In some cases, the second PDE inhibitor is mesembrine and the second amount is from about 1 µg to about 10 µg per naris. In some cases, the second PDE inhibitor is mesembrine and the second amount is from about 0.02 µg/kg to about 0.33 µg/kg per naris.

In some cases, the second PDE inhibitor is rolipram and the second amount is from about 1 µg to about 10 µg per naris. In some cases, the second PDE inhibitor is rolipram and the second amount is from about 0.02 µg/kg to about 0.33 µg/kg per naris.

In some cases, the second PDE inhibitor is ibudilast and the second amount is from about 1 µg to about 10 µg per naris. In some cases, the second PDE inhibitor is ibudilast and the second amount is from about 0.02 µg/kg to about 0.33 µg/kg per naris.

In some cases, the second PDE inhibitor is piclamilast and the second amount is from about 1 µg to about 10 µg per naris. In some cases, the second PDE inhibitor is piclamilast and the second amount is from about 0.02 µg/kg to about 0.33 µg/kg per naris.

In some cases, the second PDE inhibitor is luteolin and the second amount is from about 1 µg to about 10 µg per naris. In some cases, the second PDE inhibitor is luteolin and the second amount is from about 0.02 µg/kg to about 0.33 µg/kg per naris.

In some cases, the second PDE inhibitor is drotaverine and the second amount is from about 2 µg to about 10 µg per naris. In some cases, the second PDE inhibitor is drotaverine and the second amount is from about 0.03 µg/kg to about 0.33 µg/kg per naris.

In some cases, the second PDE inhibitor is roflumilast and the second amount is from about 1 µg to about 10 µg per naris. In some cases, the second PDE inhibitor is roflumilast and the second amount is from about 0.02 µg/kg to about 0.33 µg/kg per naris.

In some cases, the second PDE inhibitor is sildenafil and the second amount is from about 5 µg to about 150 µg per naris. In some cases, the second PDE inhibitor is sildenafil and the second amount is from about 0.08 µg/kg to about 2.5 µg/kg per naris.

In some cases, the second PDE inhibitor is tadalafil and the second amount is from about 5 µg to about 150 µg per naris. In some cases, the second PDE inhibitor is tadalafil and the second amount is from about 0.08 µg/kg to about 2.5 µg/kg per naris.

In some cases, the second PDE inhibitor is vardenafil and the second amount is from about 5 µg to about 150 µg per naris. In some cases, the second PDE inhibitor is vardenafil and the second amount is from about 0.08 µg/kg to about 2.5 µg/kg per naris.

In some cases, the second PDE inhibitor is udenafil and the second amount is from about 1 µg to about 100 µg per naris. In some cases, the second PDE inhibitor is udenafil and the second amount is from about 0.02 µg/kg to about 1.7 µg/kg per naris.

In some cases, the second PDE inhibitor is avanafil and the second amount is from about 1 µg to about 150 µg per naris. In some cases, the second PDE inhibitor is avanafil and the second amount is from about 0.02 µg/kg to about 2.5 µg/kg per naris.

In some cases, the second PDE inhibitor is dipyridamole and the second amount is from about 1 µg to about 150 µg per naris. In some cases, the second PDE inhibitor is dipyridamole and the second amount is from about 0.02 µg/kg to about 2.5 µg/kg per naris.

In some cases, the second PDE inhibitor is papaverine and the second amount is from about 10 µg to about 200 µg per naris. In some cases, the second PDE inhibitor is avanafil and the second amount is from about 0.17 µg/kg to about 3.3 µg/kg per naris. In some cases, the first amount and second amount are indicated in Table XIV.

Some cases further comprise a third amount of a third PDE inhibitor that is optionally in a different class of PDE inhibitor from the non-selective PDE inhibitor and the second PDE inhibitor. In some cases, the third amount is indicated in Table XIV.

In some cases, the combination pharmaceutical dosage unit is a steroid-free, combination pharmaceutical dosage unit.

In some cases, the pharmaceutically acceptable liquid carrier has a pH that is greater than 7.0. In some cases, the pharmaceutically acceptable liquid carrier has a pH that is from 7.1 to 8.5. In some cases, the pharmaceutically acceptable liquid carrier has a pH that is from 7.1 to 7.4. In some cases, the pharmaceutically acceptable liquid carrier has a pH that is 7.1, 7.2, 7.3, 7.4, 7.5, 7.6, 7.7, 7.8, 7.9, 8, 8.1, 8.2, 8.3, 8.4, or 8.5. In some cases, the pharmaceutically acceptable liquid carrier has a pH that is at least 7.1, 7.2, 7.3, 7.4, 7.5, 7.6, 7.7, 7.8, 7.9, 8, 8.1, 8.2, 8.3, 8.4, or 8.5.

In some cases, a volume of the dosage unit is from about 50 µL to about 750 µL. In some cases, a volume of the dosage unit is from about 100 µL to about 400 µL. In some cases, a volume of the dosage unit is about: 50 µL, 75 µL, 100 µL, 125 µL, 150 µL, 175 µL, 200 µL, 225 µL, 250 µL, 275 µL, 300 µL, 325 µL, 350 µL, 375 µL, 400 µL, 425 µL, 450 µL, 475 µL, 500 µL, 525 µL, 550 µL, 575 µL, 600 µL, 625 µL, 650 µL, 675 µL, 700 µL, 725 µL or 750 µL.

In some cases, the combination pharmaceutical dosage unit is in a plume that has a droplet size distribution characterized by one or more of the following: (a) less than 5% of the droplets in the plume having a size of less than 10 µm, (b) a D₁₀ of greater than 12.5 µm, wherein 10% of the droplets in the plume have a size less than the D₁₀, (c) a D₅₀ of from 30 to 70 µm, wherein 50% of the droplets in the plume have a size less than the D₅₀, (d) a D₉₀ of less than 200 µm, wherein 90% of the droplets in the plume have a size less than the D₉₀, and (e) a span of from 1 to 6, wherein the span is calculated according to: (D₉₀ - D₁₀)/D₅₀.

In some cases, the plume is characterized by the D₉₀ that is less than 175 µm. In some cases, the plume is characterized by the D₉₀ that is less than 150 µm. In some cases, the plume is characterized by the D₉₀ that is less than 125 µm. In some cases, the plume is characterized by the D₉₀ that is less than 100 µm. In some cases, the plume is characterized by the D₉₀ that is less than 90 µm. In some cases, the plume is characterized by the D₉₀ that is from 75 µm to 199 µm. In some cases, the plume is characterized by the D₉₀ that is from 75 µm to 175 µm. In some cases, the plume is characterized by the D₉₀ that is from 75 µm to 150 µm. In some cases, the plume is characterized by the D₉₀ that is from 75 µm to 125 µm. In some cases, the plume is characterized by the D₉₀ that is from 75 µm to 100 µm. In some cases, the plume is characterized by the D₉₀ that is about: 75 µm, 80 µm, 85 µm, 90 µm, 100 µm, 110 µm, 120 µm, 130 µm, 140 µm, 150 µm, 160 µm, 170 µm, 180 µm, or 190µm.

In some cases, the plume is characterized by the span that is from 1 to 5. In some cases, the plume is characterized by the span that is from 1 to 4. In some cases, the plume is characterized by the span that is from 1 to 3. In some cases, the plume is characterized by the span that is from 1 to 2. In some cases, the plume is characterized by the span that is about: 1, 1.1, 1.2, 1.3, 1.4, 1.5, 1.6, 1.7, 1.8, 1.9, 2, 2.1, 2.2, 2.3, 2.4, 2.5, 2.6, 2.7, 2.7, 2.9, 3, 3.25, 3.5, 3.75, 4, 4.5, 5, 5.5, or 6.

In some cases, the plume is further characterized by having an ovality of from 0.7 to 1. In some cases, the plume is further characterized by having an ovality of from 0.8 to 1. In some cases, the plume is further characterized by having an ovality of from 0.9 to 1. In some cases, the plume is further characterized by having an ovality of about 1.

In some cases, the plume is further characterized by having a geometry of from 30° to 90°. In some cases, the plume is further characterized by having a geometry of from 45° to 75°. In some cases, the plume is further characterized by having a geometry of about: 30°, 35°, 40°, 45°, 50°, 55°, 60°, 65°, 70°, 75°, 80°, 85°, or 90°.

In some cases, the dosage unit is provided as a nasal spray or aerosol. In some cases, about 50% of droplets in the nasal spray or aerosol have a size of from about 15 µm to about 150 µm, from about 20 µm to about 100 µm, or from about 30 µm to about 70 µm. In some cases, less than about 20%, 15%, 10%, 9%, 8%, 7%, 6%, 5%, 4%, 3%, 2%, or 1% of droplets in the nasal spray or aerosol have a size of less than about 10 µm. In some cases, at least about 60%, 70%, 80%, 85%, 90%, or 95% of droplets in the nasal spray or aerosol have a size of less than about 200 µm. In some cases, at least about 50%, 60%, 70%, 80%, 90%, 95%, or 99% of droplets in the nasal spray or aerosol have a size of from about 10 µm to about 200 µm.

In some cases, the pharmaceutically acceptable liquid carrier comprises aminoboronic acid and its derivatives, amastatin, surfactants, bile salts, gelified insulin, bioadhesive microspheres, phospholipids, chitosan nanoparticles, alkyl glycerides, or a combination thereof.

In some cases, the pharmaceutically acceptable liquid carrier comprises water, a buffering agent, a flavoring agent, a humectant, a penetration enhancer, a pH adjusting agent, a preservative, a solvent or co-solvent, a surfactant, a tonicity adjusting agent, a viscosity adjusting agent, or a combination thereof. In some cases, the pharmaceutically acceptable liquid carrier comprises water.

In some cases, the pharmaceutically acceptable liquid carrier comprises the buffering agent that is potassium phosphate, sodium acetate, sodium citrate, sodium phosphate, trisodium citrate, or a combination thereof. In some cases, the pharmaceutically acceptable liquid carrier comprises the flavoring agent that is menthol, saccharin sodium, sorbitol, or a combination thereof. In some cases, the pharmaceutically acceptable liquid carrier comprises the humectant that is glycerin, propylene glycol, hexylene glycol, butylenes glycol, glyceryl triacetate, vinyl alcohol, neoagarobiose, glycerol, sorbitol, xylitol, maltitol, polydextrose, quillaia, lactic acid, urea, or aloe vera. In some cases, the pharmaceutically acceptable liquid carrier comprises the penetration enhancer that is oleic acid. In some cases, the pharmaceutically acceptable liquid carrier comprises the pH adjusting agent that is acetic acid, citric acid, hydrochloric acid, sodium hydroxide, sulfuric acid, or a combination thereof. In some cases, the pharmaceutically acceptable liquid carrier comprises the preservative that is benzalkonium chloride, benzethonium chloride, benzyl alcohol, butylated hydroxy toluene, butylated hydroxyanisole, chlorobutanol, edetate disodium, methylparaben, phenylethyl alcohol, phenylmercuric acetate, propylene paraben, propylparaben, thimerosal, or a combination thereof. In some cases, the pharmaceutically acceptable liquid carrier comprises the solvent or co-solvent that is ethanol, glycerol, glyceryl dioleate, glycine, polyethylene glycol (PEG), PEG 400, propylene glycol, triglycerides, or a combination thereof. In some cases, the pharmaceutically acceptable liquid carrier comprises the surfactant that is glyceryl monoleate, lecithin, PEG 3500, PEG 400, polyoxyl 400 stearate, polysorbate 20, polysorbate 80, propylene glycol, triglycerides, or a combination thereof. In some cases, the pharmaceutically acceptable liquid carrier comprises the tonicity adjusting agent that is dextrose, potassium chloride, sodium chloride, or a combination thereof. In some cases, the pharmaceutically acceptable liquid carrier comprises the viscosity adjusting agent that is carboxymethyl cellulose (CMC), Me-OH-Pr cellulose, microcrystalline cellulose (MCC), sodium carboxymethyl cellulose (Na CMC), or a combination thereof.

In some cases, the pharmaceutically acceptable liquid carrier comprises from about 0.01% to about 0.5% w/w acetic acid. In some cases, the pharmaceutically acceptable liquid carrier comprises from about 0.001% to about 0.119% w/w benzalkonium chloride. In some cases, the pharmaceutically acceptable liquid carrier comprises from about 0.001% to about 0.0366% w/w benzyl alcohol. In some cases, the pharmaceutically acceptable liquid carrier comprises from about 0.001% to about 0.01% w/w butylated hydroxy toluene. In some cases, the pharmaceutically acceptable liquid carrier comprises from about 0.00001% to about 0.0002% w/w butylated hydroxyanisole. In some cases, the pharmaceutically acceptable liquid carrier comprises from about 0.1% to about 2% w/w carboxymethyl cellulose. In some cases, the pharmaceutically acceptable liquid carrier comprises from about 0.01% to about 0.5% w/w chlorobutanol. In some cases, the pharmaceutically acceptable liquid carrier comprises from about 0.01% to about 0.5% w/w citric acid. In some cases, the pharmaceutically acceptable liquid carrier comprises from about 0.01% to about 0.5% w/w dextrose. In some cases, the pharmaceutically acceptable liquid carrier comprises from about 0.01% to about 0.5% w/w edetate disodium. In some cases, the pharmaceutically acceptable liquid carrier comprises from about 0.1% to about 2% w/w ethanol. In some cases, the pharmaceutically acceptable liquid carrier comprises from about 0.01% to about 0.233% w/w glycerin. In some cases, the pharmaceutically acceptable liquid carrier comprises from about 0.1% to about 5% w/w glycerol. In some cases, the pharmaceutically acceptable liquid carrier comprises from about 1% to about 15% w/w glyceryl dioleate. In some cases, the pharmaceutically acceptable liquid carrier comprises from about 1% to about 10% w/w glyceryl monoleate. In some cases, the pharmaceutically acceptable liquid carrier comprises from about 0.1% to about 10% w/w lecithin. In some cases, the pharmaceutically acceptable liquid carrier comprises from about 0.1% to about 5% w/w Me-OH-Pr cellulose. In some cases, the pharmaceutically acceptable liquid carrier comprises from about 0.001% to about 0.7% w/w methylparaben. In some cases, the pharmaceutically acceptable liquid carrier comprises from about 0.1% to about 2% w/w microcrystalline cellulose. In some cases, the pharmaceutically acceptable liquid carrier comprises from about 0.01% to about 0.132% w/w oleic acid. In some cases, the pharmaceutically acceptable liquid carrier comprises from about 0.1% to about 1.5% w/w PEG 3500. In some cases, the pharmaceutically acceptable liquid carrier comprises from about 0.1% to about 20% w/w PEG 400. In some cases, the pharmaceutically acceptable liquid carrier comprises from about 0.01% to about 0.254% w/w phenylethyl alcohol. In some cases, the pharmaceutically acceptable liquid carrier comprises from about 0.1% to about 5% w/w polyethylene glycol (PEG). In some cases, the pharmaceutically acceptable liquid carrier comprises from about 0.1% to about 15% w/w polyoxyl 400 stearate. In some cases, the pharmaceutically acceptable liquid carrier comprises from about 0.01% to about 2.5% w/w polysorbate 20. In some cases, the pharmaceutically acceptable liquid carrier comprises from about 0.1% to about 10% w/w polysorbate 80. In some cases, the pharmaceutically acceptable liquid carrier comprises from about 0.1% to about 1.9% w/w potassium chloride. In some cases, the pharmaceutically acceptable liquid carrier comprises from about 0.1% to about 20% w/w propylene glycol. In some cases, the pharmaceutically acceptable liquid carrier comprises from about 0.001% to about 0.1% w/w propylene paraben. In some cases, the pharmaceutically acceptable liquid carrier comprises from about 0.01% to about 0.3% w/w propylparaben. In some cases, the pharmaceutically acceptable liquid carrier comprises from about 0.1% to about 5% w/w sodium carboxymethyl cellulose (Na CMC). In some cases, the pharmaceutically acceptable liquid carrier comprises from about 0.1% to about 1.9% w/w sodium chloride. In some cases, the pharmaceutically acceptable liquid carrier comprises from about 0.5% to about 10% w/w sorbitol. In some cases, the pharmaceutically acceptable liquid carrier comprises from about 0.01% to about 0.4% w/w sulfuric acid. In some cases, the pharmaceutically acceptable liquid carrier comprises from about 0.1% to about 5% w/w triglycerides. In some cases, the pharmaceutically acceptable liquid carrier comprises from about 0.00001% to about 0.0006% w/w trisodium citrate.

Also disclosed are methods of treating anosmia, hyposmia, dysosmia, ageusia, hypogeusia, or dysgeusia in a subject in need thereof comprising administering to the subject in need thereof any of the combination pharmaceutical dosage units disclosed herein. In some cases, the combination pharmaceutical dosage unit is administered once daily to each naris. In some cases, the combination pharmaceutical dosage unit is administered twice daily to each naris.

In some cases, the combination pharmaceutical dosage unit is administered each day for from about 7 days to about 365 days. In some cases, the combination pharmaceutical dosage unit is administered each day for from about 7 days to about 6 months. In some cases, the combination pharmaceutical dosage unit is administered each day for from about 7 days to about 4 months. In some cases, the combination pharmaceutical dosage unit is administered each day for from about 1 month to about 12 months.

In some cases, the subject experiences a decrease in a detection threshold (DT) score, a decrease in a recognition threshold (RT) score, an increase in a magnitude estimation (ME) score, and/or a change in a hedonic (H) score as measured with a forced-choice, three-stimuli, stepwise-staircase technique using one or more odorants after administering the combination pharmaceutical dosage unit to the subject. In some cases, the one or more odorants comprise pyridine, nitrobenzene, thiophene, amyl acetate, or a combination thereof.

In some cases, the subject experiences a decrease in an taste detection threshold (DT) score, a decrease in a recognition threshold (RT) score, an increase in a magnitude estimation (ME) score, and/or a change in a hedonic (H) score as measured with a forced-choice, three-stimuli, stepwise-staircase technique using one or more tastants testing compounds after administering the combination pharmaceutical dosage unit to the subject. In some cases, the one or more tastants comprise sodium chloride (NaCl), sucrose, hydrogen chloride (HCl), urea, or a combination thereof.

Also disclosed are kits for the treatment of anosmia, hyposmia, dysosmia, ageusia, hypogeusia, or dysgeusia comprising: (a) a multi-dose nasal spray device that delivers any of the combination pharmaceutical dosage units disclosed herein; and (b) instructions for use.

### BRIEF DESCRIPTION OF THE DRAWINGS

The novel features of the invention are set forth with particularity in the appended claims. A better understanding of the present disclosure will be obtained by reference to the following detailed description that sets forth illustrative cases, in which the principles of the disclosure are utilized, and the accompanying drawings of which:

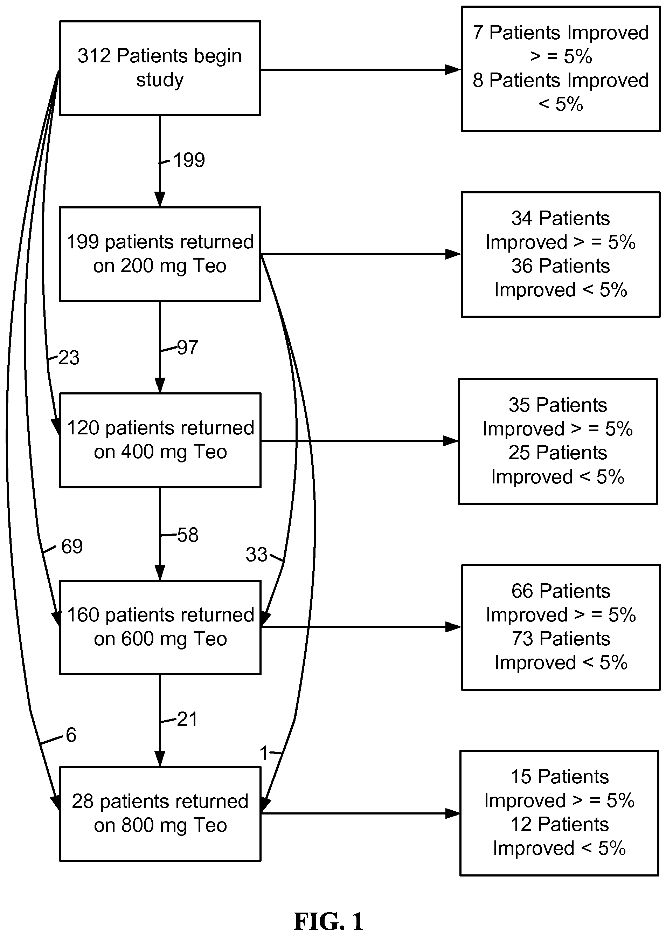

**Figure 1** illustrates the structure of the patient flow of the clinical trial showing the number and percentage of patients that returned on theophylline treatment. Three hundred twelve patients began the study on 200mg. If improved <5% patient number indicates progression to next step up in dose (400mg, 600mg, 800mg). Left sided numbers (lines) indicate numbers of distant patients who returned for first time (see text). Right sided numbers (lines) indicate patient numbers who improved <5% and returned on a higher dose. Difference between right sided numbers and patient numbers who improved <5% (in right boxes) indicate number of patient drop outs at each dose. If improved ≥5% patient data not included in further step-up doses.
**Figure 2** is a comparison of DT and RT values for pyridine (PYRD), nitrobenzene (NO2B), thiophene (THIO) and amyl acetate (AA) in 312 patients before treatment and in all patients in each group after treatment with oral theophylline at 200mg, 400mg, 600mg and 800mg (see Tables II, IV-VII).
**Figure 3** is a comparison of ME and H values for pyridine (PYRD), nitrobenzene (NO2B), thiophene (THIO) and amyl acetate (AA) in 312 patients before treatment and in all patients in each group after treatment with oral theophylline at 200mg, 400mg, 600mg and 800mg (see Tables II, IV-VII).
**Figure 4** shows gustometry in patients with hyposmia and hypogeusia before and after treatment with oral and intranasal theophylline.
**Figure 5** shows olfactometry in patients with hyposmia and hypogeusia before and after treatment with oral and intranasal theophylline.
**Figure 6** shows the comparison of quantitative subjective changes after oral and intranasal theophylline treatment.

### DETAILED DESCRIPTION

Unless defined otherwise, all technical and scientific terms used herein have the same meaning as commonly understood by one of the ordinary skill in the are to which this invention belongs. Unless mentioned otherwise, the techniques employed or contemplated herein are standard methodologies. The described materials, methods, and examples are illustrative only.

The details of one or more illustrative examples are set forth in the accompanying drawings, the claims, and the description herein.

Unless otherwise indicted, open terms for example "contain," "containing," include," "including," and the like mean comprising.

The singular forms "a," "an," and "the" are used herein to include plural references unless the context clearly dictates otherwise.

Some cases herein contemplate numberical ranges. When a numerical range is provided, unless otherwise indicated, the range includes the range endpoints. Unless otherwise indicated, numerical ranges include all values and subbranges therein as if explicitly written out.

The term "about" means the indicated numerical value ± 10%. All numerical indications within this specification are to be interpreted as being qualified by "about" unless the context clearly indicates otherwise.

"Dosage unit" as used herein, refers to a discrete amount of a pharmaceutical composition that is administered in a single event or package. The meaning of the term dosage unit is context specific. For example, a dosage unit for an intranasally administered liquid pharmaceutical composition would be the volume of the composition that is administered in a single event. For a nasal spray, the dosage unit would be the volume of the composition that is released upon each actuation of the nasal spray device. For a solid composition, a single dosage unit can, for example, be a single pill, tablet, or capsule.

"Phosphodiesterase inhibitor" or "PDE inhibitor" refers to any compound that inhibits a phosphodiesterase enzyme, isozyme or allozyme. The term refers to selective or non-selective inhibitors of cyclic guanosine 3',5'-monophosphate phosphodiesterases (cGMP-PDE) and cyclic adenosine 3',5'-monophosphate phosphodiesterases (cAMP-PDE).

"Patient" or "subject" refers to animals, mammals, humans (e.g., children, teenagers, adults, elderly).

The term "or" can be used conjunctively or disjunctively.

"Magnitude estimation" or "ME" is a measurement of the ability of a subject to determine the strength of a stimulant, such as an odorant or a tastant.

"Recognition threshold" or "RT" is a measurement of the ability of a subject to recognize the identity of a stimulant, such as an odorant or a tastant.

"Detection threshold" or "DT" is a measurement of the ability of a subject to recognize exposure to a stimulant, such as an odorant or a tastant.

A "hedonic" value or "H" value is a measurement of a subject's reaction to a stimulant, such as an odorant or a tastant, as being pleasant or unpleasant.

"Percentage by weight" or "w/w" means ratio of the mass of the specified ingredient verses the mass of the entire composition (*e.g.,* dosage unit).

"Plume geometry" or "geometry" when used in connection with a plume, means the measurement of the angle of the plume at its origin. Plume geometry can be measured at two distances from the origin of the plume, for example, at two side views 90° relative to each other. Plume geometry can also be calculated from the spray pattern.

"Spray pattern," "plume ovality," or "ovality" when used in connection with a plume, refers to shape and size of the plume at a distance from its origin. "Ovality" can be measured as the ratio of the largest diameter to the smallest diameter.

"D₁₀," "D₅₀," "D₉₀," and "span" are measurements of the droplet or particle size distribution of a plume. In a plume, 10% of the droplets have a size less than the D₁₀, 50% of the droplets have a size less than the D₅₀, and 90% of the droplets have a size less than the D₉₀. The span can be calculated from these numbers according to the following formula: span = (D₉₀ - D₁₀)/D₅₀.

"Total volume," when used in connection with a plume, refers to the total volume of all droplets or particles in the plume. For example, a plume with a total volume of 100 µL contains 100 µL of liquid.

Disclosed herein are methods, compositions, kits, and devices for the treatment of taste and smell disorders with phosphodiesterase inhibitors. Taste disorders that can be treated include ageusia, hypogeusia, and dysgeusia. Smell disorders that can be treated include anosmia, hyposmia, and dysosmia.

Also disclosed herein are multi-dose nasal spray devices for delivery of theophylline to a human's nasal epithelium that delivers a dosage unit in a plume upon actuation. The nasal spray devices provide numerous advantages in the treatment of taste and smell disorders. For example, the multi-dose nasal spray devices provided herein can ensure a high level of consistency in the delivery of the theophylline by delivering a dosage unit within a well characterized and reproducible plume directly to the nasal epithelium. Delivery of the theophylline directly to the nasal epithelium can enable the use of greatly reduced amounts of theophylline. Reducing the levels of theophylline that are administered can reduce and/or eliminate side effects associate with theophylline use. The level of theophylline (or any other active ingredient) can be reduced to a level such that it is undetectable in the bloodstream after administration. Also, the efficacy of treatment can be increased because the theophylline is delivered directly to the sites of action. Despite using much smaller dosage levels than are orally administered, a greater level of theophylline can reach the nasal olfactory epithelium. In this way, subject in need thereof can experience clinically detectable improvements in taste or smell function in a greatly reduced time frame in comparison to oral administration of theophylline.

Multi-dose nasal spray devices that deliver a dosage unit of theophylline in a plume provide advantages over intranasal administration with a syringe. When administered with a syringe, a larger volume is generally used and the doseage unit is administered in a stream of liquid. This liquid can run out of the nose, either running down the throat or dripping out of the nostril, which can reduce the effectiveness of the treatement and/or cause irritation or discomfort. Uneven exposure of the nasal epithelium to the active ingredient can also result. The disclosed multi-dose nasal spray devices that deliver a dosage unit of theophylline in a plume, by contrast, can use even smaller volumes of liquid because the droplet size distribution of the plume is calibrated to neither drip nor enter the esophogous. The spray geometry can also be calibrated to provide a more even distribution of theophylline at the site of administration. Further, the disclosed devices and plumes can increase patient compliance due to ease of use.

Also disclosed herein are pharmaceutical dosage units for intranasal administration comprising an amount of theophylline effective for treating anosmia, hyposmia, dysosmia, ageusia, hypogeusia, or dysgeusia in a human in need thereof comprising less than 1 mg of the theophylline in a pharmaceutically acceptable liquid carrier that has a pH that is greater than 7.0. The pH of intranasal dosage units can affect the rate at which the active ingredients in the dosage unit are absorbed by the nasal epithelium.

Also disclosed herein are combination pharmaceutical dosage units for intranasal administration comprising: a non-selective PDE inhibitor; and a second, specific PDE inhibitor. The combination formulations can be effective to treat anosmia, hyposmia, dysosmia, ageusia, hypogeusia, or dysgeusia in a subject that is resistant to treatment with either the non-selective PDE inhibitor or the second PDE inhibitor alone.

Theophylline and papaverine are representative members of non-specific PDE inhibitors that are prescribed orally to treat asthma and chronic obstructive pulmonary disease (COPD) through the relaxation of smooth muscle in the airways. Theophylline has anti-inflammatory effects on the airways that is useful to combat the abnormal inflammation seen in asthmatics. Most importantly, this anti-inflammatory effect is found at levels in the blood well below that which causes the common side effects seen in most people. Patients with emphysema and chronic bronchitis can also be helped with theophylline when their symptoms are partially related to reversible airway narrowing.

Theophylline is a methylxanthine derivative; other non-selective phosphodiesterase inhibitors in this class include caffeine, IBMX (3-isobutyl-1-methylxanthine, aminophylline, doxophylline, cipamphylline, theobromine, pentoxifylline (oxpentifylline) and diprophylline.

PDE1 selective inhibitors, formerly known as calcium- and calmodulin-dependent phosphodiesterases, include eburnamenine-14-carboxylic acid ethyl ester (vinpocetine), used to induce vasorelaxtion on cerebral smooth muscle tissue.

PDE2 decreases aldosterone secretion and is suggested to play an important role in the regulation of elevated intracellular concentrations of cAMP and cGMP in platelets. Several regions of the brain express PDE2 and rat experiments indicate that inhibition of PDE2 enhances memory. PDE2 may play a role in regulation of fluid and cell extravasation during inflammatory conditions as PDE2 is localized to microvessels, especially venous capillary and endothelial cells, but apparently not to larger vessels. PDE2 may also be a good pharmacological target for pathological states such as sepsis or in more localized inflammatory responses such as thrombin-induced edema formation in the lung. PDE-2 selective inhibitors include EHNA (erythro-9-(2-hydroxy-3-nonyl) adenine), 9-(6-phenyl-2-oxohex-3-yl)-2-(3,4-dimethoxybenzyl)-purin-6-one (PDP), and BAY 60-7750.

The PDE3 family hydrolyzes cAMP and cGMP, but in a manner suggesting that *in vivo,* the hydrolysis of cAMP is inhibited by cGMP. They also are distinguished by their ability to be activated by several phosphorylation pathways including the PKA and PI3K/PKB pathways. PDE3A is relatively highly expressed in platelets, as well as in cardiac myocytes and oocytes. PDE3B is a major PDE in adipose tissue, liver, and pancreas, as well as in several cardiovascular tissues. Both PDE3A and PDE3B are highly expressed in vascular smooth muscle cells and are likely to modulate contraction.

PDE3 inhibitors mimic sympathetic stimulation to increase cardiac inotropy, chronotropy and dromotropy. PDE3 inhibitors also antagonize platelet aggregation, increase myocardial contractility, and enhance vascular and airway smooth muscle relaxation. PDE3A is a regulator of this process and PDE3 inhibitors effectively prevent aggregation. In fact one drug, cilastazol (Pletal), is approved for treatment of intermittent claudication. Its mechanism of action is thought to involve inhibition of platelet aggregation along with inhibition of smooth muscle proliferation and vasodilation. PDE3-selective inhibitors include enoximone, milrinone (Primacor), amrinone, cilostamide, cilostazol (Pletal) and trequinsin.

PDE4 inhibitors can effectively suppress release of inflammatory mediators, *e.g.,* cytokines, inhibit the production of reactive oxygen species and immune cell infiltration. PDE4-selective inhibitors include mesembrine, rolipram, ibudilast, a neuroprotective and bronchodilator drug used mainly in the treatment of asthma and stroke, and roflumilast (Daxas) and cilomilast (Airflo), currently in phase III clinical trials for treatment of chronic obstructive pulmonary disease. Other inflammatory diseases for which PDE4 inhibitors are currently being developed include asthma, arthritis, and psoriasis.

PDE5 is a regulator of vascular smooth muscle contraction best known as the molecular target for several well-advertised drugs used to treat erectile dysfunction and pulmonary hypertension. In the lung, inhibition of PDE5 opposes smooth muscle vasoconstriction, and PDE5 inhibitors are in clinical trials for treatment of pulmonary hypertension.

PDE5-selective inhibitors include sildenafil, tadalafil, vardenafil, udenafil and avanafil.

PDE inhibitors inhibit cellular apoptosis by inhibiting TNF alpha, TRAIL and their metabolites. PDE inhibitors activate the production and secretion of nitric oxide in all tissues thereby inducing vasorelaxation or vasodilation of all blood vessels including those of the peripheral blood vessels (inhibiting intermittent claudication), the distal extremities and in the penile region contributing to penile erection.

PDE inhibitors useful in the present disclosure include, for example, filaminast, piclamilast, rolipram, Org 20241, MCI-154, roflumilast, toborinone, posicar, lixazinone, zaprinast, sildenafil, pyrazolopyrimidinones (such as those disclosed in WO 98/49166), motapizone, pimobendan, zardaverine, siguazodan, CI-930, EMD 53998, imazodan, saterinone, loprinone hydrochloride, 3-pyridinecarbonitrile derivatives, denbufyllene, albifylline, torbafylline, doxofylline, theophylline, pentoxofylline, nanterinone, cilostazol, cilostamide, MS 857, piroximone, milrinone, aminone, tolafentrine, dipyridamole, papaverine, E4021, thienopyrimidine derivatives (such as those disclosed in WO 98/17668), triflusal, ICOS-351, tetrahydropiperazino[1,2-b]beta-carboline-1,4-dione derivatives (such as those disclosed in U.S. Pat. No. 5,859,006, WO 97/03985 and WO 97/03675), carboline derivatives, (such as those disclosed in WO 97/43287), 2-pyrazolin-5-one derivatives (such as those disclosed in U.S. Pat. No. 5,869,516), fused pyridazine derivatives (such as those disclosed in U.S. Pat. No. 5,849,741), quinazoline derivatives (such as those disclosed in U.S. Pat. No. 5,614,627), anthranilic acid derivatives (such as those disclosed in U.S. Pat. No. 5,714,993), imidazoquinazoline derivatives (such as those disclosed in WO 96/26940), and the like. Also included are those phosphodiesterase inhibitors disclosed in WO 99/21562 and WO 99/30697. In some cases, the intranasal composition does not comprise a PDE5 selective inhibitor.

Sources of information for the above, and other phosphodiesterase inhibitors include Goodman and Gilman, The Pharmacological Basis of Therapeutics (9th Ed.), McGraw-Hill, Inc. (1995), The Physician's Desk Reference (49th Ed.), Medical Economics (1995), Drug Facts and Comparisons (1993 Ed), Facts and Comparisons (1993), and The Merck Index (12th Ed.), Merck & Co., Inc. (1996).

Other medicaments may be combined with or administered contemporaneously with at least one PDE inhibitor to complement and/or to enhance the prevention or treatment effect of a PDE inhibitor. These other medicaments include vasoactive agents, anticholinergic agents, leukotriene receptor antagonists, thromboxane synthetase inhibitors, thromboxane A₂ receptor antagonist, mediator release inhibitor, antihistamic agent, cytokine inhibitor, prostaglandins, forskolin, elastase inhibitor, steroid, expectorant, or antibacterial agent. The other medicaments can be administered simultaneously with, subsequently to, or prior to administration of the PDE inhibitors.

In one case, the patient is administered a therapeutically effective amount of a PDE inhibitor and a vasoactive agent. A vasoactive agent is any therapeutic agent capable of relaxing vascular smooth muscle. Suitable vasoactive agents include, but are not limited to, potassium channel activators (such as, for example, nicorandil, pinacidil, cromakalim, minoxidil, aprilkalim, loprazolam and the like); calcium blockers (such as, for example, nifedipine, veraparmil, diltiazem, gallopamil, niludipine, nimodipins, nicardipine, and the like); beta-blockers (such as, for example, butixamine, dichloroisoproterenol, propanolol, alprenolol, bunolol, nadolol, oxprenolol, perbutolol, pinodolol, sotalol, timolol, metoprolol, atenolol, acebutolol, bevantolol, pafenolol, tolamodol, and the like); long and short acting alpha-adrenergic receptor antagonist (such as, for example, phenoxybenzamide, dibenamine, doxazosin, terazosin, phentolamine, tolazoline, prozosin, trimazosin, yohimbine, moxisylyte and the like adenosine, ergot alkaloids (such as, for example, ergotamine, ergotamine analogs, including, for example, acetergamine, brazergoline, bromerguride, cianergoline, delorgotrile, disulergine, ergonovine maleate, ergotamine tartrate, etisulergine, lergotrile, lysergide, mesulergine, metergoline, metergotamine, nicergoline, pergolide, propisergide, proterguride, terguride); vasoactive intestinal peptides (such as, for example, peptide histidine isoleucine, peptide histidine methionine, substance P, calcitonin gene-related peptide, neurokinin A, bradykinin, neurokinin B, and the like); dopamine agonists (such as, for example, apomorphine, bromocriptine, testosterone, cocaine, strychnine, and the like); opioid antagonists (such as, for example, naltrexone, and the like); prostaglandins (such as, for example, alprostadil, prostaglandin E₂, prostaglandin F₂, misoprostol, enprostil, arbaprostil, unoprostone, trimoprostil, carboprost, limaprost, gemeprost, lantanoprost, omoprostil, beraprost, sulpostrone, rioprostil, and the like); endothelin antagonists (such as, for example, bosentan, sulfonamide endothelin antagonists, BQ-123, SQ 28608, and the like) and mixtures thereof.

In the present disclosure, methods are provided to prevent or treat diseases associated with or caused by the increased (pathological) metabolism of cyclic adenosine 3',5'-monophosphate (cAMP) or cyclic guanosine 3',5'-monophosphate (cGMP), including, for example, anosmia, hyposmia, ageusia, hypogeusia, hypertension, pulmonary hypertension, congestive heart failure, renal failure, myocardial infarction, stable, unstable and variant (Prinzmetal) angina, atherosclerosis, cardiac edema, renal insufficiency, nephrotic edema, hepatic edema, stroke, asthma, bronchitis, chronic obstructive pulmonary disease (COPD), cystic fibrosis, dementia including Alzheimer's disease, immunodeficiency, premature labor, Parkinson's disease, multiple sclerosis, dysmenorrhoea, benign prostatic hyperplasis (BPH), bladder outlet obstruction, incontinence, conditions of reduced blood vessel patency, *e.g.,* postpercutaneous transluminal coronary angioplasty (post-PTCA), peripheral vascular disease, allergic rhinitis, glaucoma, malignancies and diseases characterized by disorders of gut motility, *e.g.,* irritable bowel syndrome (IBS), rheumatoid arthritis, systemic lupus erythematosus, psoriasis, and other autoimmune diseases, Huntington's chorea, and Amyotrophic lateral sclerosis (ALS), by administering to a patient in need thereof a therapeutically effective amount of the compounds and/or compositions described herein.

In some cases, the intranasal, pulmonary or lingual administration of a PDE inhibitor can increase cell, tissue or organ levels of cAMP or cGMP. In some cases, the increase in cAMP or cGMP levels is at least 10%, 20%, 30%, 40%, 50%, 60%, 70%, 80%, 90%, 100%, 200%, 300%, 400%, 500% or 1000% over the untreated state. In other cases, cAMP or cGMP levels are increased to at least 10%, 20%, 30%, 40%, 50%, 60%, 70%, 80%, 90%, 100%, 125%, 150%, 200%, 300%, 400%, or 500% of the levels seen in controls, *e.g.,* normal individuals. In some cases, a method is provided for increasing nasal mucus or salivary cAMP or cGMP levels, wherein an effective amount of a PDE inhibitor is administered intranasally to a patient resulting in an increase in the cAMP or cGMP levels of at least 10%, 20%, 30%, 40%, 50%, 60%, 70%, 80%, 90%, 100%, 150%, 200%, 300%, 400%, 500%, or 1000% over the untreated level. In other cases, nasal mucus or salivary cAMP or cGMP levels are increased to at least 10%, 20%, 30%, 40%, 50%, 60%, 70%, 80%, 90%, 100%, 125%, 150%, 200%, 300%, 400%, or 500% of the levels seen in normal individuals.

In some cases, the administration of an effective amount of a PDE inhibitor by intranasal, lingual or pulmonary administration does not produce a detectable blood level of the PDE inhibitor. Theophylline can be measured, *e.g.,* by gas-liquid chromatography, high-performance liquid chromatrography, immunoassay, or enzyme immunoassay. Theophylline levels can be measured using commercially available test kits. In some cases, the administration of an effective amount of a PDE inhibitor by intranasal, lingual or pulmonary administration produces blood concentration of the PDE inhibitor that are less than 5 mg/dl, 2 mg/ dl, 1 mg/dl, 500 µg/dl, 250 µg/dl, 100 µg/dl, 50 µg/dl, 25 µg/dl, 10 µg/dl, 5 µg/dl, or 1 µg/dl.

In some cases, intranasal or lingual administration of an effective amount of a PDE inhibitor increases taste or smell acuity. In some cases, the increase in taste or smell acuity is at least 5%, 10%, 20%, 30%, 40%, 50%, 75%, or 100% compared to the untreated state. In other cases, taste or smell acuity is increased to at least 5%, 10%, 20%, 30%, 40%, 50%, 75%, or 100% of the acuity of normal individuals. In some cases, taste or smell acuity is measured objectively, while in other cases taste or smell acuity is measured subjectively.

When administered *in vivo,* the compounds and compositions of the present disclosure can be administered in combination with pharmaceutically acceptable carriers and in dosages described herein. The compounds and compositions of the present disclosure can be formulated as pharmaceutically acceptable neutral (free base) or salt forms. Pharmaceutically acceptable salts include, for example, those formed with free amino groups such as those derived from hydrochloric, hydrobromic, hydroiodide, phosphoric, sulfuric, acetic, citric, benzoic, fumaric, glutamic, lactic, malic, maleic, succinic, tartaric, p-toluenesulfonic, methanesulfonic acids, gluconic acid, and the like, and those formed with free carboxyl groups, such as those derived from sodium, potassium, ammonium, calcium, ferric hydroxides, isopropylamine, triethylamine, 2-ethylamino ethanol, histidine, procaine, and the like.

"Therapeutically effective amount" refers to the amount of a PDE inhibitor with or without additional agents that is effective to achieve its intended purpose. While individual patient needs may vary, determination of optimal ranges for effective amounts of each of the compounds and compositions is within the skill of an ordinary practitioner of the art. Generally, the dosage required to provide an effective amount of the composition, and which can be adjusted by one of ordinary skill in the art, will vary, depending on the age, health, physical condition, sex, weight, extent of the dysfunction of the recipient, frequency of treatment and the nature and scope of the dysfunction.

The amount of a given PDE inhibitor which will be effective in the prevention or treatment of a particular dysfunction or condition will depend on the nature of the dysfunction or condition, and can be determined by standard clinical techniques, including reference to Goodman and Gilman, supra; The Physician's Desk Reference, supra; Medical Economics Company, Inc., Oradell, N.J., 1995; and Drug Facts and Comparisons, Inc., St. Louis, Mo., 1993. The precise dose to be used in the formulation will also depend on the route of administration, and the seriousness of the dysfunction or disorder, and should be decided by the physician and the patient's circumstances.

The nasal and/or pulmonary administered PDE inhibitors can be used at dose ranges and over a course of dose regimen that are the same or substantially equivalent to those used for oral administration. The nasal and/or pulmonary administered PDE inhibitors can also be used in lower doses and in less extensive regimens of treatment. The amount of active ingredient that can be combined with one or more carrier material to produce a single dosage form will vary depending upon the host treated and the particular mode of administration.

Representative daily intranasal, lingual or pulmonary dosages can be, for example, from about 1.0 µg and 2000 mg per day, from about 1.0 µg and 500.0 mg per day, from about 10 µg and 100.0 mg per day, from about 10 µg and about 10 mg per day, from about 10 µg and 1.0 mg per day, from about 10 µg and 500 µg per day or from about 1 µg and 50 µg per day of the active ingredient comprising a preferred compound. These ranges of dosage amounts represent total dosage amounts of the active ingredient per day for a given patient. In some cases, the daily administered dose is less than 2000 mg per day, 1000 mg per day, 500 mg per day, 100 mg per day, 10 mg per day, 1.0 mg per day, 500 µg per day, 300 µg per day, 200 µg per day, 100 µg per day or 50 µg per day. In other cases, the daily administered dose is at least 2000 mg per day, 1000 mg per day, 500 mg per day, 100 mg per day, 10 mg per day, 1.0 mg per day, 500 µg per day, 300 µg per day, 200 µg per day, 100 µg per day or 50 µg per day. In some cases, on a per kilo basis, suitable dosage levels of the compounds will be from about 0.001 µg/kg and about 10.0 mg/kg of body weight per day, from about 0.5 µg/kg and about 0.5 mg/kg of body weight per day, from about 1.0 µg/kg and about 100 µg/kg of body weight per day, and from about 2.0 µg/kg and about 50 µg/kg of body weight per day of the active ingredient. In other cases, the suitable dosage level on a per kilo basis is less than 10.0 mg/kg of body weight per day, 1 mg/kg of body weight per day, 500 µg/kg of body weight per day, 100 µg/kg of body weight per day, 10 µg/kg of body weight per day of the active ingredient, or 1.0 µg/kg of body weight per day of active ingredient. In further cases, the suitable dosage level on a per kilo basis is at least 10.0 mg/kg of body weight per day, 1 mg/kg of body weight per day, 500 µg/kg of body weight per day, 100 µg/kg of body weight per day, 10 µg/kg of body weight per day of the active ingredient, or 1.0 µg/kg of body weight per day of active ingredient.

In some cases, the individual or single intranasal, lingual and/or pulmonary dose of the PDE inhibitors is less than 10 mg, less than 2 mg, less than 1 mg, less than 500 µg, less than 200 µg, less than 100 µg, or less than 50 µg per dosage unit or application. In other cases, the individual or single intranasal, lingual and/or pulmonary dose of the PDE inhibitors is at least 10 mg, 1 mg, 500 µg, 200 µg, 100 µg, 50 µg per dosage unit or application. In further cases, the individual or single intranasal, lingual and/or pulmonary dose of the PDE inhibitors ranges from 1 µg to 10 mg, 10 µ to 1 mg, 10 µg to 500 µg, 10 µg to 250 µg, 10 µg to 200 µg, 10 µg to 100 µg, 10 µg to 50 µg, 25 µg to 100 µg, 25 µg to 250 µg, 50 µg to 500 µg, or 100 µg to 1.0 mg

The number of times per day that a dose is administered will depend upon such pharmacological and pharmacokinetic factors as the half-life of the active ingredient, which reflects its rate of catabolism and clearance, as well as the minimal and optimal blood plasma or other body fluid levels of said active ingredient attained in the patient which are required for therapeutic efficacy. Typically, the PDE inhibitors are given once, twice, trice, or four times daily. PDE inhibitors may also be administered on a less frequent basis, such as every other day, every three, four, five, six or seven days.

Other factors may also be considered in deciding upon the number of doses per day and the amount of active ingredient per dose to be administered. Not the least important of such other factors is the individual response of the patient being treated. Thus, for example, where the active ingredient is used to treat or prevent asthma, and is administered loco-regionally via aerosol inhalation into the lungs, from one to four doses consisting of actuations of a dispensing device, e.g., "puffs" of an inhaler, may be administered each day, with each dose containing from about 10.0 µg to about 10.0 mg of active ingredient.

Effective doses can be extrapolated from dose-response curves derived from *in vitro* or animal model test systems and are in the same ranges or less than as described for the commercially available compounds in the Physician's Desk Reference, supra.

### Theophylline Formulations

Disclosed herein are pharmaceutical dosage units for intranasal administration comprising an amount of theophylline effective for treating anosmia, hyposmia, dysosmia, ageusia, hypogeusia, or dysgeusia in a human in need thereof, wherein the steroid-free dosage unit comprises less than 1 mg of the theophylline in a pharmaceutically acceptable liquid carrier that has a pH that is greater than 7.0. Some cases comprise less than 500 µg, less than 250 µg, or less than 100 µg of the theophylline. Some cases comprise from about 10 µg to about 500 µg of the theophylline. Some cases comprise from about 20 µg to about 100 µg of the theophylline. Some cases comprise about 20 µg of the theophylline. Some cases comprise about 100 µg of the theophylline.

Also disclosed herein are pharmaceutical dosage unit for intranasal administration comprising an amount of theophylline effective for treating anosmia, hyposmia, dysosmia, ageusia, hypogeusia, or dysgeusia in a human in need thereof, wherein the steroid-free dosage unit comprises theophylline at a dosage level that is less than about 16.7 µg/kg in a pharmaceutically acceptable liquid carrier that has a pH that is greater than 7.0. In some cases, the dosage level is less than about 8.3 µg/kg, less than about 4.2 µg/kg, or less than about 1.7 µg/kg. In some cases, the dosage level is from about 0.33 µg/kg to about 1.7 µg/kg. In some cases, the dosage level is about 0.33 µg/kg. In some cases, the dosage level is about 1.7 µg/kg.

In some cases, the pH is from 7.1 to 8.5. In some cases, the pH is from 7.1 to 7.4. In some cases, the pH is 7.1, 7.2, 7.3, 7.4, 7.5, 7.6, 7.7, 7.8, 7.9, 8, 8.1, 8.2, 8.3, 8.4, or 8.5. In some cases, the pH is at least 7.1, 7.2, 7.3, 7.4, 7.5, 7.6, 7.7, 7.8, 7.9, 8, 8.1, 8.2, 8.3, 8.4, or 8.5.

In some cases, a volume of the dosage unit is from about 50 µL to about 750 µL. In some cases, a volume of the dosage unit is from about 100 µL to about 400 µL. In some cases, a volume of the dosage unit is about: 50 µL, 75 µL, 100 µL, 125 µL, 150 µL, 175 µL, 200 µL, 225 µL, 250 µL, 275 µL, 300 µL, 325 µL, 350 µL, 375 µL, 400 µL, 425 µL, 450 µL, 475 µL, 500 µL, 525 µL, 550 µL, 575 µL, 600 µL, 625 µL, 650 µL, 675 µL, 700 µL, 725 µL or 750 µL.

The theophylline formulations and dosage units provided herein can be used in a nasal spray and be delivered in a plume. The plume can be characterized according to one or more parameters such as total volume, droplet size distribution, spray pattern, and plume geometry. The droplet size distribution can be characterized according to the percentage of droplets having a size of less than 10 µm, a D₁₀, a D₅₀, a D₉₀, a span, or a combination thereof. In a plume, 10% of the droplets have a size less than the D₁₀, 50% of the droplets have a size less than the D₅₀, and 90% of the droplets have a size less than the D₉₀. The span can be calculated from these numbers according to the following formula: span = (D₉₀ - D₁₀)/D₅₀. Spray pattern measures the ovality of the spray, which can be calculated from the ratio of maximum to minimum cross sections diameter of the plume at a distance from the spray device. Plume geometry measures the plume angle at the origin of the plume. Plume geometry can be measured at two distances from the origin of the plume, for example, at two side views 90° relative to each other. Plume geometry can also be calculated from the spray pattern.

The volume of the plume, the droplet size distribution, the spray pattern and plume geometry can all effect the treatment efficacy of active ingredients delivered by nasal spray. The formulations disclosed herein can be optimized to form a plume in the appropriate device that increases the efficacy of treatment in comparison to oral administration or intranasal administration with a syringe.

Optimal droplet sizes for a plume can be those that ensure the maximum amount of active ingredient is applied to the nasal epithelium. Minimizing the amount of very small droplets in the plume can reduce the amount of the plume that enters into the esophagus or lungs. This can reduce or eliminates side effects and ensure maximal delivery to the intended site of action. Minimizing the amount of larger drops can prevent loss of the active ingredient due to dripping out of the nose. Larger drops can also result in the formulation dripping into the back of the throat, which can cause irritation and delivery of active ingredient to undesired regions.

In some cases, the dosage unit is in a plume that has a droplet size distribution characterized by one or more of the following: (a) less than 5% of the droplets in the plume having a size of less than 10 µm, (b) a D₁₀ of greater than 12.5 µm, wherein 10% of the droplets in the plume have a size less than the D₁₀, (c) a D₅₀ of from 30 to 70 µm, wherein 50% of the droplets in the plume have a size less than the D₅₀, (d) a D₉₀ of less than 200 µm, wherein 90% of the droplets in the plume have a size less than the D₉₀, and (e) a span of from 1 to 6, wherein the span is calculated according to: (D₉₀ - D₁₀)/D₅₀.

In some cases, the plume is characterized by less than 4% of the droplets in the plume having a size of less than 10 µm. In some cases, the plume is characterized by less than 3% of the droplets in the plume having a size of less than 10 µm. In some cases, the plume is characterized by less than 2% of the droplets in the plume having a size of less than 10 µm.

In some cases, the plume is characterized by the D₁₀ that is greater than 15 µm. In some cases, the plume is characterized by the D₁₀ that is greater than 17.5 µm. In some cases, the plume is characterized by the D₁₀ that is from 12.5 µm to 30 µm. In some cases, the plume is characterized by the D₁₀ that is from 15 µm to 25 µm.

In some cases, the plume is characterized by the D₅₀ that is from 40 µm to 60 µm. In some cases, the plume is characterized by the D₅₀ that is from 30 µm to 60 µm. In some cases, the plume is characterized by the D₅₀ that is from 30 µm to 50 µm. In some cases, the plume is characterized by the D₅₀ that is from 30 µm to 40 µm. In some cases, the plume is characterized by the D₅₀ that is about: 30 µm, 32.5 µm, 35 µm, 37.5 µm, 40 µm, 42.5 µm, 45 µm, 50 µm, 55 µm, 60 µm, 65 µm, or 70 µm.

In some cases, the plume is characterized by the D₉₀ that is less than 175 µm. In some cases, the plume is characterized by the D₉₀ that is less than 150 µm. In some cases, the plume is characterized by the D₉₀ that is less than 125 µm. In some cases, the plume is characterized by the D₉₀ that is less than 100 µm. In some cases, the plume is characterized by the D₉₀ that is less than 90 µm. In some cases, the plume is characterized by the D₉₀ that is from 75 µm to 199 µm. In some cases, the plume is characterized by the D₉₀ that is from 75 µm to 175 µm. In some cases, the plume is characterized by the D₉₀ that is from 75 µm to 150 µm. In some cases, the plume is characterized by the D₉₀ that is from 75 µm to 125 µm. In some cases, the plume is characterized by the D₉₀ that is from 75 µm to 100 µm. In some cases, the plume is characterized by the D₉₀ that is about: 75 µm, 80 µm, 85 µm, 90 µm, 100 µm, 110 µm, 120 µm, 130 µm, 140 µm, 150 µm, 160 µm, 170 µm, 180 µm, or 190µm.

In some cases, the plume is characterized by the span that is from 1 to 5. In some cases, the plume is characterized by the span that is from 1 to 4. In some cases, the plume is characterized by the span that is from 1 to 3. In some cases, the plume is characterized by the span that is from 1 to 2. In some cases, the plume is characterized by the span that is about: 1, 1.1, 1.2, 1.3, 1.4, 1.5, 1.6, 1.7, 1.8, 1.9, 2, 2.1, 2.2, 2.3, 2.4, 2.5, 2.6, 2.7, 2.7, 2.9, 3, 3.25, 3.5, 3.75, 4, 4.5, 5, 5.5, or 6.

The spray pattern and geometry of the plume can affect the efficacy of treatment. For example, an irregular shape can result in uneven coating of the nasal epithelium and a resulting reduction in therapeutic efficacy. In another example, too narrow of a plume can reduce the area of the nasal epithelium that is coated by the plume. Conversely, too wide of a plume can direct the plume towards unintended targets such as the back of the throat.

In some cases, the plume is further characterized by having an ovality of from 0.7 to 1. In some cases, the plume is further characterized by having an ovality of from 0.8 to 1. In some cases, the plume is further characterized by having an ovality of from 0.9 to 1. In some cases, the plume is further characterized by having an ovality of about 1.

In some cases, the plume is further characterized by having a geometry of from 30° to 90°. In some cases, the plume is further characterized by having a geometry of from 45° to 75°. In some cases, the plume is further characterized by having a geometry of about: 30°, 35°, 40°, 45°, 50°, 55°, 60°, 65°, 70°, 75°, 80°, 85°, or 90°.

In some cases, the dosage unit is provided as a nasal spray or aerosol. In some cases, about 50% of droplets in the nasal spray or aerosol have a size of from about 15 µm to about 150 µm, from about 20 µm to about 100 µm, or from about 30 µm to about 70 µm. In some cases, less than about 20%, 15%, 10%, 9%, 8%, 7%, 6%, 5%, 4%, 3%, 2%, or 1% of droplets in the nasal spray or aerosol have a size of less than about 10 µm. In some cases, at least about 60%, 70%, 80%, 85%, 90%, or 95% of droplets in the nasal spray or aerosol have a size of less than about 200 µm. In some cases, at least about 50%, 60%, 70%, 80%, 90%, 95%, or 99% of droplets in the nasal spray or aerosol have a size of from about 10 µm to about 200 µm.

The pharmaceutically acceptable carrier and excipients in a nasal spray formulation can affect properties of the liquid, such as viscosity and osmolarity. This can effect the droplet size distribution of the plume, plume geometry and shape, and ultimately the bioavailability of the active ingredient.

In some cases, the pharmaceutically acceptable liquid carrier comprises aminoboronic acid and its derivatives, amastatin, surfactants, bile salts, gelified insulin, bioadhesive microspheres, phospholipids, chitosan nanoparticles, alkyl glycerides, or a combination thereof.

In some cases, the pharmaceutically acceptable liquid carrier comprises water, a buffering agent, a flavoring agent, a humectant, a penetration enhancer, a pH adjusting agent, a preservative, a solvent or co-solvent, a surfactant, a tonicity adjusting agent, a viscosity adjusting agent, or a combination thereof. In some cases, the pharmaceutically acceptable liquid carrier comprises water. Water can be filtered water, deionized water, distilled water, soft water, hard water, city water.

Some cases comprise the buffering agent that is potassium phosphate, sodium acetate, sodium citrate, sodium phosphate, trisodium citrate, or a combination thereof. In some cases, the pharmaceutically acceptable liquid carrier comprises or further comprises from about 0.00001% to about 0.0006% w/w trisodium citrate.

Some cases comprise or further comprise the flavoring agent that is menthol, saccharin sodium, sorbitol, or a combination thereof.

Some cases comprise or further comprise the humectant that is glycerin, propylene glycol, hexylene glycol, butylenes glycol, glyceryl triacetate, vinyl alcohol, neoagarobiose, glycerol, sorbitol, xylitol, maltitol, polydextrose, quillaia, lactic acid, urea, or aloe vera. Some cases comprise or further comprise the humectant that is propylene glycol. In some cases, the pharmaceutically acceptable liquid carrier comprises or further comprises from about 0.01% to about 0.233% w/w glycerin. In some cases, the pharmaceutically acceptable liquid carrier comprises or further comprises from about 0.1% to about 5% w/w glycerol. In some cases, the pharmaceutically acceptable liquid carrier comprises or further comprises from about 0.5% to about 10% w/w sorbitol.

Some cases comprise the penetration enhancer that is oleic acid. In some cases, the pharmaceutically acceptable liquid carrier comprises or further comprises from about 0.01% to about 0.132% w/w oleic acid.

Some cases comprise the pH adjusting agent that is acetic acid, citric acid, hydrochloric acid, sodium hydroxide, sulfuric acid, or a combination thereof. In some cases, the pharmaceutically acceptable liquid carrier comprises or further comprises from about 0.01% to about 0.5% w/w acetic acid. In some cases, the pharmaceutically acceptable liquid carrier comprises or further comprises from about 0.01% to about 0.5% w/w citric acid. In some cases, the pharmaceutically acceptable liquid carrier comprises or further comprises from about 0.01% to about 0.4% w/w sulfuric acid.

Some cases comprise the preservative that is benzalkonium chloride, benzethonium chloride, benzyl alcohol, butylated hydroxy toluene, butylated hydroxyanisole, chlorobutanol, edetate disodium, methylparaben, phenylethyl alcohol, phenylmercuric acetate, propylene paraben, propylparaben, thimerosal, or a combination thereof. Some cases comprise the preservative that is methylparaben, propylparaben, or a combination thereof. In some cases, the pharmaceutically acceptable liquid carrier comprises or further comprises from about 0.001% to about 0.119% w/w benzalkonium chloride. In some cases, the pharmaceutically acceptable liquid carrier comprises or further comprises from about 0.001% to about 0.0366% w/w benzyl alcohol. In some cases, the pharmaceutically acceptable liquid carrier comprises or further comprises from about 0.001% to about 0.01% w/w butylated hydroxy toluene. In some cases, the pharmaceutically acceptable liquid carrier comprises or further comprises from about 0.00001% to about 0.0002% w/w butylated hydroxyanisole. In some cases, the pharmaceutically acceptable liquid carrier comprises or further comprises from about 0.01% to about 0.5% w/w chlorobutanol. In some cases, the pharmaceutically acceptable liquid carrier comprises or further comprises from about 0.01% to about 0.5% w/w edetate disodium. In some cases, the pharmaceutically acceptable liquid carrier comprises or further comprises from about 0.01% to about 0.254% w/w phenylethyl alcohol. In some cases, the pharmaceutically acceptable liquid carrier comprises or further comprises from about 0.001% to about 0.1% w/w propylene paraben. In some cases, the pharmaceutically acceptable liquid carrier comprises or further comprises from about 0.001% to about 0.7% w/w methylparaben. In some cases, the pharmaceutically acceptable liquid carrier comprises or further comprises from about 0.01% to about 0.3% w/w propylparaben.

Some cases comprise the solvent or co-solvent that is ethanol, glycerol, glyceryl dioleate, glycine, polyethylene glycol (PEG), PEG 400, propylene glycol, triglycerides, or a combination thereof. Some cases comprise the solvent or co-solvent that is propylene glycol. In some cases, the pharmaceutically acceptable liquid carrier comprises or further comprises from about 0.1% to about 2% w/w ethanol. In some cases, the pharmaceutically acceptable liquid carrier comprises or further comprises from about 0.1% to about 5% w/w glycerol. In some cases, the pharmaceutically acceptable liquid carrier comprises or further comprises from about 1% to about 15% w/w glyceryl dioleate. In some cases, the pharmaceutically acceptable liquid carrier comprises or further comprises from about 0.1% to about 20% w/w PEG 400. In some cases, the pharmaceutically acceptable liquid carrier comprises or further comprises from about 0.1% to about 5% w/w polyethylene glycol (PEG). In some cases, the pharmaceutically acceptable liquid carrier comprises or further comprises from about 0.1% to about 5% w/w triglycerides. In some cases, the pharmaceutically acceptable liquid carrier comprises or further comprises from about 0.1% to about 20% w/w propylene glycol.

Some cases comprise the surfactant that is glyceryl monoleate, lecithin, PEG 3500, PEG 400, polyoxyl 400 stearate, polysorbate 20, polysorbate 80, propylene glycol, triglycerides, or a combination thereof. In some cases, the pharmaceutically acceptable liquid carrier comprises or further comprises from about 1% to about 10% w/w glyceryl monoleate. In some cases, the pharmaceutically acceptable liquid carrier comprises or further comprises from about 0.1% to about 10% w/w lecithin. In some cases, the pharmaceutically acceptable liquid carrier comprises or further comprises from about 0.1% to about 1.5% w/w PEG 3500. In some cases, the pharmaceutically acceptable liquid carrier comprises or further comprises from about 0.1% to about 20% w/w PEG 400. In some cases, the pharmaceutically acceptable liquid carrier comprises or further comprises from about 0.1% to about 15% w/w polyoxyl 400 stearate. In some cases, the pharmaceutically acceptable liquid carrier comprises or further comprises from about 0.01% to about 2.5% w/w polysorbate 20. In some cases, the pharmaceutically acceptable liquid carrier comprises or further comprises from about 0.1% to about 10% w/w polysorbate 80. In some cases, the pharmaceutically acceptable liquid carrier comprises or further comprises from about 0.1% to about 20% w/w propylene glycol.

Some cases comprise the tonicity adjusting agent that is dextrose, potassium chloride, sodium chloride, or a combination thereof. Some cases comprise the tonicity adjusting agent that is sodium chloride. In some cases, the pharmaceutically acceptable liquid carrier comprises or further comprises from about 0.01% to about 0.5% w/w dextrose. In some cases, the pharmaceutically acceptable liquid carrier comprises or further comprises from about 0.1% to about 1.9% w/w potassium chloride. In some cases, the pharmaceutically acceptable liquid carrier comprises or further comprises from about 0.1% to about 1.9% w/w sodium chloride.

Some cases comprise the viscosity adjusting agent that is carboxymethyl cellulose (CMC), Me-OH-Pr cellulose, microcrystalline cellulose (MCC), sodium carboxymethyl cellulose (Na CMC), or a combination thereof. In some cases, the pharmaceutically acceptable liquid carrier comprises or further comprises from about 0.1% to about 2% w/w carboxymethyl cellulose. In some cases, the pharmaceutically acceptable liquid carrier comprises or further comprises from about 0.1% to about 5% w/w Me-OH-Pr cellulose. In some cases, the pharmaceutically acceptable liquid carrier comprises or further comprises from about 0.1% to about 2% w/w microcrystalline cellulose. In some cases, the pharmaceutically acceptable liquid carrier comprises or further comprises from about 0.1% to about 5% w/w sodium carboxymethyl cellulose (Na CMC).

In some cases, the pharmaceutically acceptable liquid carrier comprises or further comprises from about 0.001% to about 0.7% w/w methylparaben, from about 0.01% to about 0.3% w/w propylparaben, from about 0.1% to about 20% w/w propylene glycol, from about 0.1% to about 1.9% w/w sodium chloride, or a combination thereof. In some cases, the pharmaceutically acceptable liquid carrier comprises or further comprises from about 0.001% to about 0.7% w/w methylparaben, from about 0.01% to about 0.3% w/w propylparaben, from about 0.1% to about 20% w/w propylene glycol, and from about 0.1% to about 1.9% w/w sodium chloride.

The osmolarity of an intranasal dosage unit can affect the rate at which an active ingredient is absorbed by the nasal epithelium. For example, a higher permeability of the active ingredient can be seen in hypotonic nasal sprays. In some cases, the dosage unit has an osmolarity of from 10 mOsm/L to 1.2 Osm/L. For example, the dosage unit can have an osmolarity of 10-1200mOsm/L, 10-750mOsm/L, 10-500mOsm/L, 10-300mOsm/L, 10-250mOsm/L, 10-150mOsm/L, 10-100mOsm/L, 10-50mOsm/L, 50-1200mOsm/L, 50-750mOsm/L, 50-500mOsm/L, 50-300mOsm/L, 50-250mOsm/L, 50-150mOsm/L, 50-100mOsm/L, 100-1200mOsm/L, 100-750mOsm/L, 100-500mOsm/L, 100-300mOsm/L, 100-250mOsm/L, 100-150mOsm/L, 150-1200mOsm/L, 150-750mOsm/L, 150-500mOsm/L, 150-300mOsm/L, 150-250mOsm/L, 250-1200mOsm/L, 250-750mOsm/L, 250-500mOsm/L, 250-300mOsm/L, 300-1200mOsm/L, 300-750mOsm/L, 300-500mOsm/L, 500-1200mOsm/L, 500-750mOsm/L, or 750-1200mOsm/L.

In some cases, the dosage unit has an osmolarity of from 10 mOsm/L to 295 mOsm/L. In some cases, the dosage unit has an osmolarity of from 295 mOsm/L to 1.2 mOsm/L. In some cases, the dosage unit has an osmolarity of from 200 mOsm/L to 400 mOsm/L.

In some cases, the dosage unit has an osmolarity of greater than 10 mOsm/L, 25 mOsm/L, 50 mOsm/L, 75 mOsm/L, 100 mOsm/L, 125 mOsm/L, 150 mOsm/L, 175 mOsm/L, 200 mOsm/L, 225 mOsm/L, 250 mOsm/L, 300 mOsm/L, 400 mOsm/L, 500 mOsm/L, 750 mOsm/L, or 1000 mOsm/L.

In some cases, the dosage unit has an osmolarity of less than 1200 mOsm/L, 1000 mOsm/L, 750 mOsm/L, 500 mOsm/L, 300 mOsm/L, 275 mOsm/L, 250 mOsm/L, 225 mOsm/L, 200 mOsm/L, 175 mOsm/L, 150 mOsm/L, 125 mOsm/L, 100 mOsm/L, 75 mOsm/L, 50 mOsm/L, or 25 mOsm/L.

The viscosity of an intranasal dosage unit can affect the residence time in the nasal cavity. In some cases, the dosage unit has a higher kinematic viscosity than water at the same temperature. In some cases, the dosage unit has a kinematic viscosity of from 0.5 cSt to 2 cSt at 20 °C. For example, the dosage unit can have a kinematic viscosity of 0.5-2 cSt, 0.5-1.5 cSt, 0.5-1.25 cSt, 0.5-1.1 cSt, 0.5-1 cSt, 0.5-0.9 cSt, 0.5-0.75 cSt, 0.75-2 cSt, 0.75-1.5 cSt, 0.75-1.25 cSt, 0.75-1.1 cSt, 0.75-1 cSt, 0.75-0.9 cSt, 0.9-2 cSt, 0.9-1.5 cSt, 0.9-1.25 cSt, 0.9-1.1 cSt, 0.9-1 cSt, 1-2 cSt, 1-1.5 cSt, 1-1.25 cSt, 1-1.1 cSt, 1.1-2 cSt, 1.1-1.5 cSt, 1.1-1.25 cSt, 1.25-2 cSt, 1.25-1.5 cSt, or 1.5-2 cSt at 20 °C. In some cases, the dosage unit has a kinematic viscosity of 0.9-1.25 cSt at 20 °C. In some cases, the dosage unit has a higher kinematic viscosity than water at the same temperature.

The dosage units disclosed herein can be stable for from 1 month to 5 years or more at room temperature.

Also disclosed are methods of treating anosmia, hyposmia, dysosmia, ageusia, hypogeusia, or dysgeusia in a subject in need thereof comprising administering to the subject in need thereof any of the pharmaceutical dosage units disclosed herein. In some cases, the pharmaceutical dosage unit is administered once daily to each naris. In some cases, the pharmaceutical dosage unit is administered twice daily to each naris.

In some cases, the pharmaceutical dosage unit is administered each day for from about 7 days to at least about 365 days. In some cases, the pharmaceutical dosage unit is administered each day for from about 7 days to about 6 months. In some cases, the pharmaceutical dosage unit is administered each day for from about 7 days to about 4 months. In some cases, the pharmaceutical dosage unit is administered each day for from about 1 month to about 12 months.

In some cases, the subject experiences a decrease in a detection threshold (DT) score, a decrease in a recognition threshold (RT) score, an increase in a magnitude estimation (ME) score, and/or a change in a hedonic (H) score as measured with a forced-choice, three-stimuli, stepwise-staircase technique using one or more odorants after administering the pharmaceutical dosage unit to the subject. In some cases, the one or more odorants comprise pyridine, nitrobenzene, thiophene, amyl acetate, or a combination thereof.

In some cases, the subject experiences a decrease in an taste detection threshold (DT) score, a decrease in a recognition threshold (RT) score, an increase in a magnitude estimation (ME) score, and/or a change in a hedonic (H) score as measured with a forced-choice, three-stimuli, stepwise-staircase technique using one or more tastants testing compounds after administering the pharmaceutical dosage unit to the subject. In some cases, the one or more tastants comprise sodium chloride (NaCl), sucrose, hydrogen chloride (HCl), urea, or a combination thereof.

Also disclosed are kits for the treatment of anosmia, hyposmia, dysosmia, ageusia, hypogeusia, or dysgeusia comprising: (a) a multi-dose nasal spray device that delivers any of the pharmaceutical dosage units disclosed herein; and (b) instructions for use.

### Combination PDE Inhibitor Formulations

Some subjects can be resistant to one or more PDE inhibitors. Combination formulations can be used in such subjects to increase treatment efficacy. Combination formulations can also be used in a wider spectrum of subjects.

Disclosed herein are pharmaceutical dosage units for intranasal administration comprising: a first amount of a non-selective PDE inhibitor; a second amount of a second PDE inhibitor that is a PDE 1 inhibitor, a PDE2 inhibitor, a PDE3 inhibitor, a PDE 4 inhibitor, a PDE 5 inhibitor, or a PDE 10 inhibitor; and a pharmaceutically acceptable liquid carrier; wherein the first amount and the second amount are effective in combination to treat hyposmia or anosmia in a subject in need thereof, wherein the subject is resistant to treatment with either the non-selective PDE inhibitor or the second PDE inhibitor alone. In some cases, the non-selective PDE inhibitor is caffeine, aminophylline, paraxanthine, pentoxifylline, theobromine, theophylline, or oxphylline. In some cases, the non-selective PDE inhibitor is theophylline.

In some cases, the second PDE inhibitor is the PDE 1 inhibitor that is vinpocetine. In some cases, the second PDE inhibitor is the PDE 2 inhibitor that is EHNA. In some cases, the second PDE inhibitor is the PDE 3 inhibitor that is inamrinone, anagrelide, or cilostazol. In some cases, the second PDE inhibitor is the PDE 4 inhibitor that is mesembrine, rolipram, ibudilast, pclamilast, luteolin, drotaverine, or roflumilast. In some cases, the second PDE inhibitor is the PDE 5 inhibitor that is sildenafil, tadalafil, vardenafil, udenafil, avanafil, or dipyridamole. In some cases, the second PDE inhibitor is the PDE 10 inhibitor that is papaverine. In some cases, the first amount and second amount are indicated in Table XIV.

Some cases comprise a third amount of a third PDE inhibitor that is optionally in a different class of PDE inhibitor from the non-selective PDE inhibitor and the second PDE inhibitor. In some cases, the third amount is indicated in Table XIV.

**Table XIV. PDE inhibitors for combination formulations.**

| **PDE Inhibitors for Combination Formulations** | **Exemplary Intranasal Dose in µg** | **Exemplary Intranasal Dose (µg/kg)** |
|---|---|---|
| **Nonselective PDE Inhibitors** | | |
| Methylated xanthines and derivatives: | | |
| Caffeine | 20 - 100 | 0.33 - 1.7 |
| Aminophylline | 20 - 100 | 0.33 - 1.7 |
| Paraxanthine | 10 - 100 | 0.17 - 1.7 |
| Pentoxifylline | 20 - 100 | 0.33 - 1.7 |
| Theobromine | 30 - 150 | 0.50 - 2.5 |
| Theophylline | 20 - 100 | 0.33 - 1.7 |
| Oxphylline | 10 - 100 | 0.17 - 1.7 |
| **PDE1 -** Vinpocetine | --- | |
| | | |
| **PDE2** - EHNA | --- | |
| | | |

| **PDE3** | | |
|---|---|---|
| Inamrinone | 2 - 40 | 0.03 - 0.67 |
| Anagrelide | 2 - 40 | 0.03 - 0.67 |
| Cilostazol | 2 - 40 | 0.03 - 0.67 |
| | | |

| **PDE4** | | |
|---|---|---|
| Mesembrine | 1 - 10 | 0.02 - 0.33 |
| Rolipram | 1 - 10 | 0.02 - 0.33 |
| Ibudilast | 1 - 10 | 0.02 - 0.33 |
| Piclamilast | 1 - 10 | 0.02 - 0.33 |
| Luteolin | 1 - 10 | 0.02 - 0.33 |
| Drotaverine | 2 - 10 | 0.03 - 0.33 |
| Roflumilast | 1 - 10 | 0.02 - 0.33 |
| | | |

| **PDE5** | | |
|---|---|---|
| Sildenafil | 5 - 150 | 0.08 - 2.5 |
| Tadalafil | 5 - 150 | 0.08 - 2.5 |
| Vardenafil | 5 - 150 | 0.08 - 2.5 |
| Udenafil | 1 - 100 | 0.02 - 1.7 |
| Avanafil | 1 - 150 | 0.02 - 2.5 |
| Dipyridamole | 1 - 150 | 0.02 - 2.5 |
| | | |

| **PDE10** | | |
|---|---|---|
| Papaverine | 10 - 200 | 0.17 - 3.3 |

Disclosed are combination pharmaceutical dosage units for intranasal administration comprising: (a) a first amount of a non-selective PDE inhibitor; (b) a second amount of a second PDE inhibitor that is a PDE 1 inhibitor, a PDE2 inhibitor, a PDE3 inhibitor, a PDE 4 inhibitor, a PDE 5 inhibitor, or a PDE 10 inhibitor; and (c) a pharmaceutically acceptable liquid carrier; wherein the first amount and the second amount are effective in combination to treat anosmia, hyposmia, dysosmia, ageusia, hypogeusia, or dysgeusia in a subject in need thereof, wherein the subject is resistant to treatment with either the non-selective PDE inhibitor or the second PDE inhibitor alone.

In some cases, the non-selective PDE inhibitor is caffeine, aminophylline, paraxanthine, pentoxifylline, theobromine, theophylline, or oxphylline. In some cases, the non-selective PDE inhibitor is theophylline.

In some cases, the second PDE inhibitor is the PDE 1 inhibitor that is vinpocetine. In some cases, the second PDE inhibitor is the PDE 2 inhibitor that is EHNA. In some cases, the second PDE inhibitor is the PDE 3 inhibitor that is inamrinone, anagrelide, or cilostazol. In some cases, the second PDE inhibitor is the PDE 4 inhibitor that is mesembrine, rolipram, ibudilast, pclamilast, luteolin, drotaverine, or roflumilast. In some cases, the second PDE inhibitor is the PDE 5 inhibitor that is sildenafil, tadalafil, vardenafil, udenafil, avanafil, or dipyridamole. In some cases, the second PDE inhibitor is the PDE 10 inhibitor that is papaverine.

In some cases, the non-selective PDE inhibitor is caffeine and the first amount is from about 20 µg to about 100 µg per naris. In some cases, the non-selective PDE inhibitor is caffeine and the first amount is from about 0.33 µg/kg to about 1.7 µg/kg per naris.

In some cases, the non-selective PDE inhibitor is aminophylline and the first amount is from about 20 µg to about 100 µg per naris. In some cases, the non-selective PDE inhibitor is aminophylline and the first amount is from about 0.33 µg/kg to about 1.7 µg/kg per naris.

In some cases, the non-selective PDE inhibitor is paraxanthine and the first amount is from about 10 µg to about 100 µg per naris. In some cases, the non-selective PDE inhibitor is paraxanthine and the first amount is from about 0.17 µg/kg to about 1.7 µg/kg per naris.

In some cases, the non-selective PDE inhibitor is pentoxifylline and the first amount is from about 20 µg to about 100 µg per naris. In some cases, the non-selective PDE inhibitor is pentoxifylline and the first amount is from about 0.33 µg/kg to about 1.7 µg/kg per naris.

In some cases, the non-selective PDE inhibitor is theobromine and the first amount is from about 30 µg to about 150 µg per naris. In some cases, the non-selective PDE inhibitor is theobromine and the first amount is from about 0.5 µg/kg to about 2.5 µg/kg per naris.

In some cases, the non-selective PDE inhibitor is theophylline and the first amount is from about 20 µg to about 100 µg per naris. In some cases, the non-selective PDE inhibitor is theophylline and the first amount is from about 0.33 µg/kg to about 1.7 µg/kg per naris.

In some cases, the non-selective PDE inhibitor is oxphylline and the first amount is from about 100 µg to about 100 µg per naris. In some cases, the non-selective PDE inhibitor is oxphylline and the first amount is from about 0.17 µg/kg to about 1.7 µg/kg per naris.

In some cases, the second PDE inhibitor is inamrinone and the second amount is from about 2 µg to about 40 µg per naris. In some cases, the second PDE inhibitor is inamrinone and the second amount is from about 0.03 µg/kg to about 0.67 µg/kg per naris.

In some cases, the second PDE inhibitor is anagrelide and the second amount is from about 2 µg to about 40 µg per naris. In some cases, the second PDE inhibitor is anagrelide and the second amount is from about 0.03 µg/kg to about 0.67 µg/kg per naris.

In some cases, the second PDE inhibitor is cilostazol and the second amount is from about 2 µg to about 40 µg per naris. In some cases, the second PDE inhibitor is cilostazol and the second amount is from about 0.03 µg/kg to about 0.67 µg/kg per naris.

In some cases, the second PDE inhibitor is mesembrine and the second amount is from about 1 µg to about 10 µg per naris. In some cases, the second PDE inhibitor is mesembrine and the second amount is from about 0.02 µg/kg to about 0.33 µg/kg per naris.

In some cases, the second PDE inhibitor is rolipram and the second amount is from about 1 µg to about 10 µg per naris. In some cases, the second PDE inhibitor is rolipram and the second amount is from about 0.02 µg/kg to about 0.33 µg/kg per naris.

In some cases, the second PDE inhibitor is ibudilast and the second amount is from about 1 µg to about 10 µg per naris. In some cases, the second PDE inhibitor is ibudilast and the second amount is from about 0.02 µg/kg to about 0.33 µg/kg per naris.

In some cases, the second PDE inhibitor is piclamilast and the second amount is from about 1 µg to about 10 µg per naris. In some cases, the second PDE inhibitor is piclamilast and the second amount is from about 0.02 µg/kg to about 0.33 µg/kg per naris.

In some cases, the second PDE inhibitor is luteolin and the second amount is from about 1 µg to about 10 µg per naris. In some cases, the second PDE inhibitor is luteolin and the second amount is from about 0.02 µg/kg to about 0.33 µg/kg per naris.

In some cases, the second PDE inhibitor is drotaverine and the second amount is from about 2 µg to about 10 µg per naris. In some cases, the second PDE inhibitor is drotaverine and the second amount is from about 0.03 µg/kg to about 0.33 µg/kg per naris.

In some cases, the second PDE inhibitor is roflumilast and the second amount is from about 1 µg to about 10 µg per naris. In some cases, the second PDE inhibitor is roflumilast and the second amount is from about 0.02 µg/kg to about 0.33 µg/kg per naris.

In some cases, the second PDE inhibitor is sildenafil and the second amount is from about 5 µg to about 150 µg per naris. In some cases, the second PDE inhibitor is sildenafil and the second amount is from about 0.08 µg/kg to about 2.5 µg/kg per naris.

In some cases, the second PDE inhibitor is tadalafil and the second amount is from about 5 µg to about 150 µg per naris. In some cases, the second PDE inhibitor is tadalafil and the second amount is from about 0.08 µg/kg to about 2.5 µg/kg per naris.

In some cases, the second PDE inhibitor is vardenafil and the second amount is from about 5 µg to about 150 µg per naris. In some cases, the second PDE inhibitor is vardenafil and the second amount is from about 0.08 µg/kg to about 2.5 µg/kg per naris.

In some cases, the second PDE inhibitor is udenafil and the second amount is from about 1 µg to about 100 µg per naris. In some cases, the second PDE inhibitor is udenafil and the second amount is from about 0.02 µg/kg to about 1.7 µg/kg per naris.

In some cases, the second PDE inhibitor is avanafil and the second amount is from about 1 µg to about 150 µg per naris. In some cases, the second PDE inhibitor is avanafil and the second amount is from about 0.02 µg/kg to about 2.5 µg/kg per naris.

In some cases, the second PDE inhibitor is dipyridamole and the second amount is from about 1 µg to about 150 µg per naris. In some cases, the second PDE inhibitor is dipyridamole and the second amount is from about 0.02 µg/kg to about 2.5 µg/kg per naris.

In some cases, the second PDE inhibitor is papaverine and the second amount is from about 10 µg to about 200 µg per naris. In some cases, the second PDE inhibitor is avanafil and the second amount is from about 0.17 µg/kg to about 3.3 µg/kg per naris.

In some cases, the first amount and second amount are indicated in Table XIV.

Some cases further comprise a third amount of a third PDE inhibitor that is optionally in a different class of PDE inhibitor from the non-selective PDE inhibitor and the second PDE inhibitor. In some cases, the third amount is indicated in Table XIV.

Some cases comprise further amounts of further PDE inhibitor that is optionally in a different class of PDE inhibitor from the non-selective PDE inhibitor and the second PDE inhibitor and/or third PDE inhibitor. In some cases, the further amount(s) are indicated in Table XIV.

In some cases, the pharmaceutically acceptable liquid carrier has a pH that is greater than 7.0. In some cases, the pharmaceutically acceptable liquid carrier has a pH that is from 7.1 to 8.5. In some cases, the pharmaceutically acceptable liquid carrier has a pH that is from 7.1 to 7.4. In some cases, the pharmaceutically acceptable liquid carrier has a pH that is 7.1, 7.2, 7.3, 7.4, 7.5, 7.6, 7.7, 7.8, 7.9, 8, 8.1, 8.2, 8.3, 8.4, or 8.5. In some cases, the pharmaceutically acceptable liquid carrier has a pH that is at least 7.1, 7.2, 7.3, 7.4, 7.5, 7.6, 7.7, 7.8, 7.9, 8, 8.1, 8.2, 8.3, 8.4, or 8.5.

In some cases, a volume of the dosage unit is from about 50 µL to about 750 µL. In some cases, a volume of the dosage unit is from about 100 µL to about 400 µL. In some cases, a volume of the dosage unit is about: 50 µL, 75 µL, 100 µL, 125 µL, 150 µL, 175 µL, 200 µL, 225 µL, 250 µL, 275 µL, 300 µL, 325 µL, 350 µL, 375 µL, 400 µL, 425 µL, 450 µL, 475 µL, 500 µL, 525 µL, 550 µL, 575 µL, 600 µL, 625 µL, 650 µL, 675 µL, 700 µL, 725 µL or 750 µL.

The combination formulations and dosage units provided herein can be used in a nasal spray and be delivered in a plume. The plume can be characterized according to one or more parameters such as total volume, droplet size distribution, spray pattern, and plume geometry. The droplet size distribution can be characterized according to the percentage of droplets having a size of less than 10 µm, a D₁₀, a D₅₀, a D₉₀, a span, or a combination thereof. In a plume, 10% of the droplets have a size less than the D₁₀, 50% of the droplets have a size less than the D₅₀, and 90% of the droplets have a size less than the D₉₀. The span can be calculated from these numbers according to the following formula: span = (D₉₀ - D₁₀)/D₅₀. Spray pattern measures the ovality of the spray, which can be calculated from the ratio of maximum to minimum cross sections diameter of the plume at a distance from the spray device. Plume geometry measures the plume angle at the origin of the plume. Plume geometry can be measured at two distances from the origin of the plume, for example, at two side views 90° relative to each other. Plume geometry can also be calculated from the spray pattern.

The volume of the plume, the droplet size distribution, the spray pattern and plume geometry can all effect the treatment efficacy of active ingredients delivered by nasal spray. The formulations disclosed herein can be optimized to form a plume in the appropriate device that increases the efficacy of treatment in comparison to oral administration or intranasal administration with a syringe.

Optimal droplet sizes for a plume can be those that ensure the maximum amount of active ingredient is applied to the nasal epithelium. Minimizing the amount of very small droplets in the plume can reduce the amount of the plume that enters into the esophagus or lungs. This can reduce or eliminates side effects and ensure maximal delivery to the intended site of action. Minimizing the amount of larger drops can prevent loss of the active ingredient due to dripping out of the nose. Larger drops can also result in the formulation dripping into the back of the throat, which can cause irritation and delivery of active ingredient to undesired regions.

In some cases, the combination pharmaceutical dosage unit is in a plume that has a droplet size distribution characterized by one or more of the following: (a) less than 5% of the droplets in the plume having a size of less than 10 µm, (b) a D₁₀ of greater than 12.5 µm, wherein 10% of the droplets in the plume have a size less than the D₁₀, (c) a D₅₀ of from 30 to 70 µm, wherein 50% of the droplets in the plume have a size less than the D₅₀, (d) a D₉₀ of less than 200 µm, wherein 90% of the droplets in the plume have a size less than the D₉₀, and (e) a span of from 1 to 6, wherein the span is calculated according to: (D₉₀ - D₁₀)/D₅₀.

In some cases, the plume is characterized by the D₉₀ that is less than 175 µm. In some cases, the plume is characterized by the D₉₀ that is less than 150 µm. In some cases, the plume is characterized by the D₉₀ that is less than 125 µm. In some cases, the plume is characterized by the D₉₀ that is less than 100 µm. In some cases, the plume is characterized by the D₉₀ that is less than 90 µm. In some cases, the plume is characterized by the D₉₀ that is from 75 µm to 199 µm. In some cases, the plume is characterized by the D₉₀ that is from 75 µm to 175 µm. In some cases, the plume is characterized by the D₉₀ that is from 75 µm to 150 µm. In some cases, the plume is characterized by the D₉₀ that is from 75 µm to 125 µm. In some cases, the plume is characterized by the D₉₀ that is from 75 µm to 100 µm. In some cases, the plume is characterized by the D₉₀ that is about: 75 µm, 80 µm, 85 µm, 90 µm, 100 µm, 110 µm, 120 µm, 130 µm, 140 µm, 150 µm, 160 µm, 170 µm, 180 µm, or 190µm.

In some cases, the plume is characterized by the span that is from 1 to 5. In some cases, the plume is characterized by the span that is from 1 to 4. In some cases, the plume is characterized by the span that is from 1 to 3. In some cases, the plume is characterized by the span that is from 1 to 2. In some cases, the plume is characterized by the span that is about: 1, 1.1, 1.2, 1.3, 1.4, 1.5, 1.6, 1.7, 1.8, 1.9, 2, 2.1, 2.2, 2.3, 2.4, 2.5, 2.6, 2.7, 2.7, 2.9, 3, 3.25, 3.5, 3.75, 4, 4.5, 5, 5.5, or 6.
In some cases, the plume is further characterized by having an ovality of from 0.7 to 1. In some cases, the plume is further characterized by having an ovality of from 0.8 to 1. In some cases, the plume is further characterized by having an ovality of from 0.9 to 1. In some cases, the plume is further characterized by having an ovality of about 1.

The spray pattern and geometry of the plume can affect the efficacy of treatment. For example, an irregular shape can result in uneven coating of the nasal epithelium and a resulting reduction in therapeutic efficacy. In another example, too narrow of a plume can reduce the area of the nasal epithelium that is coated by the plume. Conversely, too wide of a plume can direct the plume towards unintended targets such as the back of the throat.

In some cases, the plume is further characterized by having a geometry of from 30° to 90°. In some cases, the plume is further characterized by having a geometry of from 45° to 75°. In some cases, the plume is further characterized by having a geometry of about: 30°, 35°, 40°, 45°, 50°, 55°, 60°, 65°, 70°, 75°, 80°, 85°, or 90°.

In some cases, the dosage unit is provided as a nasal spray or aerosol. In some cases, about 50% of droplets in the nasal spray or aerosol have a size of from about 15 µm to about 150 µm, from about 20 µm to about 100 µm, or from about 30 µm to about 70 µm. In some cases, less than about 20%, 15%, 10%, 9%, 8%, 7%, 6%, 5%, 4%, 3%, 2%, or 1% of droplets in the nasal spray or aerosol have a size of less than about 10 µm. In some cases, at least about 60%, 70%, 80%, 85%, 90%, or 95% of droplets in the nasal spray or aerosol have a size of less than about 200 µm. In some cases, at least about 50%, 60%, 70%, 80%, 90%, 95%, or 99% of droplets in the nasal spray or aerosol have a size of from about 10 µm to about 200 µm.

The pharmaceutically acceptable carrier and excipients in a nasal spray formulation can affect properties of the liquid, such as viscosity and osmolarity. This can effect the droplet size distribution of the plume, plume geometry and shape, and ultimately the bioavailability of the active ingredients.

In some cases, the pharmaceutically acceptable liquid carrier comprises aminoboronic acid and its derivatives, amastatin, surfactants, bile salts, gelified insulin, bioadhesive microspheres, phospholipids, chitosan nanoparticles, alkyl glycerides, or a combination thereof.

In some cases, the pharmaceutically acceptable liquid carrier comprises water, a buffering agent, a flavoring agent, a humectant, a penetration enhancer, a pH adjusting agent, a preservative, a solvent or co-solvent, a surfactant, a tonicity adjusting agent, a viscosity adjusting agent, or a combination thereof. In some cases, the pharmaceutically acceptable liquid carrier comprises water.

Some cases comprise the buffering agent that is potassium phosphate, sodium acetate, sodium citrate, sodium phosphate, trisodium citrate, or a combination thereof. In some cases, the pharmaceutically acceptable liquid carrier comprises or further comprises from about 0.00001% to about 0.0006% w/w trisodium citrate.

Some cases comprise or further comprise the flavoring agent that is menthol, saccharin sodium, sorbitol, or a combination thereof.

Some cases comprise or further comprise the humectant that is glycerin, propylene glycol, hexylene glycol, butylenes glycol, glyceryl triacetate, vinyl alcohol, neoagarobiose, glycerol, sorbitol, xylitol, maltitol, polydextrose, quillaia, lactic acid, urea, or aloe vera. Some cases comprise or further comprise the humectant that is propylene glycol. In some cases, the pharmaceutically acceptable liquid carrier comprises or further comprises from about 0.01% to about 0.233% w/w glycerin. In some cases, the pharmaceutically acceptable liquid carrier comprises or further comprises from about 0.1% to about 5% w/w glycerol. In some cases, the pharmaceutically acceptable liquid carrier comprises or further comprises from about 0.5% to about 10% w/w sorbitol.

Some cases comprise the penetration enhancer that is oleic acid. In some cases, the pharmaceutically acceptable liquid carrier comprises or further comprises from about 0.01% to about 0.132% w/w oleic acid.

Some cases comprise the pH adjusting agent that is acetic acid, citric acid, hydrochloric acid, sodium hydroxide, sulfuric acid, or a combination thereof. In some cases, the pharmaceutically acceptable liquid carrier comprises or further comprises from about 0.01% to about 0.5% w/w acetic acid. In some cases, the pharmaceutically acceptable liquid carrier comprises or further comprises from about 0.01% to about 0.5% w/w citric acid. In some cases, the pharmaceutically acceptable liquid carrier comprises or further comprises from about 0.01% to about 0.4% w/w sulfuric acid.

Some cases comprise the preservative that is benzalkonium chloride, benzethonium chloride, benzyl alcohol, butylated hydroxy toluene, butylated hydroxyanisole, chlorobutanol, edetate disodium, methylparaben, phenylethyl alcohol, phenylmercuric acetate, propylene paraben, propylparaben, thimerosal, or a combination thereof. Some cases comprise the preservative that is methylparaben, propylparaben, or a combination thereof. In some cases, the pharmaceutically acceptable liquid carrier comprises or further comprises from about 0.001% to about 0.119% w/w benzalkonium chloride. In some cases, the pharmaceutically acceptable liquid carrier comprises or further comprises from about 0.001% to about 0.0366% w/w benzyl alcohol. In some cases, the pharmaceutically acceptable liquid carrier comprises or further comprises from about 0.001% to about 0.01% w/w butylated hydroxy toluene. In some cases, the pharmaceutically acceptable liquid carrier comprises or further comprises from about 0.00001% to about 0.0002% w/w butylated hydroxyanisole. In some cases, the pharmaceutically acceptable liquid carrier comprises or further comprises from about 0.01% to about 0.5% w/w chlorobutanol. In some cases, the pharmaceutically acceptable liquid carrier comprises or further comprises from about 0.01% to about 0.5% w/w edetate disodium. In some cases, the pharmaceutically acceptable liquid carrier comprises or further comprises from about 0.01% to about 0.254% w/w phenylethyl alcohol. In some cases, the pharmaceutically acceptable liquid carrier comprises or further comprises from about 0.001% to about 0.1% w/w propylene paraben. In some cases, the pharmaceutically acceptable liquid carrier comprises or further comprises from about 0.001% to about 0.7% w/w methylparaben. In some cases, the pharmaceutically acceptable liquid carrier comprises or further comprises from about 0.01% to about 0.3% w/w propylparaben.

Some cases comprise the solvent or co-solvent that is ethanol, glycerol, glyceryl dioleate, glycine, polyethylene glycol (PEG), PEG 400, propylene glycol, triglycerides, or a combination thereof. Some cases comprise the solvent or co-solvent that is propylene glycol. In some cases, the pharmaceutically acceptable liquid carrier comprises or further comprises from about 0.1% to about 2% w/w ethanol. In some cases, the pharmaceutically acceptable liquid carrier comprises or further comprises from about 0.1% to about 5% w/w glycerol. In some cases, the pharmaceutically acceptable liquid carrier comprises or further comprises from about 1% to about 15% w/w glyceryl dioleate. In some cases, the pharmaceutically acceptable liquid carrier comprises or further comprises from about 0.1% to about 20% w/w PEG 400. In some cases, the pharmaceutically acceptable liquid carrier comprises or further comprises from about 0.1% to about 5% w/w polyethylene glycol (PEG). In some cases, the pharmaceutically acceptable liquid carrier comprises or further comprises from about 0.1% to about 5% w/w triglycerides. In some cases, the pharmaceutically acceptable liquid carrier comprises or further comprises from about 0.1% to about 20% w/w propylene glycol.

Some cases comprise the surfactant that is glyceryl monoleate, lecithin, PEG 3500, PEG 400, polyoxyl 400 stearate, polysorbate 20, polysorbate 80, propylene glycol, triglycerides, or a combination thereof. In some cases, the pharmaceutically acceptable liquid carrier comprises or further comprises from about 1% to about 10% w/w glyceryl monoleate. In some cases, the pharmaceutically acceptable liquid carrier comprises or further comprises from about 0.1% to about 10% w/w lecithin. In some cases, the pharmaceutically acceptable liquid carrier comprises or further comprises from about 0.1% to about 1.5% w/w PEG 3500. In some cases, the pharmaceutically acceptable liquid carrier comprises or further comprises from about 0.1% to about 20% w/w PEG 400. In some cases, the pharmaceutically acceptable liquid carrier comprises or further comprises from about 0.1% to about 15% w/w polyoxyl 400 stearate. In some cases, the pharmaceutically acceptable liquid carrier comprises or further comprises from about 0.01% to about 2.5% w/w polysorbate 20. In some cases, the pharmaceutically acceptable liquid carrier comprises or further comprises from about 0.1% to about 10% w/w polysorbate 80. In some cases, the pharmaceutically acceptable liquid carrier comprises or further comprises from about 0.1% to about 20% w/w propylene glycol.

Some cases comprise the tonicity adjusting agent that is dextrose, potassium chloride, sodium chloride, or a combination thereof. Some cases comprise the tonicity adjusting agent that is sodium chloride. In some cases, the pharmaceutically acceptable liquid carrier comprises or further comprises from about 0.01% to about 0.5% w/w dextrose. In some cases, the pharmaceutically acceptable liquid carrier comprises or further comprises from about 0.1% to about 1.9% w/w potassium chloride. In some cases, the pharmaceutically acceptable liquid carrier comprises or further comprises from about 0.1% to about 1.9% w/w sodium chloride.

Some cases comprise the viscosity adjusting agent that is carboxymethyl cellulose (CMC), Me-OH-Pr cellulose, microcrystalline cellulose (MCC), sodium carboxymethyl cellulose (Na CMC), or a combination thereof. In some cases, the pharmaceutically acceptable liquid carrier comprises or further comprises from about 0.1% to about 2% w/w carboxymethyl cellulose. In some cases, the pharmaceutically acceptable liquid carrier comprises or further comprises from about 0.1% to about 5% w/w Me-OH-Pr cellulose. In some cases, the pharmaceutically acceptable liquid carrier comprises or further comprises from about 0.1% to about 2% w/w microcrystalline cellulose. In some cases, the pharmaceutically acceptable liquid carrier comprises or further comprises from about 0.1% to about 5% w/w sodium carboxymethyl cellulose (Na CMC).

In some cases, the pharmaceutically acceptable liquid carrier comprises or further comprises from about 0.001% to about 0.7% w/w methylparaben, from about 0.01% to about 0.3% w/w propylparaben, from about 0.1% to about 20% w/w propylene glycol, from about 0.1% to about 1.9% w/w sodium chloride, or a combination thereof. In some cases, the pharmaceutically acceptable liquid carrier comprises or further comprises from about 0.001% to about 0.7% w/w methylparaben, from about 0.01% to about 0.3% w/w propylparaben, from about 0.1% to about 20% w/w propylene glycol, and from about 0.1% to about 1.9% w/w sodium chloride.

Also disclosed are methods of treating anosmia, hyposmia, dysosmia, ageusia, hypogeusia, or dysgeusia in a subject in need thereof comprising administering to the subject in need thereof any of the combination pharmaceutical dosage units disclosed herein. In some cases, the combination pharmaceutical dosage unit is administered once daily to each naris. In some cases, the combination pharmaceutical dosage unit is administered twice daily to each naris.

In some cases, the combination pharmaceutical dosage unit is administered each day for from about 7 days to about 365 days. In some cases, the combination pharmaceutical dosage unit is administered each day for from about 7 days to about 6 months. In some cases, the combination pharmaceutical dosage unit is administered each day for from about 7 days to about 4 months. In some cases, the combination pharmaceutical dosage unit is administered each day for from about 1 month to about 12 months.

In some cases, the subject experiences a decrease in a detection threshold (DT) score, a decrease in a recognition threshold (RT) score, an increase in a magnitude estimation (ME) score, and/or a change in a hedonic (H) score as measured with a forced-choice, three-stimuli, stepwise-staircase technique using one or more odorants after administering the combination pharmaceutical dosage unit to the subject. In some cases, the one or more odorants comprise pyridine, nitrobenzene, thiophene, amyl acetate, or a combination thereof.

In some cases, the subject experiences a decrease in an taste detection threshold (DT) score, a decrease in a recognition threshold (RT) score, an increase in a magnitude estimation (ME) score, and/or a change in a hedonic (H) score as measured with a forced-choice, three-stimuli, stepwise-staircase technique using one or more tastants testing compounds after administering the combination pharmaceutical dosage unit to the subject. In some cases, the one or more tastants comprise sodium chloride (NaCl), sucrose, hydrogen chloride (HCl), urea, or a combination thereof.

Also disclosed are kits for the treatment of anosmia, hyposmia, dysosmia, ageusia, hypogeusia, or dysgeusia comprising: (a) a multi-dose nasal spray device that delivers any of the combination pharmaceutical dosage units disclosed herein; and (b) instructions for use.

### Nasal Sprays and Multi-dose Nasal Spray Devices

Any of the compositions comprising one or more PDE inhibitors can be administered in a nasal spray to treat taste and smell dysfunctions. Advantages of nasal spray administration include the ability to use lower amounts of the PDE inhibitors than are required for oral administration, which can reduce or eliminate side effects associated with the PDE inhibitors. Nasal spray administration of the PDE inhibitors can also speed up the therapeutic effect of the PDE inhibitors in comparison to oral administration.

Accordingly, provided herein are multi-dose nasal spray devices that deliver a dosage unit of one or more PDE inhibitors in a plume when actuated.

The plume can be characterized according to one or more parameters such as total volume, droplet size distribution, spray pattern, and plume geometry. The droplet size distribution can be characterized according to the percentage of droplets having a size of less than 10 µm, a D₁₀, a D₅₀, a D₉₀, a span, or a combination thereof. In a plume, 10% of the droplets have a size less than the D₁₀, 50% of the droplets have a size less than the D₅₀, and 90% of the droplets have a size less than the D₉₀. The span can be calculated from these numbers according to the following formula: span = (D₉₀ - D₁₀)/D₅₀. Spray pattern measures the ovality of the spray, which can be calculated from the ratio of maximum to minimum cross sections diameter of the plume at a distance from the spray device. Plume geometry measures the plume angle at the origin of the plume. Plume geometry can be measured at two distances from the origin of the plume, for example, at two side views 90° relative to each other. Plume geometry can also be calculated from the spray pattern. The volume of the plume, the droplet size distribution, the spray pattern and plume geometry can all effect the treatment efficacy of active ingredients delivered by nasal spray. The multi-dose nasal spray devices and plumes disclosed herein can optimize treatment efficacy by controlling the delivery of the active ingredient to the correct target site.

Accordingly, disclosed herein are multi-dose nasal spray devices for delivery of theophylline to a human's nasal epithelium that delivers a dosage unit in a plume upon actuation, wherein the dosage unit comprises from 10 µg to 200 µg of theophylline in a pharmaceutically acceptable carrier comprising one or more excipients; and wherein the plume is characterized by: (a) a total volume of from 25 µL to 200 µL; and (b) a droplet size distribution characterized by one or more of the following: (i) less than 5 % of the droplets in the plume having a size of less than 10 µm, (ii) a D₁₀ of greater than 12.5 µm, wherein 10% of the droplets in the plume have a size less than the D₁₀, (iii) a D₅₀ of from 30 to 70 µm, wherein 50% of the droplets in the plume have a size less than the D₅₀, (iv) a D₉₀ of less than 200 µm, wherein 90% of the droplets in the plume have a size less than the D₉₀, and (v) a span of from 1 to 6, wherein the span is calculated according to: (D₉₀ - D₁₀)/D₅₀.

In some cases, the dosage unit comprises from 15 µg to 150 µg of theophylline. In some cases, the dosage unit comprises from 20 µg to 100 µg of theophylline. In some cases, the dosage unit comprises about 100 µg theophylline. In some cases, the dosage unit comprises about 75 µg theophylline. In some cases, the dosage unit comprises about 50 µg of theophylline. In some cases, the dosage unit comprises about 20 µg of theophylline.

Also disclosed are multi-dose nasal spray devices for delivery of theophylline to a human's nasal epithelium that delivers a dosage unit in a plume upon actuation, wherein the dosage unit comprises from 0.17 µg/kg to 3.4 µg/kg of theophylline in a pharmaceutically acceptable carrier comprising one or more excipients; and wherein the plume is characterized by: (a) a total volume of from 25 µL to 200 µL; and (b) a droplet size distribution characterized by one or more of the following: (i) less than 5% of the droplets in the plume having a size of less than 10 µm, (ii) a D₁₀ of greater than 12.5 µm, wherein 10% of the droplets in the plume have a size less than the D₁₀, (iii) a D₅₀ of from 30 to 70 µm, wherein 50% of the droplets in the plume have a size less than the D₅₀, (iv) a D₉₀ of less than 200 µm, wherein 90% of the droplets in the plume have a size less than the D₉₀, and (v) a span of from 1 to 6, wherein the span is calculated according to: (D₉₀ - D₁₀)/D₅₀.

In some cases, the dosage unit comprises from 0.25 µg/kg to 2.6 µg/kg of theophylline. In some cases, the dosage unit comprises from 0.33 µg/kg to 1.7 µg/kg of theophylline. In some cases, the dosage unit comprises about 1.7 µg/kg theophylline. In some cases, the dosage unit comprises about 1.3 µg/kg theophylline. In some cases, the dosage unit comprises about 0.85 µg/kg of theophylline. In some cases, the dosage unit comprises about 0.33 µg/kg of theophylline.

In some cases, the pharmaceutically acceptable carrier has a pH that is greater than 7.0. In some cases, the pharmaceutically acceptable carrier has a pH that is from 7.1 to 8.5. In some cases, the pharmaceutically acceptable carrier has a pH that is from 7.1 to 7.4. In some cases, the pharmaceutically acceptable carrier has a pH that is about: 7.1, 7.2, 7.3, 7.4, 7.5, 7.6, 7.7, 7.8, 7.9, 8, 8.1, 8.2, 8.3, 8.4, or 8.5. In some cases, the pharmaceutically acceptable carrier has a pH that is at least 7.1, 7.2, 7.3, 7.4, 7.5, 7.6, 7.7, 7.8, 7.9, 8, 8.1, 8.2, 8.3, 8.4, or 8.5.

The total volume of a plume can be important. Too large of a volume, and active ingredient can be lost due to dripping out of the nose or down the throat. Too small a volume can result in an insufficient amount of the nasal epithelium being coated by the plume. This can result in sub-optimal therapeutic outcomes for the patient.

The multi-dose nasal spray devices disclosed herein can deliver a dosage unit in a plume that has a total volume of from 25 to 200 µL. "Total volume," when used in connection with a plume, is the total liquid volume of all the droplets in the plume. In some cases, the plume is characterized by the total volume of the plume that is from 50 µL to 150 µL. In some cases, the plume is characterized by the total volume of the plume that is from 75 µL to 125 µL. In some cases, the plume is characterized by the total volume of the plume that is from 90 µL to 110 µL. In some cases, the plume is characterized by the total volume of the plume that is about: 25 µL, 30 µL, 35 µL, 40 µL, 45 µL, 50 µL, 55 µL, 60 µL, 65 µL, 70 µL, 75 µL, 80 µL, 85 µL, 90 µL, 100 µL, 110 µL, 120 µL, 130 µL, 140 µL, 150 µL, 160 µL, 170 µL, 180 µL, 190 µL, or 200 µL. In some cases, the plume is characterized by the total volume of the plume that is about 50 µL. In some cases, the plume is characterized by the total volume of the plume that is about 100 µL. In some cases, the plume is characterized by the total volume of the plume that is about 140 µL.

Optimal droplet sizes for a plume can be those that ensure the maximum amount of active ingredient is applied to the nasal epithelium. Minimizing the amount of very small droplets in the plume can reduce the amount of the plume that enters into the esophagus or lungs. This can reduce or eliminates side effects and ensure maximal delivery to the intended site of action. Minimizing the amount of larger drops can prevent loss of the active ingredient due to dripping out of the nose. Larger drops can also result in the formulation dripping into the back of the throat, which can cause irritation and delivery of active ingredient to undesired regions.

Accordingly, in some cases, the plume is characterized by less than 4% of the droplets in the plume having a size of less than 10 µm. In some cases, the plume is characterized by less than 3% of the droplets in the plume having a size of less than 10 µm. In some cases, the plume is characterized by less than 2% of the droplets in the plume having a size of less than 10 µm.

In some cases, the plume is characterized by the D₁₀ that is greater than 15 µm. In some cases, the plume is characterized by the D₁₀ that is greater than 17.5 µm. In some cases, the plume is characterized by the D₁₀ that is from 12.5 µm to 30 µm. In some cases, the plume is characterized by the D₁₀ that is from 15 µm to 25 µm.
In some cases, the plume is characterized by the D₅₀ that is from 40 µm to 60 µm. In some cases, the plume is characterized by the D₅₀ that is from 30 µm to 60 µm. In some cases, the plume is characterized by the D₅₀ that is from 30 µm to 50 µm. In some cases, the plume is characterized by the D₅₀ that is from 30 µm to 40 µm. In some cases, the plume is characterized by the D₅₀ that is about: 30 µm, 32.5 µm, 35 µm, 37.5 µm, 40 µm, 42.5 µm, 45 µm, 50 µm, 55 µm, 60 µm, 65 µm, or 70 µm.

In some cases, the plume is characterized by the D₉₀ that is less than 175 µm. In some cases, the plume is characterized by the D₉₀ that is less than 150 µm. In some cases, the plume is characterized by the D₉₀ that is less than 125 µm. In some cases, the plume is characterized by the D₉₀ that is less than 100 µm. In some cases, the plume is characterized by the D₉₀ that is less than 90 µm. In some cases, the plume is characterized by the D₉₀ that is from 75 µm to 199 µm. In some cases, the plume is characterized by the D₉₀ that is from 75 µm to 175 µm. In some cases, the plume is characterized by the D₉₀ that is from 75 µm to 150 µm. In some cases, the plume is characterized by the D₉₀ that is from 75 µm to 125 µm. In some cases, the plume is characterized by the D₉₀ that is from 75 µm to 100 µm. In some cases, the plume is characterized by the D₉₀ that is about: 75 µm, 80 µm, 85 µm, 90 µm, 100 µm, 110 µm, 120 µm, 130 µm, 140 µm, 150 µm, 160 µm, 170 µm, 180 µm, or 190µm.
In some cases, the plume is characterized by the span that is from 1 to 5. In some cases, the plume is characterized by the span that is from 1 to 4. In some cases, the plume is characterized by the span that is from 1 to 3. In some cases, the plume is characterized by the span that is from 1 to 2. In some cases, the plume is characterized by the span that is about: 1, 1.1, 1.2, 1.3, 1.4, 1.5, 1.6, 1.7, 1.8, 1.9, 2, 2.1, 2.2, 2.3, 2.4, 2.5, 2.6, 2.7, 2.7, 2.9, 3, 3.25, 3.5, 3.75, 4, 4.5, 5, 5.5, or 6.

The spray pattern and geometry of the plume can affect the efficacy of treatment. For example, an irregular shape can result in uneven coating of the nasal epithelium and a resulting reduction in therapeutic efficacy. In another example, too narrow of a plume can reduce the area of the nasal epithelium that is coated by the plume. Conversely, too wide of a plume can direct the plume towards unintended targets such as the back of the throat.

Accordingly, in some cases, the plume is further characterized by having an ovality of from 0.7 to 1. In some cases, the plume is further characterized by having an ovality of from 0.8 to 1. In some cases, the plume is further characterized by having an ovality of from 0.9 to 1. In some cases, the plume is further characterized by having an ovality of about 1.

In some cases, the plume is further characterized by having a geometry of from 30° to 90°. In some cases, the plume is further characterized by having a geometry of from 45° to 75°. In some cases, the plume is further characterized by having a geometry of about: 30°, 35°, 40°, 45°, 50°, 55°, 60°, 65°, 70°, 75°, 80°, 85°, or 90°.

The pharmaceutically acceptable carrier and excipients in a nasal spray formulation can affect properties of the liquid, such as viscosity and osmolarity. This can effect the droplet size distribution of the plume, plume geometry and shape, and ultimately the bioavailability of the active ingredient.

In some cases, the pharmaceutically acceptable carrier comprises water, aminoboronic acid and its derivatives, amastatin, surfactants, bile salts, gelified insulin, bioadhesive microspheres, phospholipids, chitosan nanoparticles, alkyl glycerides, or a combination thereof. In some cases, the pharmaceutically acceptable carrier comprises water.

In some cases, the one or more excipients comprise a buffering agent, a flavoring agent, a humectant, a penetration enhancer, a pH adjusting agent, a preservative, a solvent or co-solvent, a surfactant, a tonicity adjusting agent, a viscosity adjusting agent, or a combination thereof.

Some cases comprise the buffering agent that is potassium phosphate, sodium acetate, sodium citrate, sodium phosphate, trisodium citrate, or a combination thereof. In some cases, the one or more excipients comprise or further comprises from about 0.00001% to about 0.0006% w/w trisodium citrate.

Some cases comprise or further comprise the flavoring agent that is menthol, saccharin sodium, sorbitol, or a combination thereof.

Some cases comprise or further comprise the humectant that is glycerin, propylene glycol, hexylene glycol, butylenes glycol, glyceryl triacetate, vinyl alcohol, neoagarobiose, glycerol, sorbitol, xylitol, maltitol, polydextrose, quillaia, lactic acid, urea, or aloe vera. Some cases comprise or further comprise the humectant that is propylene glycol. In some cases, the one or more excipients comprise or further comprises from about 0.01% to about 0.233% w/w glycerin. In some cases, the one or more excipients comprise or further comprises from about 0.1% to about 5% w/w glycerol. In some cases, the one or more excipients comprise or further comprises from about 0.5% to about 10% w/w sorbitol.

Some cases comprise the penetration enhancer that is oleic acid. In some cases, the one or more excipients comprise or further comprises from about 0.01% to about 0.132% w/w oleic acid.

Some cases comprise the pH adjusting agent that is acetic acid, citric acid, hydrochloric acid, sodium hydroxide, sulfuric acid, or a combination thereof. In some cases, the one or more excipients comprise or further comprises from about 0.01% to about 0.5% w/w acetic acid. In some cases, the one or more excipients comprise or further comprises from about 0.01% to about 0.5% w/w citric acid. In some cases, the one or more excipients comprise or further comprises from about 0.01% to about 0.4% w/w sulfuric acid.

Some cases comprise the preservative that is benzalkonium chloride, benzethonium chloride, benzyl alcohol, butylated hydroxy toluene, butylated hydroxyanisole, chlorobutanol, edetate disodium, methylparaben, phenylethyl alcohol, phenylmercuric acetate, propylene paraben, propylparaben, thimerosal, or a combination thereof. Some cases comprise the preservative that is methylparaben, propylparaben, or a combination thereof. In some cases, the one or more excipients comprise or further comprises from about 0.001% to about 0.119% w/w benzalkonium chloride. In some cases, the one or more excipients comprise or further comprises from about 0.001% to about 0.0366% w/w benzyl alcohol. In some cases, the one or more excipients comprise or further comprises from about 0.001% to about 0.01% w/w butylated hydroxy toluene. In some cases, the one or more excipients comprise or further comprises from about 0.00001% to about 0.0002% w/w butylated hydroxyanisole. In some cases, the one or more excipients comprise or further comprises from about 0.01% to about 0.5% w/w chlorobutanol. In some cases, the one or more excipients comprise or further comprises from about 0.01% to about 0.5% w/w edetate disodium. In some cases, the one or more excipients comprise or further comprises from about 0.01% to about 0.254% w/w phenylethyl alcohol. In some cases, the one or more excipients comprise or further comprises from about 0.001% to about 0.1% w/w propylene paraben. In some cases, the one or more excipients comprise or further comprises from about 0.001% to about 0.7% w/w methylparaben. In some cases, the one or more excipients comprise or further comprises from about 0.01% to about 0.3% w/w propylparaben.

Some cases comprise the solvent or co-solvent that is ethanol, glycerol, glyceryl dioleate, glycine, polyethylene glycol (PEG), PEG 400, propylene glycol, triglycerides, or a combination thereof. Some cases comprise the solvent or co-solvent that is propylene glycol. In some cases, the one or more excipients comprise or further comprises from about 0.1% to about 2% w/w ethanol. In some cases, the one or more excipients comprise or further comprises from about 0.1% to about 5% w/w glycerol. In some cases, the one or more excipients comprise or further comprises from about 1% to about 15% w/w glyceryl dioleate. In some cases, the one or more excipients comprise or further comprises from about 0.1% to about 20% w/w PEG 400. In some cases, the one or more excipients comprise or further comprises from about 0.1% to about 5% w/w polyethylene glycol (PEG). In some cases, the one or more excipients comprise or further comprises from about 0.1% to about 5% w/w triglycerides. In some cases, the one or more excipients comprise or further comprises from about 0.1% to about 20% w/w propylene glycol.

Some cases comprise the surfactant that is glyceryl monoleate, lecithin, PEG 3500, PEG 400, polyoxyl 400 stearate, polysorbate 20, polysorbate 80, propylene glycol, triglycerides, or a combination thereof. In some cases, the one or more excipients comprise or further comprises from about 1% to about 10% w/w glyceryl monoleate. In some cases, the one or more excipients comprise or further comprises from about 0.1% to about 10% w/w lecithin. In some cases, the one or more excipients comprise or further comprises from about 0.1% to about 1.5% w/w PEG 3500. In some cases, the one or more excipients comprise or further comprises from about 0.1% to about 20% w/w PEG 400. In some cases, the one or more excipients comprise or further comprises from about 0.1% to about 15% w/w polyoxyl 400 stearate. In some cases, the one or more excipients comprise or further comprises from about 0.01% to about 2.5% w/w polysorbate 20. In some cases, the one or more excipients comprise or further comprises from about 0.1% to about 10% w/w polysorbate 80. In some cases, the one or more excipients comprise or further comprises from about 0.1% to about 20% w/w propylene glycol.

Some cases comprise the tonicity adjusting agent that is dextrose, potassium chloride, sodium chloride, or a combination thereof. Some cases comprise the tonicity adjusting agent that is sodium chloride. In some cases, the one or more excipients comprise or further comprises from about 0.01% to about 0.5% w/w dextrose. In some cases, the one or more excipients comprise or further comprises from about 0.1% to about 1.9% w/w potassium chloride. In some cases, the one or more excipients comprise or further comprises from about 0.1% to about 1.9% w/w sodium chloride.

Some cases comprise the viscosity adjusting agent that is carboxymethyl cellulose (CMC), Me-OH-Pr cellulose, microcrystalline cellulose (MCC), sodium carboxymethyl cellulose (Na CMC), or a combination thereof. In some cases, the one or more excipients comprise or further comprises from about 0.1% to about 2% w/w carboxymethyl cellulose. In some cases, the one or more excipients comprise or further comprises from about 0.1% to about 5% w/w Me-OH-Pr cellulose. In some cases, the one or more excipients comprise or further comprises from about 0.1% to about 2% w/w microcrystalline cellulose. In some cases, the one or more excipients comprise or further comprises from about 0.1% to about 5% w/w sodium carboxymethyl cellulose (Na CMC).

In some cases, the one or more excipients comprise or further comprises from about 0.001% to about 0.7% w/w methylparaben, from about 0.01% to about 0.3% w/w propylparaben, from about 0.1% to about 20% w/w propylene glycol, from about 0.1% to about 1.9% w/w sodium chloride, or a combination thereof. In some cases, the one or more excipients comprise or further comprises from about 0.001% to about 0.7% w/w methylparaben, from about 0.01% to about 0.3% w/w propylparaben, from about 0.1% to about 20% w/w propylene glycol, and from about 0.1% to about 1.9% w/w sodium chloride.

Also disclosed are methods of treating anosmia, hyposmia, dysosmia, ageusia, hypogeusia, or dysgeusia in a subject in need thereof comprising intranasal administration of theophylline with any of the multi-dose nasal spray devices disclosed herein. In some cases, the theophylline is administered once daily to each naris. In some cases, the theophylline is administered twice daily to each naris.

In some cases, the theophylline is administered each day for from about 7 days to about 365 days. In some cases, the theophylline is administered each day for from about 7 days to about 6 months. In some cases, the theophylline is administered each day for from about 7 days to about 4 months. In some cases, the theophylline is administered each day for from about 1 month to about 12 months.

In some cases, the subject experiences a decrease in a detection threshold (DT) score, a decrease in a recognition threshold (RT) score, an increase in a magnitude estimation (ME) score, and/or a change in a hedonic (H) score as measured with a forced-choice, three-stimuli, stepwise-staircase technique using one or more odorants after administering the theophylline to the subject. In some cases, the one or more odorants comprise pyridine, nitrobenzene, thiophene, amyl acetate, or a combination thereof.

In some cases, the subject experiences a decrease in an taste detection threshold (DT) score, a decrease in a recognition threshold (RT) score, an increase in a magnitude estimation (ME) score, and/or a change in a hedonic (H) score as measured with a forced-choice, three-stimuli, stepwise-staircase technique using one or more tastants testing compounds after administering the theophylline to the subject. In some cases, the one or more tastants comprise sodium chloride (NaCl), sucrose, hydrogen chloride (HCl), urea, or a combination thereof.

In some cases, the subject experiences a clinically detectable improvement in taste or smell function within 1-4 weeks of starting treatment.

Also disclosed are kits for the treatment of anosmia, hyposmia, dysosmia, ageusia, hypogeusia, or dysgeusia comprising: (a) any of the multi-dose nasal spray devices for delivery of theophylline to a human's nasal epithelium disclosed herein; and instructions for use.

### Aerosols

By "aerosol" is meant any composition of a PDE inhibitor administered as an aerosolized formulation, including for example an inhalation spray, inhalation solution, inhalation suspension, a nebulized solution, or nasal spray. Aerosolized formulations can deliver high concentrations of a PDE inhibitor directly to the airways with low systemic absorption. Solutions for aerosolization typically contain at least one therapeutically active PDE inhibitor dissolved or suspended in an aqueous solution that may further include one or more excipients (e.g., preservatives, viscosity modifiers, emulsifiers, or buffering agents). The solution acts as a carrier for the PDE inhibitor. In some cases, the preservative is methylparaben or propylparaben. These formulations are intended for delivery to the respiratory airways by inspiration.

A major limitation of pulmonary delivery is the difficulty of reaching the deep lung. To achieve high concentrations of a PDE inhibitor solution in both the upper and lower respiratory airways, the PDE inhibitor solution is preferably nebulized in jet nebulizers, a ultrasonic nebulizer, or an electronic nebulizer particularly those modified with the addition of one-way flow valves, such as for example, the Pari LC Plus™ nebulizer, commercially available from Pari Respiratory Equipment, Inc., Richmond, Va., which delivers up to 20% more drug than other unmodified nebulizers.

### Drops and Gels

In some cases, the PDE inhibitor is directly applied to the nasal or lingual epithelium as a liquid, cream, lotion, ointment or gel. These fluids or semifluids contain at least one therapeutically active PDE inhibitor and may further include at least one excipient (e.g., preservatives, viscosity modifiers, emulsifiers, or buffering agents) that are formulated for administration as nose drops, or applied with an applicator to the inside of the nasal passages. In some cases, the preservative is methylparaben or propylparaben. The pH of the formulation is preferably maintained from 4.5 and 7.0, more preferably from 5.0 and 7.0 and most preferably from 5.5 and 6.5. The osmolarity of the formulation can also be adjusted to osmolarities of about 250 to 350 mosm/L.

### Dry Powder Formulation

As an alternative therapy to aerosol, liquid or gel delivery, the PDE inhibitor may be administered in a dry powder formulation for efficacious delivery into the nasal cavity and/or endobronchial space. Dry powder formulation is convenient because it does not require further handling by a physician, pharmacist or patient such as diluting or reconstituting the agent as is often required with nebulizers. Furthermore, dry powder delivery devices are sufficiently small and fully portable. Dry powder formulations may also be applied directly on the lingual epithelium.

For dry powder formulations, a PDE inhibitor and/or carrier is processed to median diameter ranging from 0.001-250 µm typically by media milling, jet milling, spray drying, supercritical fluid energy, or particle precipitation techniques. Particles of a desired size ranges can also be obtained through the use of sieves. Frequently, milled particles are passed through one or more sieves to isolate a desired size range. In some cases intended for pulmonary administration, the PDE inhibitor and/or carrier has a median diameter ranging from 0.01-25 µm, 0.1-10 µm, 1-10 µm, 1-5 µm, or 2-5µm. In further cases intended for pulmonary administration, the PDE inhibitor and/or carrier has a median diameter ranging less than 20 µm, 10 µm, 5 µm, 4,µm, 3 µm, 2 µm, or 1 µm. In some cases intended for nasal administration, the PDE inhibitor and/or carrier has a median diameter ranging from 1-250 µm, 5-200 µm, 10-150 µm, 10-100 µm, 10-50 µm, 15-100 µm, 15-50 µm, or 20-60 µm. In further cases intended for nasal administration, the PDE inhibitor and/or carrier has a median diameter of less than 250 µm, 200 µm, 150 µm, 100 µm, 75 µm, 60 µm, 50 µm, 40 µm or 30 µm. In other cases intended for nasal administration, the PDE inhibitor and/or carrier has a median diameter of at least 20 µm, 30 µm, 40 µm, 50 µm, 60 µm, 75 µm, 100 µm, 150 µm or 200 µm.

In some cases, a pharmaceutically acceptable carrier for the present compositions and formulations include but are not limited to amino acids, peptides, proteins, non-biological polymers, biological polymers, simple sugars, carbohydrates, gums, inorganic salts and metal compounds which may be present singularly or in combination. In some cases, the pharmaceutically acceptable carrier comprises native, derivatized, modified forms, or combinations thereof.

In some cases, useful proteins include, but are not limited to, gelatin or albumin. In some cases, useful sugars that can serve as pharmaceutically acceptable carriers include, but are not limited to fructose, galactose, glucose, lactitol, lactose, maltitol, maltose, mannitol, melezitose, myoinositol, palatinite, raffinose, stachyose, sucrose, trehalose, xylitol, hydrates thereof, and combinations of thereof.

In some cases, useful carbohydrates that can serve as pharmaceutically acceptable carriers include, but are not limited to starches such as corn starch, potato starch, amylose, amylopectin, pectin, hydroxypropyl starch, carboxymethyl starch, and cross-linked starch. In other cases, useful carbohydrates that can serve as pharmaceutically acceptable carriers include, but are not limited to cellulose, crystalline cellulose, microcrystalline cellulose, α-cellulose, methylcellulose, hydroxypropyl cellulose, carboxymethyl cellulose, ethyl cellulose, hydroxypropyl methyl cellulose, and cellulose acetate.

In some cases, the composition or formulation includes an excipient. Useful excipients include, but are not limited to, fluidizers, lubricants, adhesion agents, surfactants, acidifying agents, alkalizing agents, agents to adjust pH, antimicrobial preservatives, antioxidants, anti-static agents, buffering agents, chelating agents, humectants, gel-forming agents, or wetting agents. Excipients also include coloring agents, coating agents, sweetening, flavoring and perfuming and other masking agents. The compositions and formulations of this disclosure may include a therapeutic agent with an individual excipient or with multiple excipients in any suitable combination, with or without a carrier.

The dry powder formulations of the present disclosure may be used directly in metered dose or dry powder inhalers. With dry powder inhalers, the inspiratory flow of the patient accelerates the powder out of the device and into the nasal and/or oral cavity. Alternatively, dry powder inhalers may employ an air source, a gas source, or electrostatics, to deliver the therapeutic agent. The dry powder formulations are temperature stable and have a physiologically acceptable pH of 4.0-7.5, preferably 6.5 to 7.0.

### Kits/Articles of Manufacture

For use of the therapeutic compositions described herein, kits and articles of manufacture are also described. In some cases, such kits include a carrier, package, or container that is compartmentalized to receive one or more blister packs, bottles, tubes, capsules, and the like. In certain cases, the pharmaceutical compositions are presented in a pack or dispenser device which contains one or more unit dosage forms containing a compound provided herein. In other cases, the pack contains metal or plastic foil, such as a blister pack. In some cases, the pack contains capsules, vials, or tubes. In other cases, the pack or dispenser device is accompanied by instructions for administration. In some cases, the dispenser is disposable or single use, while in other cases, the dispenser is reusable. In certain cases, the pharmaceutical formulations are preloaded into the device.

In still other cases, the pack or dispenser also accompanied with a notice as required by a governmental agency regulating the manufacture, use, or sale of pharmaceuticals. This notice states that the drug is approved by the agency for human or veterinary administration. Such notice, for example, is the labeling approved by the U.S. Food and Drug Administration for prescription drugs, or the approved product insert. Compositions containing a compound provided herein formulated in a compatible pharmaceutical carrier are also prepared, placed in an appropriate container, and labeled for treatment of an indicated condition.

The articles of manufacture provided herein may also contain an administration or dispensing device. Examples of administration devices include pulmonary inhalers and intranasal applicators. Pumps may be provided with the inhalers and intranasal devices, or the pumps may be built into the devices. Alternatively, a propellant may be included with or it may be stored within the devices.

Such kits optionally comprise an identifying description or label for the containers. In further cases, the label is on a container with letters, numbers or other characters forming the label and attached, molded or etched into the container itself; a label is associated with a container when it is present within a receptacle or carrier that also holds the container, e.g., as a package insert. In some cases, a label is used to indicate that the contents are to be used for a specific therapeutic application. In yet other cases, the label also indicates directions for use of the contents, such as in the methods described herein. In some cases, a set of instructions may also be included, generally in the form of a package insert. The informational material may contain instructions on how to dispense the pharmaceutical composition, including description of the type of patients who may be treated, the schedule (*e*.*g*., dose and frequency), and the like.

The disclosure also relates to a set (kit) consisting of separate packs of kits that are frequently assembled for shipping or for patient convenience, such as a weekly, biweekly or monthly supply of a medicament.

### EXPERIMENTAL SECTION

While some illustrative examples have been shown and described herein, such cases are provided by way of example only. The examples do not use the claimed multi-dose nasal spray device, and are hence for reference only.

### EXAMPLE 1

Patients with smell loss (hyposmia) reflect a clinically diverse group of patients (1-10). Whereas there is common agreement that many patients exhibit this clinical problem, there is no agreement with respect to their treatment. Indeed, most groups who evaluate these patients consider that there are few, if any, medically relevant treatments for them.

In an attempt to elucidate the biochemical pathology of hyposmia, total protein fractionation was performed on saliva (14, 15) and nasal mucus (16), since these fluids bathe both taste buds and olfactory epithelial tissues, respectively, and contain substances which are critical to maintain these sense organs (14-16). It was discovered that some patients with smell loss had diminished salivary (17) and nasal mucus (18) levels of the saliva and nasal mucus protein carbonic anhydrase (CA) VI, a putative stem cell growth factor; treatment of these patients with exogenous zinc increases both salivary and nasal mucus CA VI (19) leading to an increase in smell acuity (19). These CAVI deficient patients, however, represent only a fraction of the total patient group (1, 2).

Further investigation revealed many of the non-CAVI deficient patients exhibited lower than normal levels of both cAMP and cGMP in their saliva (31) and nasal mucus (32). When the cAMP and cGMP levels in the saliva and in the nasal mucus of these patients was compared against the severity of their smell loss, it was noted that as smell loss severity increased (worsened) levels of these cyclic nucleotides in saliva (34) and nasal mucus (35) decreased. Since these cyclic nucleotides act as growth factors for several neural tissues (36-38) including olfactory tissues (39-45), it was reasoned that lower than normal levels of cyclic nucleotides may play a role in generation of their hyposmia. To test this hypothesis, patients were treated with the PDE inhibitor theophylline in an effort to increase saliva and nasal mucus levels of these cyclic nucleotides. It was found that hyposmia was corrected in many of them as demonstrated by improvement of psychophysical measurements of hyposmia, (1, 2, 49), by increased brain activation to several olfactory stimuli through measurements of functional magnetic resonance imaging (fMRI) (48) and associated with changes in serum theophylline (44).

In order to confirm these initial studies, theophylline was given to 312 patients in a fixed design, controlled, open trial over a period of seven years. These patients exhibited salivary (32) and nasal mucus (33) levels of cAMP and cGMP below the normal mean. Studies were approved by an Institutional Review Board and all patients gave informed consent.

The patients ranged in age from 18 to 86 y (55±1y, mean±SEM) and consisted of 178 women, aged 18-85y (55±2y) and 134 men, aged 23-86y (54±3y). Patients reported a history of smell loss extending from 2 months to 40 y (6.5±1.0 y). Etiology of smell loss varied; the major causes of hyposmia were post influenza-like hyposmia [97 patients, 31.1% of the total (50)] and allergic rhinitis [97 patients, 31.1% of the total (51)] followed by head injury [42 patients, 13.5% (52)] and several other causes, as previously described [76 patients, 24.4% (1, 2)]. Levels of CA VI in their saliva and nasal mucus were within normal levels.

Patients initially reported their sensory dysfunction as either loss of taste (e.g., flavor) and/or smell function. This subjective response was documented by objective psychophysical measurements of olfactory function administered to each patient by use of a forced-choice, three-stimuli, stepwise-staircase technique in a fixed, controlled design (1, 53). Efficacy of this technique and results of testing were previously documented in a double-blind clinical trial (53). Four odors were used; they were pyridine (dead-fish odor), nitrobenzene (bitter-almond odor), thiophene (petroleum-like odor) and amyl acetate (banana-oil odor). Detection thresholds (DT), recognition thresholds (RT) and magnitude estimation (ME) values for each odor were determined as previously described (1, 53). Thresholds were converted into bottle units (BU) as previously described (53) and results reported as M±SEM of correct responses for each odor in each treatment group; ME was reported in % and results calculated to obtain M±SEM for each treatment group for all correct responses using data for the four highest odor concentrations presented (from 10^{-2M} - an absolute odor concentration).

In addition, each patient graded the hedonic (H) value of each odor presented for these same odor concentrations (from 10^{-2M} - an absolute odor concentration using a -100 - 0-+100 scale). If they considered the presented odor pleasant ("they wished to smell the odor again") they graded the odor as +1 - +100 with respect to pleasantness; if they considered the odor unpleasant ("they did not wish to smell the odor again") they graded the odor as -1 - -100 with respect to unpleasantness; if they did not consider the odor either pleasant or unpleasant they graded the odor as neutral or 0. Results were obtained by calculating the arithmetical sum of each correct recognition response for each odor with respect to its pleasantness, unpleasantness or neutrality. Arithmetic M±SEM were obtained for each treatment group for each odor presented.

Independently, patients were also required to grade their ability to smell daily on a scale from 0-100, with 0 reflecting no overall smell function over a 24 hour period, 100 reflecting normal overall smell function over this period and numbers from 0-100 reflecting their estimation of their overall ability to smell odors over this period.

Based upon values for the DT, RT and ME tests, patients were classified with respect to severity of smell loss into the four types [(1, 2) (Table I)]. Anosmia is the complete loss of smell. Patients with anosmia have DT, RT and ME test values of zero, since they cannot detect, recognize nor grade the intensity of any odor including an absolute concentration of any odorant (Table I). No patients with anosmia were present in the study due to the relative rarity of this condition (1). Patients with Type I hyposmia (96 patients) could detect some odors but could not recognize any odor correctly; thus, DTs for some odors were present, but RTs and MEs for all odors were zero since they could neither recognize correctly nor thereby grade correctly intensity of any odor (Table I). Patients with Type II hyposmia (208 patients) could detect and recognize some odors, but at levels greater than normal; thus DTs and RTs were present, but elevated above normal and MEs were present but at levels lower than normal (Table I). Patients with Type III hyposmia (8 patients) could detect and recognize all odors at normal levels (e.g., normal DT and RT), but ME values for one or more odors were significantly decreased below normal (Table I). Severity of smell loss graded from most to least severe loss was typed as anosmia > hyposmia Type I > Type II > Type III and verified by demonstrating that as smell loss severity increased levels of nasal mucus cAMP and cGMP decreased (32, 34).

**TABLE I: CLASSIFICATION OF SMELL LOSS**

| | **DETECTION THRESHOLD** | **RECOGNITION THRESHOLD** | **MAGNITUDE ESTIMATION** |
|---|---|---|---|
| | **DT in M/L** | **RT in M/L** | **MEAN ME in %** |
| **NORMALS** | + | +* | ≥48 |

| **PATIENTS** | | | |
|---|---|---|---|
| **ANOSMIA∞ HYPOSMIA** | 0 | 0 | 0 |
| **TYPE I** | ± | 0 | 0 |
| **TYPE II** | ± | ±* | <48 |

| | | | |
|---|---|---|---|
| + Normal (≤10^{-5M} for all odorants) +* Normal (≤10^{-2M} for all odorants) 0 Absent response (∞) ± Present but < normal (>10^{-5M}<∞ for all odorants) ±* Present but < normal (>10^{-2M}<∞ for all odorants) ∞ Inability to detect, recognize or judge intensity of an absolute concentration of odorant | | | |

After determination of hyposmia, patients were treated in an open label, fixed design, controlled open trial. Patients were given an oral extended release theophylline in divided daily doses taken in the middle of breakfast and lunch. All patients were initially given 200mg of theophylline; changes in this dose were made based upon subjective responses to therapy. If at a subsequent return visit, patients reported ≥5% subjective improvement in overall smell function, they continued on this same dose of theophylline and were reevaluated after four to six months of continued treatment. Results from any subsequent return from these improved patients were not included in any subsequent data report. All subsequent comparisons between treated and untreated patients were made only between those patients continuing in the study compared to their own measurements obtained in the untreated state. If patients reported <5% subjective improvement, their theophylline dose was increased by 200mg daily and they were scheduled for retesting at the study site after two to four months at this new dosage. For patients that reported <5% improvement, this sequence continued until patients reached 800mg of theophylline, at which time the study was considered completed.

Patients were initially divided into two groups based upon proximity to the study site. One group, consisting of local patients (212 patients), returned for reevaluation after two-four months of treatment. The other group, consisting of distant patients (100 patients), called the study site at two to four month intervals and visited the study site after six to 10 months of treatment. Some of the distant patients returned after treatment on 200mg, 400mg, 600mg of theophylline and these results were included with those of local patients at each dose level (see Fig. 1 for patient details).

### Local Patients - First Return (200mg theophylline)

Patients returned for reevaluation after two to four months of treatment. Measurements of blood serum theophylline were measured by a fluorescence polarization assay (Abbott, Chicago, IL). At this time, subjective changes in smell function were measured independently (compared to patient's memory of previous normal smell function monitored by use of the patients' daily measurements) using the scale from -100 - 0 - +100 previously described (vs). After independently recording their subjective responses, smell function was measured for all four odors with DT, RT, ME and H determined without recourse to prior measurements.

### Local Patients - Second Return (400mg theophylline)

Patients returned after four to eight months of treatment and consisted of patients who previously reported <5% improvement on 200mg of theophylline but also some distant patients who returned on this dose. Blood theophylline was measured as before. All patients independently reported subjective overall changes in smell function as done previously. At this visit, smell function was measured as before to determine DT, RT, ME and H for all four odors without recourse to prior measurements.

### Local Patients - Third Return (600mg theophylline)

Patients returned after four - 10 months of treatment and included both patients who reported <5% improvement on 400mg theophylline and also some of the distant patient group. Blood theophylline was obtained as before. Patients independently reported subjective changes in overall smell function and measurements of DT, RT, ME and H were obtained as before for all four odors without recourse to prior measurements.

### Local Patients - Fourth Return (800mg theophylline)

Patients returned after four - eight months of treatment and included patients who reported <5% improvement on 600mg theophylline and also some of the distant patient group. Blood theophylline was obtained as before. Patients independently reported subjective changes in overall smell function and measurements of DT, RT, ME and H were obtained as before for all four odors without recourse to prior measurements.

### Distant Patients - First Call In (200mg theophylline)

Subjective overall changes in smell acuity were reported by telephone and by FAX or email at two - four month intervals after treatment was initiated using the standardized form in which they measured daily changes in smell acuity using the -100 - 0 - +100 scale as noted above (vs). Using the same criteria noted above, if at their initial two - four month call-in they reported improvement in smell acuity of >5%, they continued at 200mg and returned to the study site at four - six months on this treatment dose. If they reported improvement in smell acuity of <5% their dose of theophylline was increased to 400mg for an additional two - four months and they then called the study site at the end of this period.

### Distant Patients - Second Call In (400mg theophylline)

At this time, if patients reported >5% improvement on 400mg they continued on this dose and returned to the study site after four - six months on this treatment dose. If they reported <5% improvement in smell function their theophylline dose was increased to 600mg and they then called the study site after four - six months on this treatment dose.

### Distant Patients - Third Call In (600mg theophylline)

At this time, if patients reported ≥5% improvement they were continued on this treatment dose and were requested to return to the study site in four - six months. If they reported <5% improvement in smell function they were requested to return to the study site as soon as possible to reevaluate their smell function. At this return, blood theophylline levels were measured. Smell function was measured using the subjective and psychophysical techniques (DT, RT, ME, H) previously described.

### Distant Patients - Fourth Call In (800mg theophylline)

At this time, if patients reported either ≥5% improvement or <5% improvement, they were requested to return to the study site as soon as possible to reevaluate their smell function. At this return, blood theophylline levels were measured and smell function were measured using both subjective and psychophysical techniques with results as shown on Table VII and discussed previously.

Since data for all measurements from each patient group (local or distant) on each dose level were combined mean and SEM for each measurement (DT, RT, ME, H) of smell function for each treatment group were calculated on this basis. Differences in measurements between before treatment and each treatment level were calculated and significance of differences estimated using Student t tests (values p<0.05 considered significant). Some patient responses were also analyzed by use of non-parametric statistics (sign test) and the Spearman rank correlation technique; r<0.05 was considered significant related to smell loss type and treatment response. Since each patient served as his/her own control with respect to before and after treatment, paired t tests were also calculated; t values <0.05 were considered significant. These values were not included in the results presented.

Results were previously obtained in 150 normal subjects for each of these measurements (1, 53-55) and are reported here for comparison.

### RESULTS

### Comparison of smell function in normal subjects and in untreated patients with hyposmia

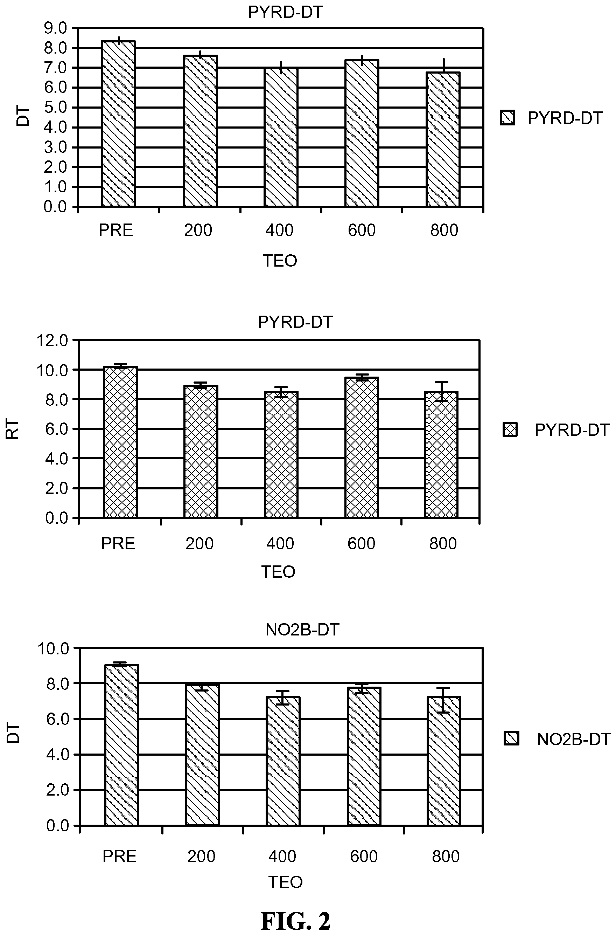

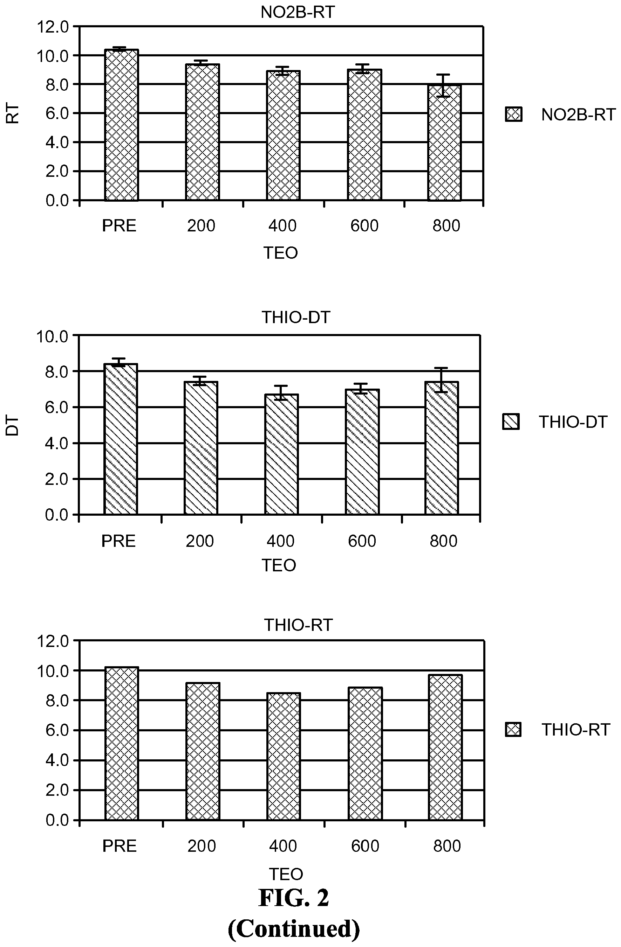

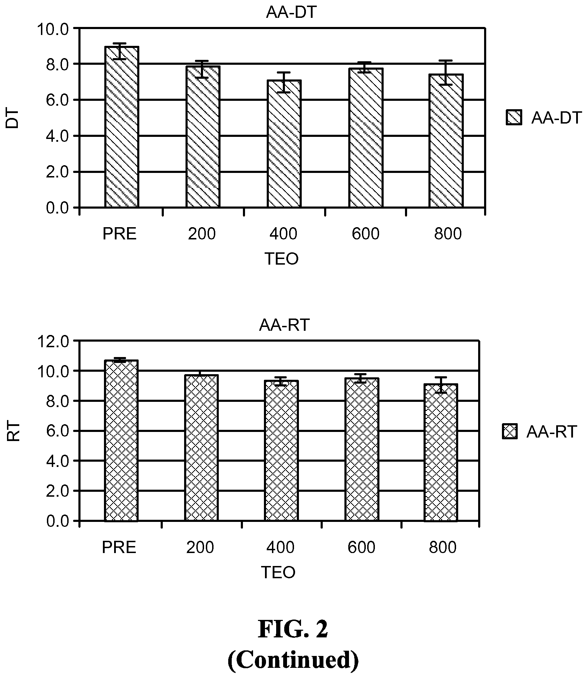

Results of each measurement (DT, RT, ME and H) in untreated patients indicated significant impairment of smell function compared to normal subjects (Table II, Fig. 2). For DT and RT, patient responses were significantly higher (less sensitive) than in normals (Table II), ME responses were significantly lower (less sensitive) than in normals (Fig. 2). H responses were significantly lower (less pleasant) for amyl acetate and nitrobenzene (odors usually considered pleasant) and significantly higher for pyridine and thiophene (less unpleasant - closer to zero for odors usually considered unpleasant) (Fig. 2). Analysis of H data indicate that for normal subjects H values for pyridine and thiophene (unpleasant responses) are similar to or slightly higher than ME values, whereas H values for nitrobenzene and amyl acetate (pleasant responses) are similar or slightly lower. Ratios of H:ME in normals for pyridine and thiophene are 1.09 and 1.05 whereas for nitrobenzene and amyl acetate they are 0.94 and 0.96. For untreated patients (patients with Type II and III hyposmia only) these ratios are quite different. For pyridine and thiophene, these ratios are 0.87 and 0.81 whereas for nitrobenzene and amyl acetate they are 0.81 and 0.10. In part, H decreases for nitrobenzene and amyl acetate are related to decreased patient acuity; however, the major discrepancy is that about 50% of patients with hyposmia also exhibit dysosmia (1, 2) in which odors usually considered pleasant are considered unpleasant (*e*.*g*., banana-oil odor may be considered putrid) and even unpleasant odors may be considered pleasant (*e*.*g*., pyridine may be considered flowery). These distortions in patients comprise a bimodal response for H values (some patients reporting a normal hedonic response related to appropriate pleasantness and unpleasantness of the perceived odor whereas others reporting a distortion) which reduces the overall arithmetic mean obtained for H for each odor.

**TABLE II: DIFFERENCES IN SMELL FUNCTION BETWEEN STUDY PATIENTS BEFORE TREATMENT AND NORMAL CONTROLS**

| | **PATIENTS (312)** | | | | **NORMALS (155)** | | | |
|---|---|---|---|---|---|---|---|---|
| **SMELL FUNCTION\ODOR** | **PYRD** | **NO₂B** | **THIO** | **AA** | **PYRD** | **NO₂B** | **THIO** | **AA** |
| **DT** | 8.5±0.2*^{,a} | 9.0±0.2^{a} | 8.5±0.2^{a} | 8.9±0.2^{a} | 4.2±0.1 | 3.9±0.1 | 3.6±0.1 | 3.7±0.1 |
| **RT** | 10.2±0.1^{a} | 10.5±0.2^{a} | 10.2±0.2^{a} | 10.7±0.01^{a} | 7.2±0.1 | 5.7±0.1 | 7.1±0.1 | 6.8±0.1 |
| **ME** | 23±2^{a} | 12±1^{a} | 16±2^{a} | 10±1^{a} | 64±3 | 51±4 | 66±4 | 51±4 |
| **H** | -20±2^{a} | 4±1^{a} | -13±2^{a} | 1±1^{a} | -74±3 | 48±5 | -80±2 | 54±5 |

| | | | | | | | | |
|---|---|---|---|---|---|---|---|---|
| ( ) Patient number * Mean±SEM DT, detection threshold, in bottle units [BU ( )] RT, recognition threshold, in bottle units [BU ( )] ME, magnitude estimation, in % ( ) H, hedonic estimation, in % (+, pleasant, -, unpleasant, 0, neutral) PYRD, pyridine; NO₂B, nitrobenzene; THIO, thiophene; AA, amyl acetate With respect to normals a p<0.001 | | | | | | | | |

### Comparison of smell function in patients classified by degree of smell loss

Categorized by smell loss type, results of each measurement indicate that before treatment, patients exhibited a consistent pattern of abnormality associated with their loss type such that Type I hyposmia > Type II > Type III (Table III). H:ME ratios were closer to those of normals in patients with Type III compared to those with Type II hyposmia, as would be expected due to the lesser degree of abnormality in the former patients (Table III).

**TABLE III: COMPARISON OF SMELL FUNCTION BETWEEN UNTREATED PATIENTS WITH TYPES I, II AND III HYPOSMIA**

| **I (96)** | | | | | **II (208)** | | | | **III (8)** | | | |
|---|---|---|---|---|---|---|---|---|---|---|---|---|
| | **PYRD** | **NO₂B** | **THIO** | **AA** | **PYRD** | **NO₂B** | **THIO** | **AA** | **PYRD** | **NO₂B** | **THIO** | **AA** |
| **DT** | 9.8±0.1* | 11.2±0.2 | 10.9±0.2 | 11.7±0.2 | 8.8±0.2 | 8.3±0.2^{a} | 8.0±0.2^{a} | 8.2±0.2^{a} | 4.0±0.5 | 3.4±0.6^{a,a1} | 3.3±0.8^{a,a1} | 2.5±0.5^{a,a1} |
| **RT** | 11.7±0.1 | 11.8±0.1 | 11.8±0.1 | 11.8±0.1 | 9.7±0.1^{a} | 10.1±0.2^{a} | 9.7±0.2^{a} | 10.4±0.2^{a} | 5.6±0.8 | 4.0±0.9^{a,a1} | 3.9±0.8^{a,a1} | 5.3±1.0 |
| **ME** | 0 | 0 | 0 | 0 | 29±2^{a} | 16±1^{a} | 21±2^{a} | 13±1^{a} | 42±4^{a,a1} | 34±4^{a,a1} | 34±10^{a} | 40±2^{a,a1} |
| **H** | 0 | 0 | 0 | 0 | -25±2^{a} | 5±1^{a} | -16±2^{a} | 1±1 | -27±14^{a} | 12±7^{a} | -46±13^{a} | 34±6^{a,a1} |

| | | | | | | | | | | | | |
|---|---|---|---|---|---|---|---|---|---|---|---|---|
| ( ) Patient number * Mean±SEM DT, detection threshold, in BU RT, recognition threshold, in BU ME, magnitude estimation, in % H, hedonic estimation, in % PYRD, pyridine; NO₂B, nitrobenzene; THIO, thiophene; AA, amyl acetate | | | | | | | | | | | | |

| With respect to Type I | With respect to Type II |
|---|---|
| a p< 0.001 | a1 p<0.001 |

### Changes in smell function in all patients treated with theophylline, 200mg daily

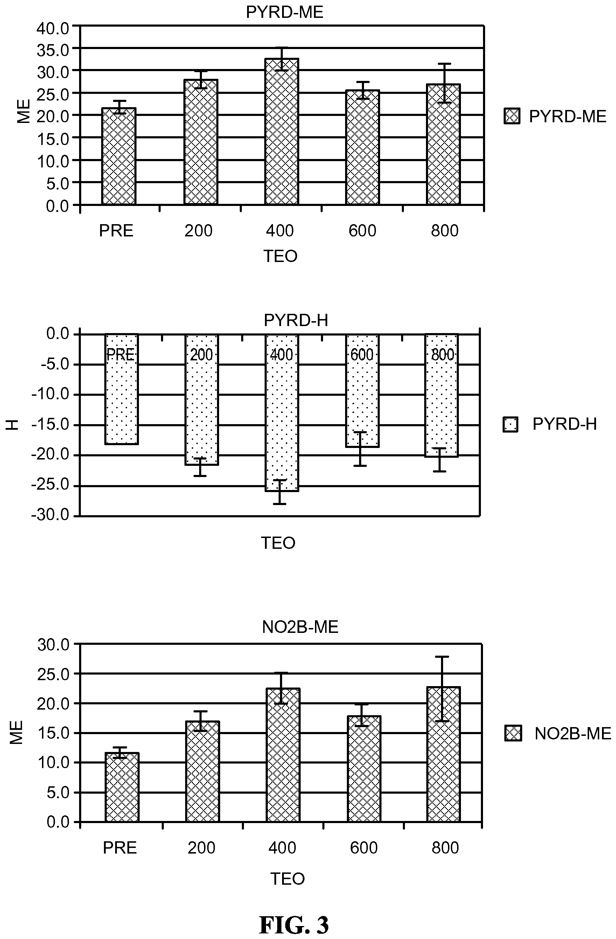

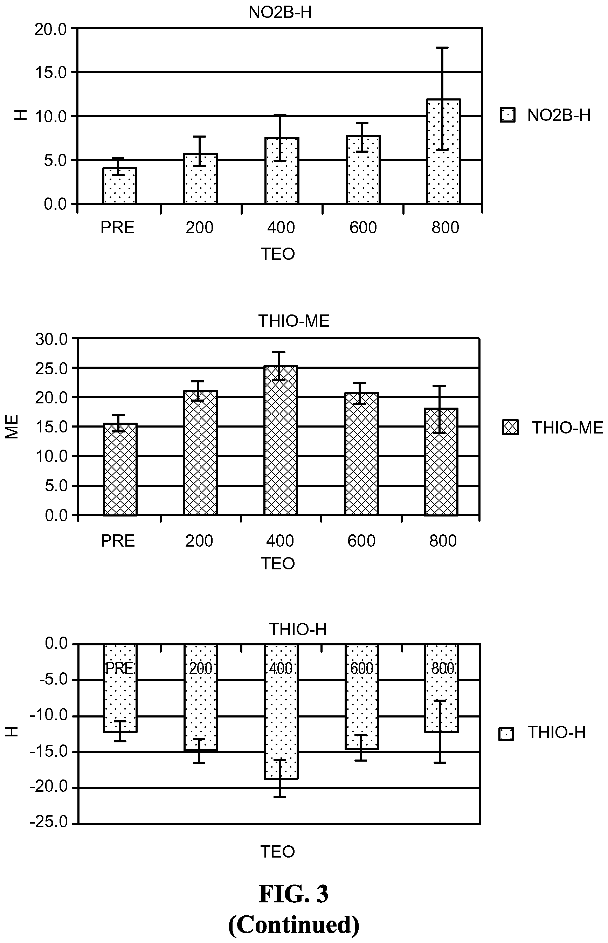

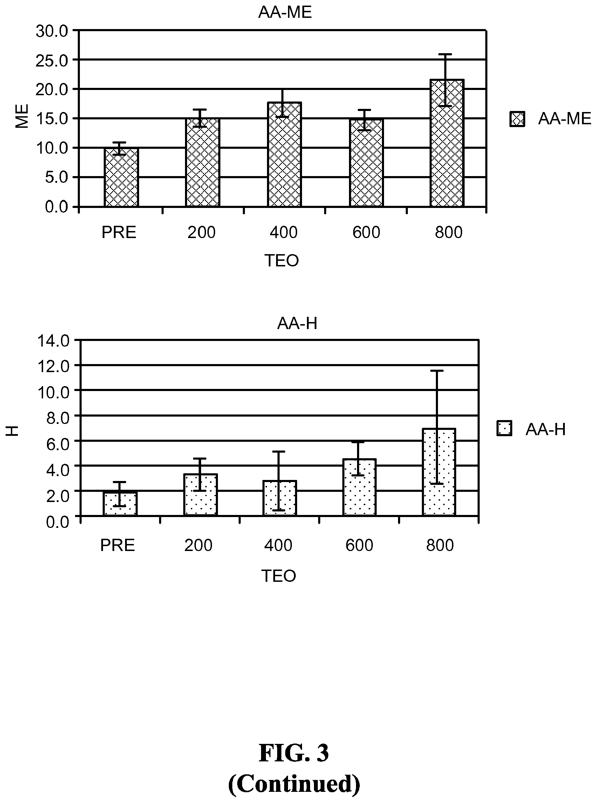

Changes in smell function in 199 patients with hyposmia after treatment with 200mg daily of theophylline for two - six months are shown in Table IV and in Fig. 3. These patients constituted mainly local patients minus 15 of the original 312 who did not return after treatment was initiated. Of the 15 non-returning patients, seven reported >5% improvement (46.7%) and one (14.3%) reported a return to normal; eight reported improvement of <5%. With returning patients, 34 patients (17.1%) improved ≥5% (and were not included in subsequent studies). Among this group nine (26.5%) considered their smell function had returned to normal. Two hundred sixty-three patients improved <5% (160 local and 103 distant patients); among this group 36 patients did not continue in the study.

Among the 199 patients (both improved and not improved) who returned on 200mg, DT and RT for all odors decreased (improved) significantly (Table IV). ME for pyridine and amyl acetate also increased (improved) significantly (Fig. 3). H values for pyridine and thiophene decreased significantly [e.g., odors pyridine and thiophene were recognized as more unpleasant (Fig. 3)] and H values for nitrobenzene increased significantly [e.g., the odor of nitrobenzene was recognized as more pleasant (Fig. 3)]. While not statistically significant, H values for amyl acetate increased 50% (perceived as more pleasant with improved odor recognition). On treatment, H:ME ratios did not change significantly since both ME and H values changed to similar degrees. No differences in results with respect to age or gender of these patients were apparent. At subsequent visits over the next six - 36 months with continued treatment on this dose, the 34 patients with initially improved smell function maintained their increased acuity or improved further. Mean serum theophylline on this treatment was 4.0±0.2 mg/dl.

**TABLE IV: CHANGES IN SMELL FUNCTION FOLLOWING TREATMENT WITH ORAL THEOPHYLLINE, 200MG DAILY**

| | **Before Treatment (199)** | | | | **After Treatment (199)** | | | |
|---|---|---|---|---|---|---|---|---|
| **SMELL FUNCTION\ODOR** | **PYRD** | **NO₂B** | **THIO** | **AA** | **PYRD** | **NO₂B** | **THIO** | **AA** |
| **DT** | 8.5±0.2* | 9.0±0.2 | 8.5±0.2 | 8.9±0.2 | 7.6±0.2^{a} | 7.8±0.2^{d} | 7.5±0.2^{c} | 7.9±0.2^{c} |
| **RT** | 10.2±0.1 | 10.5±0.2 | 10.2±0.2 | 10.7±0.1 | 9.0±02^{a} | 9.5±0.2^{a} | 9.1±0.2^{c} | 9.9±0.2^{c} |
| **ME** | 23±2 | 12±1 | 16±2 | 10±1 | 28±2^{a} | 17±2 | 21±2 | 15±2^{d} |
| **H** | -20±2 | 4±1 | -13±2 | 1±1 | -22±2^{a} | 6±2^{a} | -15±2^{d} | 3±1 |

| | | | | | | | | |
|---|---|---|---|---|---|---|---|---|
| ( ) Patient number * Mean±SEM DT, detection threshold, in BU RT, recognition threshold, in BU ME, magnitude estimation, in % H, hedonic estimation, in % PYRD, pyridine; NO₂B, nitrobenzene; THIO, thiophene; AA, amyl acetate With respect to before treatment a p<0.001 b p<0.005 c p<0.01 d p<0.05 | | | | | | | | |

### Changes in smell function in patients with hyposmia treated with theophylline 400mg daily

One hundred twenty patients (97 from the 165 who returned on <5% on 200mg and 23 who returned for the first time on 400mg) were reevaluated after taking 400mg of theophylline for two - six months (Table IV). On this treatment dose, 35 patients (29.2%) improved ≥5% (and were not included in subsequent studies). Among this group, three (8.6%) considered their smell function had returned to normal. One hundred ninety-two patients improved <5% (96 local and 96 distant patients). Twenty-five patients of these 192 did not continue in the study. On this dose, DT and RT for all odors decreased (improved) significantly and ME for all odors increased (improved) significantly (Table V) (both improved and unimproved patients included). Hedonic values also increased significantly for pyridine and thiophene [e.g., odors of pyridine and thiophene were recognized as more unpleasant (Fig. 2)]. While not statistically significant, H values for nitrobenzene increased 50% (perceived as more pleasant); H values for thiophene decreased 40% (perceived as more unpleasant). Overall, H:ME ratios did not change significantly on treatment. No differences in results with respect to age or gender of these patients were apparent. Among patients who exhibited improvement at this dose, on subsequent visits over six - 36 months, their improvement persisted. Mean serum theophylline on this treatment dose was 7.4±0.4 mg/dl.

**TABLE V: CHANGES IN SMELL FUNCTION FOLLOWING TREATMENT WITH ORAL THEOPHYLLINE, 400MG DAILY**

| | **Before Treatment (120)** | | | | **After Treatment (120)** | | | |
|---|---|---|---|---|---|---|---|---|
| **SMELL FUNCTION\ODOR** | **PYRD** | **NO₂B** | **THIO** | **AA** | **PYRD** | **NO₂B** | **THIO** | **AA** |
| **DT** | 8.4±0.2* | 9.0±0.3 | 8.6±0.3 | 8.9±0.3 | 7.0±0.3^{c} | 7.2±0.3^{c} | 6.8±0.4^{c} | 7.1±0.3^{b} |
| **RT** | 10.3±0.2 | 10.5±0.2 | 10.5±0.2 | 10.6±0.2 | 8.5±0.3^{a} | 8.9±0.3^{b} | 8.4±0.3^{a} | 9.3±0.3^{b} |
| **ME** | 21±2 | 12±2 | 14±2 | 10±1 | 33±3^{a} | 23±2^{a} | 26±2^{c} | 18±2^{c} |
| **H** | -19±2 | 4±1 | -12±2 | 2±1 | -26±3^{b} | 8±3^{a} | -19±2^{d} | 3±2 |

| | | | | | | | | |
|---|---|---|---|---|---|---|---|---|
| ( ) Patient number * Mean±SEM DT, detection threshold, in BU RT, recognition threshold, in BU ME, magnitude estimation, in % H, hedonic estimation, in % PYRD, pyridine; NO₂B, nitrobenzene; THIO, thiophene; AA, amyl acetate With respect to before treatment a p<0.001 b p<0.005 c p<0.01 d p<0.05 | | | | | | | | |

### Change in smell function in patients with hyposmia treated with theophylline, 600mg daily

Changes in smell function in 160 patients after treatment with 600mg daily for two - 12 months are shown on Table VI. This patient number includes 77 distant patients who returned on this theophylline dose as well as 83 local patients who improved <5% on 400mg. Among this group, 66 (41.2%) improved ≥5% and 17 (10.6%) considered their smell function had returned to normal. On subsequent visits, over the next six - 36 months, improvement on this dose either persisted or improved further. One hundred thirty-seven patients improved <5% and 73 did not continue. On this dose, DT and RT for all odors decreased (improved) significantly and ME for all odors increased (improved) significantly (Table VI) (both improved and unimproved patients included). H values did not change significantly for any odor, although pyridine and thiophene were recognized as more unpleasant (Fig. 3) and nitrobenzene and amyl acetate were recognized as more pleasant (Fig. 3). Overall, H:ME ratios did not change significantly. Again, as noted on the previous theophylline doses, no differences were apparent with respect to age or gender of these patients. Mean serum theophylline on this dose was 9.4±0.38mg/dl.

**TABLE VI: CHANGES IN SMELL FUNCTION FOLLOWING TREATMENT WITH ORAL THEOPHYLLINE, 600MG DAILY**

| | **Before Treatment (160)** | | | | **After Treatment (160)** | | | |
|---|---|---|---|---|---|---|---|---|
| **SMELL FUNCTION\ODOR** | **PYRD** | **NO₂B** | **THIO** | **AA** | **PYRD** | **NO₂B** | **THIO** | **AA** |
| **DT** | 8.4±0.2* | 9.2±0.2 | 8.8±0.2 | 9.3±0.2 | 7.4±0.2^{c} | 7.8±0.3^{a} | 7.1±0.3^{a} | 7.8±0.3^{a} |
| **RT** | 10.3±0.2 | 10.5±0.2 | 10.3±0.2 | 10.8±0.2 | 9.4±0.2^{c} | 9.1±0.2^{c} | 9.0±0.3^{c} | 9.6±0.2^{a} |
| **ME** | 19±2 | 11±1 | 13±2 | 8±1 | 26±2^{d} | 18±2^{d} | 21±2^{d} | 15±2^{c} |
| **H** | -16±2 | 3±1 | -9±2 | 2±1 | -19±2 | 8±2^{d} | -14±2 | 4±1 |

| | | | | | | | | |
|---|---|---|---|---|---|---|---|---|
| ( ) Patient number * Mean±SEM DT, detection threshold, in BU RT, recognition threshold, in BU ME, magnitude estimation, in % H, hedonic estimation, in % PYRD, pyridine; NO₂B, nitrobenzene; THIO, thiophene; AA, amyl acetate With respect to before treatment a p<0.001 b p<0.005 c p<0.01 d p<0.05 | | | | | | | | |

### Change in smell function in patients with hyposmia treated with theophylline, 800mg daily

Changes in smell function in 28 patients after treatment with 800mg daily for two - 12 months are shown in Table VII. Among this group, 15 (53.6%) improved ≥5% and three (33%) considered their smell function had returned to normal. On subsequent visits over the next two - six months, improvement on this dose persisted or improved further. Thirteen patients improved <5%. At this dose, the study terminated. DT and RT for all odors increased on treatment, but were statistically significant only for DT for nitrobenzene, RT for nitrobenzene and amyl acetate. ME for all odors increased, but was significant only for amyl acetate. H for both pyridine and thiophene decreased 55% and 37%, respectively (became more unpleasant) and H for nitrobenzene and amyl acetate increased about 50% (became more pleasant). When analyzed by paired t test (data not shown) DT, RT and ME for all odors increased significantly, H for pyridine and thiophene decreased significantly and H for nitrobenzene and amyl acetate increased significantly. Mean serum theophylline on this dose was 11.2±0.8mg/dl.

As drug doses increased mean DT and RT for most odors decreased (acuity increased) whereas, mean ME for most odors increased from 200mg to 400mg and then remained relatively constant. H also decreased from 200mg to 400mg (increased unpleasantness) for pyridine and thiophene and then remained relatively constant as doses increased; H for nitrobenzene and amyl acetate (increased pleasantness) increased in a similar manner.

**TABLE VII: CHANGES IN SMELL FUNCTION FOLLOWING TREATMENT WITH ORAL THEOPHYLLINE, 800MG DAILY**

| | **Before Treatment (28)** | | | | **After Treatment (28)** | | | |
|---|---|---|---|---|---|---|---|---|
| **SMELL FUNCTION\ODOR** | **PYRD** | **NO₂B** | **THIO** | **AA** | **PYRD** | **NO₂B** | **THIO** | **AA** |
| **DT** | 7.9±0.6* | 9.3±0.7 | 8.9±0.6 | 8.8±0.7 | 6.9±0.6 | 7.0±0.8^{d} | 7.2±0.7 | 7.2±0.8 |
| **RT** | 10.6±0.3 | 10.1±0.6 | 10.2±0.5 | 10.9±0.3 | 8.5±0.6^{c} | 7.8±0.8^{d} | 9.6±0.6 | 9.0±0.5^{b} |
| **ME** | 18±4 | 11±4 | 14±4 | 10±4 | 29±5 | 25±6 | 20±4 | 24±2^{a} |
| **H** | -12±4 | 6±2 | -9±4 | 4±4 | -22±6 | 13±7 | -15±4 | 8±5 |

| | | | | | | | | |
|---|---|---|---|---|---|---|---|---|
| ( ) Patient number * Mean ± SEM DT, detection threshold, in BU RT, recognition threshold, in BU ME, magnitude estimation, in % H, hedonic estimation, in % PYRD, pyridine; NO₂B, nitrobenzene; THIO, thiophene; AA, amyl acetate With respect to before treatment a p<0.001 b p<0.005 c p<0.01 d p<0.05 | | | | | | | | |

### Changes in smell function and after treatment with theophylline in patients classified by hyposmia type

Type I Hyposmia Treatment. After treatment with either 200mg, 400mg, 600mg or 800mg, there were significant decreases in DT and RT, increases in ME and changes in H for specific odors consistent with improvement in smell function (Table VIII). Of the 96 patients with Type I hyposmia in the study, 32 (33.3%) reported >5% improvement and 5 (15.6%) reported their smell function had returned to normal.

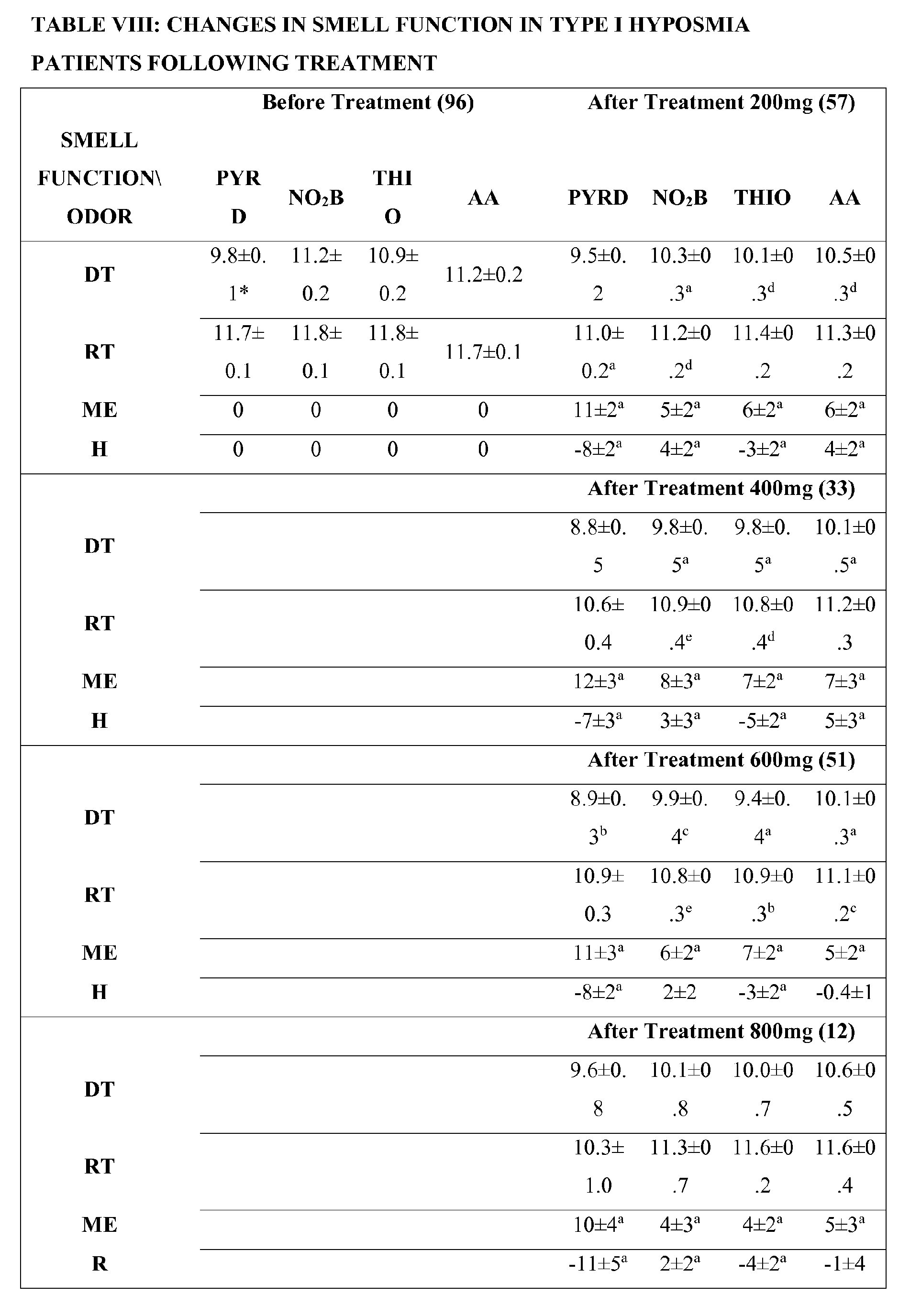

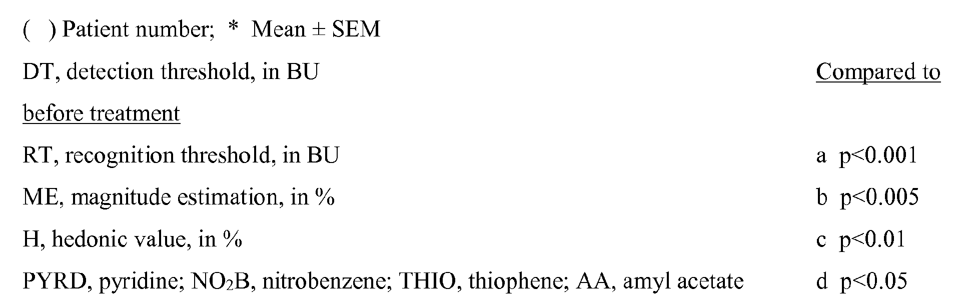

Type II Hyposmia Treatment. After treatment with either 200mg, 400mg, 600mg or 800mg, there were significant decreases in DT and RT, increases in ME and changes in H for specific odor consistent with improvement in smell function (Table IX). Of the 208 patients with Type II hyposmia in the study, 129 (62%) reported >5% improvement and 26 (20.2%) reported their smell function had returned to normal.

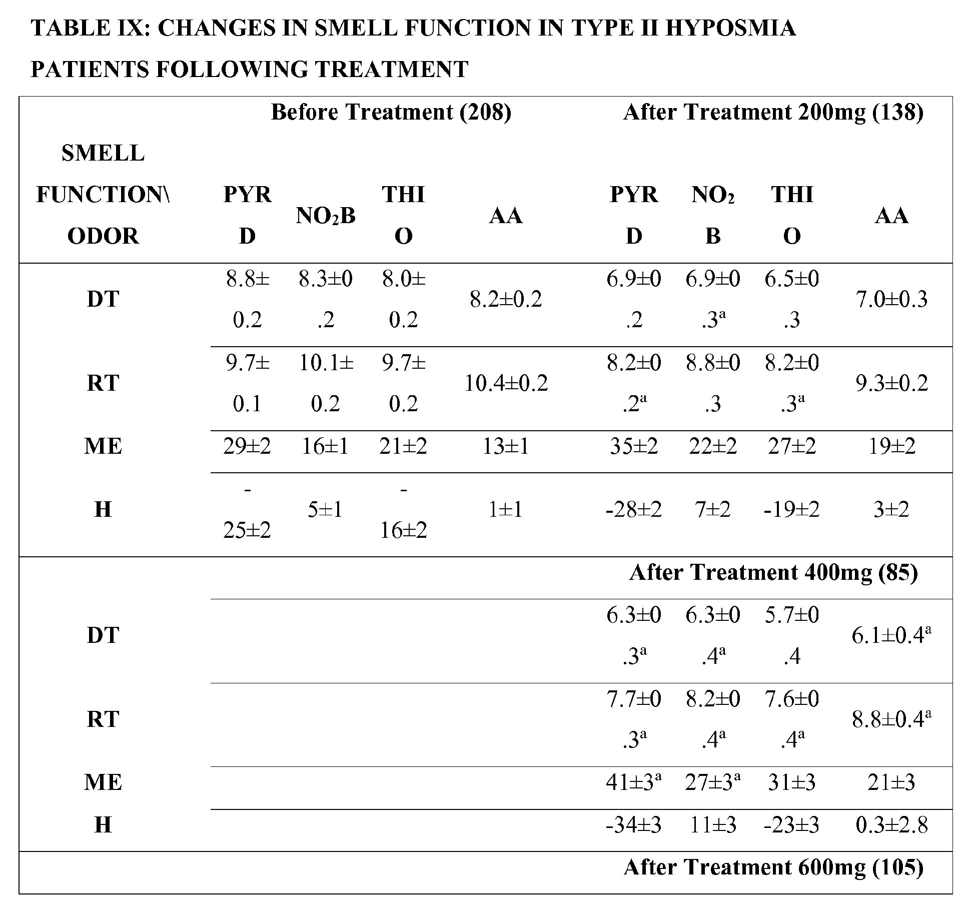

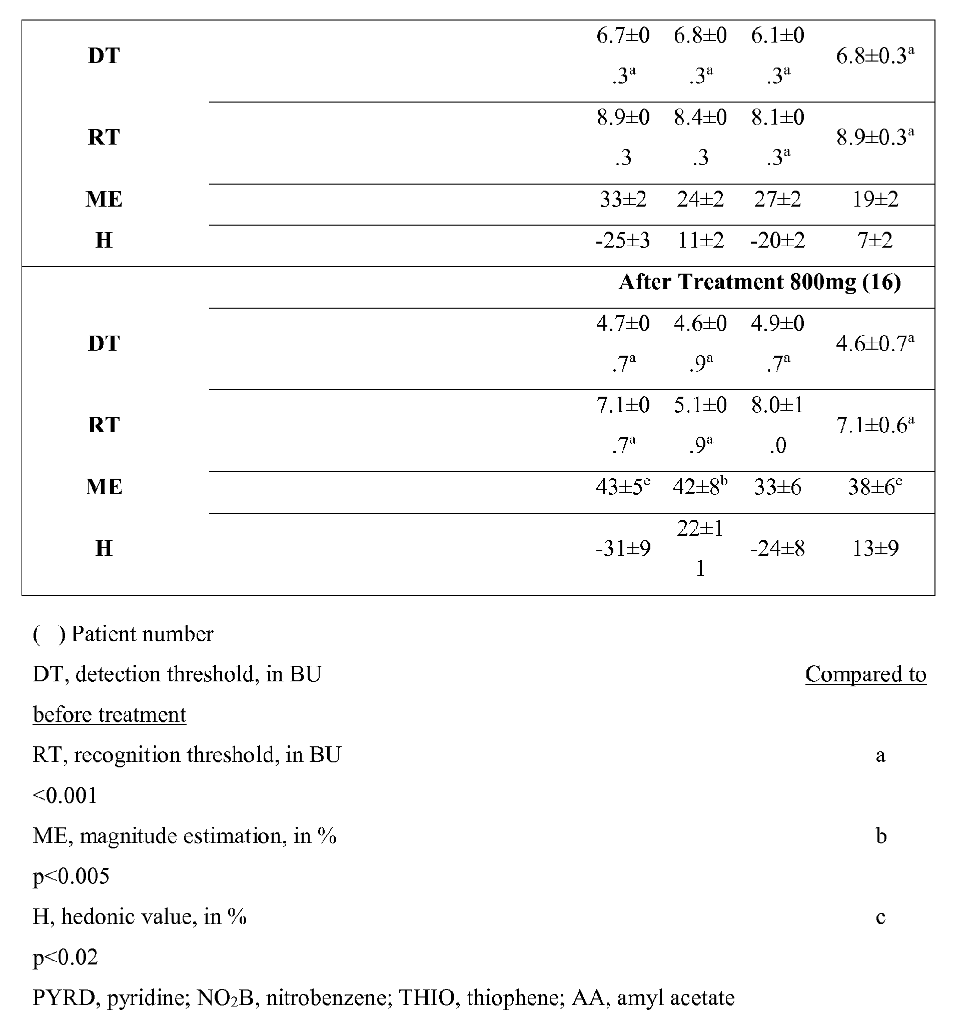

Type III Hyposmia Treatment. After treatment with either 200mg, 400mg or 600mg, there were no significant changes in DT or RT, since these values were not significantly different from normal before treatment. After treatment, there were changes in ME and H, but values were variable due to the small number of patients in each treatment series (Table X). Of the eight patients with Type III hyposmia in the study, 5 (62.5%) reported >5% improvement and 3 (60%) reported their smell function had returned to normal (see Table X).

**TABLE X: CHANGES IN SMELL FUNCTION IN TYPE III HYPOSMIA PATIENTS FOLLOWING TREATMENT**

| | **Before Treatment (8)** | | | | **After Treatment 200mg (4)** | | | |
|---|---|---|---|---|---|---|---|---|
| **SMELL FUNCTION\ODOR** | **PYRD** | **NO₂B** | **THIO** | **AA** | **PYRD** | **NO₂B** | **THIO** | **AA** |
| **DT** | 4.0±0.5 | 3.4±0.6 | 3.3±0.8 | 2.5±0.5 | 4.0±0.6 | 3.8±0.7 | 3.2±0.8 | 2.8±0.5 |
| **RT** | 5.6±0.8 | 4.0±0.9 | 3.9±0.8 | 5.3±1.0 | 5.2±0.5 | 5.0±0.6 | 3.8±0.6 | 5.2±1.6 |
| **ME** | 42±4 | 34±4 | 34±10 | 40±2 | 45±11 | 33±8 | 47±15 | 32±10 |
| **H** | 27±14 | 12±7 | 46±13 | 34±8 | 40±13 | 13±12 | 40±18 | 14±13 |

| | | | | | **After Treatment 400mg (2)** | | | |
|---|---|---|---|---|---|---|---|---|
| | | | | | | | | |
| **DT** | | | | | 5.0 | 2.5 | 1 | 2 |
| **RT** | | | | | 5.0 | 4.0 | 1 | 3 |
| **ME** | | | | | 45 | 79 | 99 | 75 |
| **H** | | | | | -40 | -62 | -99 | 75 |

| | | | | | **After Treatment 600mg (4)** | | | |
|---|---|---|---|---|---|---|---|---|
| | | | | | | | | |
| **DT** | | | | | 3.8±0.9 | 3.8±0.9 | 2.0±1.0 | 2.5±1.0 |
| **RT** | | | | | 5.5±0.9 | 4.5±1.3 | 3.8±0.9 | 4.8±1.7 |
| **ME** | | | | | 41±15 | 33±13 | 42±20 | 31±17 |
| **H** | | | | | 26±22 | 12±16 | 35±24 | 28±19 |

| | | | | | | | | |
|---|---|---|---|---|---|---|---|---|
| ( ) Patient number DT, detection threshold, in BU RT, recognition threshold, in BU ME, magnitude estimation, in % H, hedonic value, in % PYRD, pyridine; NO₂B, nitrobenzene; THIO, thiophene; AA, amyl acetate | | | | | | | | |

### DISCUSSION

These results indicate that 157 of the 312 patients in the study (50.3%) were subjectively responsive to treatment with theophylline. Of these, 34 (21.7%) considered their smell function returned to normal levels. Overall, 10.9% of all patients in the study considered their smell function had returned to normal. Improvement in smell function, once occurred, persisted and sometimes continued to improve as long as treatment continued. These results also indicate that a significant number of patients demonstrated significant increases in sensitivity to detection and recognition of odors and increased ability to determine odor intensity. They also demonstrated significant improvement in hedonic responses such that unpleasant odors were significantly more unpleasant and pleasant odors were significantly more pleasant. In addition, pleasant odors which were initially recognized as unpleasant were recognized as pleasant after treatment.

Initial studies were made at two - six month intervals after drug initiation. Subjective responses indicated that return of function was both time and dose related; patients reported little or no improvement before four - six weeks of treatment and reported greater improvement as doses increased to 400mg and especially to 600mg and 800mg.

Patients with smell loss exhibited varying degrees of loss prior to treatment, however, improvement was noted in patients regardless of degree of loss. Patients with lesser degrees of smell loss (Type II and III hyposmia) exhibited more improvement with more patients reporting a return to normal smell function on treatment than those with a more severe degree of loss (Type I hyposmia). Indeed, in terms of percent patients reporting a return to normal function, twice as many patients with Type II and III hyposmia compared to Type I reported a return to normal function. These results are useful since smell loss degree is related to severity of biochemical changes (35) responsible for the loss. This result lends credence to the smell loss classification previously devised (1, 2, 56) and alerts physicians treating these patients that patients with a greater degree of smell loss require greater care and diligence with respect to successful treatment responses. Among patients who did not improve (those who responded <5% improvement), increasing the dose of theophylline further to 800mg improved smell function further. In another group of patients who improved <5%, the addition of another PDE inhibitor (*e*.*g*., cilostazol) to their dose of 600mg or 800mg of theophylline improved smell function in an additional 15% of patients.

When analyzed with respect to changes in objective psychophysical measurements of smell function, mean values for DT and RT improved significantly for all odors for each theophylline dose, whether or not subjective improvement occurred (Tables IV, V, VI). These results indicate that treatment with theophylline improved standardized threshold measurements of sensory function, albeit, not enough to be perceived by the patients as significant. While mean DT and RT for all odors improved on treatment, none of these means reached the level of improvement exhibited by normal subjects (*e*.*g*., Tables III, IV, V, VI).

When analyzed with respect to ME on 200mg theophylline (Table IV), only responses to pyridine were significantly greater than prior to treatment. ME for each odor, however, increased with the total mean increase (5.1%) consistent with the overall reported subjective improvement among the successfully treated patients. On 400mg theophylline (Table V), ME for each odor increased significantly with a total mean increase of 10.4%. On 600mg theophylline (Table VI), ME for each odor also increased significantly with a total mean increase of 6.9%. These results are probably more consistent with patient subjective changes, since most patients are less focused on whether or not they can detect or recognize weak (threshold) concentrations of odors. Rather, they are more concerned about the intensity at which odors are perceived. These results are consistent with the subjective responses of the patients.

In general, as noted before, ME and H values in normal subjects are similar, since subjects usually equate pleasantness or unpleasantness of any odor with its intensity, be it pleasant or unpleasant (Table V). For example, pyridine odor of 50% intensity is generally considered as 50% unpleasant whereas amyl acetate odor of 50% intensity is generally considered as 50% pleasant. Patients with hyposmia, however, may not only manifest decreased sensory acuity, but also sensory distortions. This phenomena was observed upon comparison of ME and H values between untreated patients and normals (Table II). The disparate ratios of H:ME reflected among the patients not only manifest decreased acuity (lower ME) but also the bimodal distribution of H values (as discussed previously). This bimodal distribution becomes more apparent for H values as patients recovered their sensory acuity (manifested by increased DT, RT and ME) since there was an even greater divergence of pleasantness-unpleasantness among all odors presented than in normals. Thus, H values among some patients in whom sensory acuity increased, pleasant odors (*e*.*g*., nitrobenzene - bitter almond or marzipan-like or amyl acetate - banana-like) were considered putrid because the distorted aspect of these odors also increased. This type of change may not be as readily apparent with respect to changes in more unpleasant odors (*e*.*g*., pyridine, thiophene), but may also occur with these unpleasant odors considered sweet or fruity as part of the distortion.

On 200mg theophylline, ME values for pyridine and thiophene increased 6% and 5%, respectively, whereas H values decreased (became less unpleasant) 2%, respectively; ME values for nitrobenzene and amyl acetate increased 5%, respectively, whereas H values increased (became more pleasant) only by 2% (Table IV). This effect is better appreciated on 400mg theophylline with ME increases for pyridine and thiophene at 12%, respectively, whereas increases in H values were 9% and 7%, respectively; ME for nitrobenzene and amyl acetate increased 11% and 8%, respectively, whereas H values decreased (became more pleasant) only 4% and 1%, respectively (Table V). Similar results hold for 600mg theophylline also (Table VI).

Differences with respect to subjective responses and changes measured in DT and RT before and after treatment may reflect differences in how patients considered their overall improvement on treatment. A response of ≥5% was chosen to indicate improvement in smell function on treatment. This apparently small response number may in actuality be a conservative estimate of return of smell function since this number is a composite of all odors which the patient considered improved on treatment. Thus, responses to some strong odors (*e*.*g*., gasoline, bleach, ammonia, etc.) may have been considered improved by a great deal, but these responses may have been tempered by the patients' responses to weaker odors (*e*.*g*., flowers, perfume, shampoo, etc.) in which no improvement may have occurred. Therefore, the overall composite which the patient was required to consider, included overall improvement in both strong and weak odors.

Most patients are usually unconcerned whether or not they can detect or recognize weak concentrations of odors (DT or RT), but they are concerned about the intensity (ME) at which odors are perceived. After treatment with 200mg theophylline, the average improvement in ME for all odors was 5.1% consistent with the results reported with respect to subjective responses to treatment (Table IV). After treatment with 400mg, mean ME increased 10.4% consistent with increased response to this dose (Table V) and after treatment with 600mg, mean ME increased 6.9% (Table VI). These results are more consistent with the subjective responses of the patients, although data for DT and RT increased significantly.

Side effects of theophylline among patients were generally minimal. Patients were required to take the drug in divided doses in middle of meals (breakfast and lunch). While this technique delayed drug absorption it did not inhibit absorption. Thus, the more common and usual side effects of nervousness, jitteriness and difficulty in falling asleep were obviated. Mild gastrointestinal upset, tachycardia, nausea, diarrhea, headache and insomnia were occasionally reported but were usually obviated by temporarily decreasing drug intake for a short period and then increasing drug dose to the required amount.

Treatment with theophylline improved smell function probably by acting through its effect as a PDE inhibitor on cAMP and cGMP levels in saliva (31) and nasal mucus (34, 35). As drug dose increased, presumably with increasing PDE inhibition, patient responses also increased. Subsequent increases in cyclic nucleotides may have increased activation of olfactory receptor stem cell growth and maturation, as previously described (20, 21).

This extensive study was performed over an extended time period in an effort to evaluate treatment of smell loss on the basis of a biochemical molecular abnormality as the basic pathology of the loss. Although unblinded, a majority of patients expressed subjective improvement in their sensory function on treatment that was confirmed through objective testing.

### REFERENCES

1. Henkin, R.I. Evaluation and treatment of human olfactory dysfunction, in Otolaryngology (English, G.M. Ed.), Lippincott, Philadelphia, 1993, Vol.2, pp. 1-86.
2. Henkin, R.I. Taste and smell disorders, human. Encyclopedia of Neuroscience, 3rd Ed., (Adelman, G., Smith, B.H., Eds.), Birkhauser, Boston, 2004.
3. Deems, D.A., Doty, R.L., Settle, R.G., Moore-Gillon, V., Shaman, P., Mester, A.F., Kimmelman, C.P., Brightman, V.J., Snow, J.B. Jr. Smell and taste disorders, a study of 750 patients from the University of Pennsylvania Smell and Taste Center. Arch. Otolaryngol. Head Neck Surg. 1991;177:519-528.
4. Wysocki, C.J., Gilbert, A.N. National Geographic Smell Survey: Effects of age are heterogeneous. Ann. NY Acad. Sci. 1989;561:12-28.
5. Temmel, A.F.P., Quint, C., Schickinger-Fischer, B., Klimek, L., Stoller, E., Hummel, T. Characteristics of olfactory disorders in relation to major causes of olfactory loss. Arch. Otolaryngol. Head Neck Surg. 2002;128:635-641.
6. Cullen, M., Leopold, D. Disorders of smell and taste. Med. Clin. North Amer. 1999;83:57-74.
7. Bromley, S.M. Smell and taste disorders: a primary care approach. Amer. Fam. Physician. 2000;61:427-436.
8. Seiden, A.M., Duncan, H.J., Smith, D.V. Office management of taste and smell disorders. Otolaryngol. Clin. North Amer. 1992;25:817-835.
9. Davidson, T.M., Murphy, C., Jalowayski, A.A. Smell impairment: can it be reversed? Postgrad. Med. 1995;98:107-109, 112-118.
10. Harris, R., Davidson, T.M., Murphy, C., Gilbert, P.E., Chem, M. Clinical evaluation and symptoms of chemosensory impairment: one thousand consecutive cases from the Nasal Dysfunction Clinic in San Diego. Amer. J. Rhinol. 2006;20:101-108.
11. Henkin, R.I. Effects of ACTH, adrenocorticosteroids and thyroid hormone on sensory function, in Anatomical Neuroendocrinology, (Stumpf, W.E., Grant, L.D., Eds.), Karger, A.G. , Basel, 1975, pp.298-316.
12. Henkin, R.I. The role of adrenal corticosteroids in sensory processes, in Adrenal Gland, (Blaschko, H., Sayers, G., Smith, A.D., Eds.), Handbook of Physiology. Endocrinology, Washington, DC. Amer. Physiol. Soc., Sect. 7, Vol. VI, 1975, pp. 209-230.
13. Henkin, R.I. Zinc, saliva and taste: Interrelationships of gustin, nerve growth factor, saliva and zinc, in Zinc and Copper in Clinical Medicine, (Hambidge, K.M., Nichols, B.L., Eds.), Spectrum Publ. Inc., Jamaica, NY, 1978, pp. 35-48.
14. Henkin, R.I., Lippoldt, R.E., Bilstad, J., Edelhoch, H. A zinc protein isolated from human parotid saliva. Proc. Nat. Acad. Sci. USA 1975;72:488-492.
15. Henkin, R.I., Lippoldt, R.E., Bilstad, J., Wolf, R.O., Lum, C.K.L., Edelhoch, H. Fractionation of human parotid saliva. J. Biol. Chem. 1978;253:7556-7565.
16. Henkin, R.I., Doherty, A.E., Martin, B.M. Nasal seroproteins: a new frontier in the exploration of physiology and pathology of nasal and sinus disease. New Frontiers in Immunobiology in Otolaryngology (Veldman, J.E., Passali, D., Lim, D.J., Eds.), Kugler, The Hague, 2000, pp.127-152.
17. Henkin, R.I., Martin, B.M., Agarwal, R.P. Decreased parotid saliva gustin/carbonic anhydrase VI secretion: an enzyme disorder manifested by gustatory and olfactory dysfunction. Amer. J. Med. Sci. 1999;318:380-391.
18. Doerty, A.E., Matin, B.M., Dai, W.L., Henkin, R.I. Carbonic anhydrase (CA) activity in nasal mucus appears to be a marker for loss of smell (hyposmia) in humans. J. Invest. Med. 1997;45:237A.
19. Henkin, R.I., Martin, B.M., Agarwal, R.P. Efficacy of exogenous zinc in treatment of patients with carbonic anhydrase VI deficiency. Amer. J. Med. Sci. 1999;318:392-404.
20. Henkin, R.I., Velicu, I. Age related changes in cyclic nucleotides in saliva and nasal mucus - possible feedback mechanism in development of gustatory and olfactory receptor function. FASEB J. 2005;19:A1368.
21. Papathanassiu, A., Henkin, R.I. cAMP is present in human nasal mucus and may act as a growth factor in cells of the olfactory epithelium. FASEB J. 2002;16:A1153.
22. Asakura, K., Kataura, A. cAMP and cGMP in the human parotid saliva. Arch. Otorhinolaryngol. 1980;226:1529-30.
23. Schaeffer, L.D., Sproles, A., Krakowski, A. Detection of cAMP in parotid saliva of normal individuals. J. Dent. Res. 1973;52:629.
24. Glenert, U., Geisler, A. A single assay for cyclic adenosine 3':5'-monophosphate in human saliva. J. Cyclic Nucleotide Protein Phosphor. Res. 1985;10:451-461.
25. Hanamori, T., Nagotsu, T., Matsumoto, S. Origin of cyclic adenosine monophosphate in saliva. J. Dent. Res. 1975;54:535-539.
26. Rosenzweig, S., Yan, W., Sasso, M., et al. Possible novel mechanism for bitter taste mediated through cGMP. J. Neurophysiol. 1999;81:1661-5.
27. Pace, U., Hanski, E., Salomon, Y, et al. Odorant-sensitive adenylate cyclase may mediate olfactory reception. Nature. 1985;316:255-8.
28. Anholt, R.R.H. Molecular neurobiology of olfaction. Crit. Rev. Neurobiol. 1993;7:1-22.
29. Firestein, S., Zufall, F., Sheperd, G.M. Single odor-sensitive channels in olfactory receptor neurons are also gated by cyclic nucleotides. J. Neurosci. 1991;11:3565-72.
30. Moon, C., Simpson, P.J., Cho, H., et al. Regulation of intracellular cyclic GMP levels in olfactory sensory neurons. J. Neurochem. 2005;95:200-9.
31. Henkin R.I., Velicu I., Papathanasiu A. cAMP and cGMP in human parotid saliva: relationships to taste and smell dysfunction, gender and age. Amer. J. Med. Sci. 2007;334:431-440.
32. Henkin, R.I., Velicu, I. cAMP and cGMP in nasal mucus: relationships to taste and smell dysfunction, gender and age. Clinical Invest. Med. 2008;31:E71-E77.
33. Henkin, R.I. The definition of primary and accessory areas of olfaction as the basis for a classification of decreased olfactory acuity, in Olfaction and Taste II, (Hayashi, T. Ed.), Pergamon Press, London, 1967, pp. 235-252.
34. Henkin, R.I., Velicu, I. Decreased parotid salivary cyclic nucleotides related to smell loss severity in patients with taste and smell dysfunction. Metabolism. 2009; in press.
35. Henkin, R.I., Velicu, I. cAMP and cGMP in nasal mucus related to severity of smell loss in patients with smell dysfunction. Clinical Invest. Med. 2008;31:E78-E84.
36. Cai, D., Qiu, J., Cao, Z., McAtee, M., Bregman, B.S., Filben, M.T. Neuronal cyclic AMP controls the developmental loss in ability of axons to regenerate. J. Neurosci. 2001;21:4731-4739.
37. Neumann, S., Bradke, F., Tessier-Lavigne, M., Basbaum, A.I. Regeneration of sensory axons within the injured spinal cord induced by intraganglionic cAMP elevation. Neuron. 2002;34:885-893.
38. Cai, D., Shen, Y., DeBellard, M., Tang, S., Filben, M.T. Prior exposure to neurotrophins blocks inhibition of axonal regeneration by MAG and myelin via a cAMP dependent mechanism. Neuron. 1999;22:89-101.
39. Kurihara, K., Koyama, N. High activity of adenylyl cyclase in olfactory and gustatory organs. Biochem. Biophys. Rev. Comm. 1972;48:30-34.
40. Pace, U., Hanski, E., Salomon, Y., Lancet, D. Odorant-sensitive adenylate cyclase may mediate olfactory reception. Nature. 1985;316:255-258.
41. Moon, C., Simpson, P.J., Cho, H., Ronnett, G.Y. Regulation of intracellular cyclic GMP levels in olfactory sensory neurons. J. Neurochem. 2005;95:205-209.
42. Shepherd, G.M. Sensory transduction entering the mainstream of membrane signaling. Cell. 1991;67:845-851.
43. Thompson, W.J. Cyclic nucleotide phosphodiesterase: pharmacology, biochemistry and function. Pharmacol. Ther. 1991;51:13-33.
44. Firestein, B.I., Bredt, D.S. Regulation of sensory neuron precursor proliferation by cyclic GMP-dependent protein kinase. J. Neurochem. 1998;71:1846-1853.
45. Anholt, R.R.H. Molecular neurobiology of olfaction. Crit. Rev. Neurobiol. 1993;7:1-22.
46. Henkin, R.I., Velicu, I., Papathanasiu, A. Dichotomous changes in cAMP and cGMP in human parotid saliva after oral theophylline. FASEB J. 2003;17:A1028.
47. Velicu, I., Henkin, R.I. On the antiapoptotic mechanism of action of theophylline in restoring smell function in patients with hyposmia. J. Invest. Med. 2005;53(Suppl. 2):S402.
48. Levy, L.M., Henkin, R.I., Hutter, A, Lin, C.S., Schellinger, D. Increased brain activation in response to odors in patients with hyposmia after theophylline treatment demonstrated by fMRI. J. Comp. Asst. Tomog. 1998;22:760-770.
49. Henkin, R.I., Velicu, I., Schmidt, L. Effective treatment of smell loss with theophylline. Exper. Biol. 2008;22:B976.2.
50. Henkin, R.I., Larson, A.L., Powell R.D. Hypogeusia, dysgeusia, hyposmia and dysosmia following influenza-like infection. Ann. Otol. Rhin. Laryngol. 1975;84:672-682.
51. Church, J.A., Bauer, H., Bellanti, J.A., Satterly, R.A., Henkin, R.I. Hyposmia associated with atopy. Ann. Aller. 1978;40:105-109.
52. Schechter, P.J., Henkin, R.I. Abnormalities of taste and smell following head trauma. J. Neurol. Neurosurg. Psychiat. 1974;37:802-810.
53. Henkin, R.I., Schecter, P.J., Friedewald, W.T., DeMets, D.L., Raff, M.S. A double blind study of the effects of zinc sulfate on taste and smell dysfunction. Amer. J. Med. Sci. 1976;272: 285-299.
54. Henkin, R.I., Schechter, P.J., Hoye, R.C., Mattern, C.F.T. Idiopathic hypogeusia with dysgeusia, hyposmia and dysosmia: a new syndrome. J. Amer. Med. Assoc. 1971;217:434-440.
55. Schechter, P.J., Friedwald, W.T., Bronzert, D.A., Raff, M.S., Henkin, R.I. Idiopathic hypogeusia: a description of the syndrome and a single blind study with zinc sulfate, in Internat. Rev. Neurobiol. Suppl. 1., (Pfeiffer, C., Ed.), Academic Press, NY, 1972, pp. 125-133.
56. Henkin, R.I. The definition of primary and accessory areas of olfaction as the basis for a classification of decreased olfactory acuity, in Olfaction and Taste II, (Hayashi, T. Ed.), Pergamon Press, London, 1967, pp. 235-252.

### EXAMPLE 2

Theophylline treatment restored smell function in over 50% of the hyposmic patients in Example 1. This study, however, was an open label clinical trial and not all patients responded to the drug. These results raise questions about the character of the study and the efficacy of the drug to correct the smell loss.

In an effort to understand more about these results, levels of cAMP and cGMP in saliva before and after theophylline treatment were studied in patients who participated in the clinical study of Example 1. Cyclic nucleotide levels were not assayed until the entire analysis of the clinical trial results was completed.

### METHODS

Thirty-one patients, aged 29-85y (56±3y, Mean±SEM) from among the 312 patients who participated in the open label, fixed design clinical trial treated with theophylline of Example 1 were studied. There were 13 men, aged 54±3y and 18 women, aged 58±4y. All patients exhibited hyposmia. Six had Type I hyposmia, 25 had Type II hyposmia. Patients had a variety of etiologies for their hyposmia; six had PVIL and hypogeusia, thirteen had allergic rhinitis, nine had a head injury, and three had a variety of the etiologies contributing to their loss including a drug reaction, an idiopathic cause and post chemotherapy.

Measurements of smell function were obtained for each patient by use of a standard three stimuli forced choice technique using four odors (pyridine, nitrobenzene, thiophene and amyl acetate as described in Example 1. Subjective measurements of smell function were also obtained for each patient by use of standard technique in which smell acuity was graded on a scale from 0-100 with 0 indicating an absence of overall smell function and 100 indicating normal smell function as described in Example 1.

Parotid saliva was collected from each patient by application of a Lashley cup over Stensen's duct with lingual stimulation by placement of concentrated lemon juice. Saliva was collected in plastic tubes and stored at -20°C until assayed. cAMP and cGMP were measured in saliva by a sensitive 96 plate spectrophotometric assay (R&D Systems, Minneapolis, MN).

All patients were placed in a fixed design open label clinical trial with treatment with oral theophylline. Treatment consisted of fixed treatment periods of two-eight months with sequential doses of the drug at 200mg, 400mg and 600mg. At termination of each of these intervals, patients returned to study site for reevaluation. At the end of each interval subjective responses to treatment were measured with a modification of the 0-100 scale previously used (vs). Subjective responses to treatment were graded on a sliding scale from -100 - 0 - +100 with patients recording improvement (+0 - +100), no improvement (0) or worsening (0 - -100) of their overall smell function. If overall smell function improved ≥5% they were considered to improve clinically (1). If smell function improved <5% they were considered not to improve (1). At the end of each interval subjective measurements of smell function, measurements of DT, RT, ME and H and measurements of parotid saliva for levels of cAMP and cGMP were obtained. In addition, blood plasma was obtained by venipuncture, placed in ice into zinc free tubes which contained 100mg zinc free heparin, centrifuged at 3000 rpm for 10-20 min, the plasma transferred to plastic PCR tubes and stored at -20°C until assayed. Theophylline was assayed by a fluorescence polarization assay (Abbott, Chicago, IL), as previously described.

At each return visit, if patients noted improvement in their overall smell function (see Example 1) they continued on this same drug dose and were not included in any further data. If smell function did not improve on 200mg of theophylline, their dose was increased to 400mg and they returned to study site after an additional two-four months of treatment. These same measurement processes occurred after treatment with 400mg and 600mg of theophylline.

Data for the cyclic nucleotides from these 31 patients was not analyzed until the entire study of the 312 patients in Example 1 was assembled and analyzed in its entirety. After completion of these analyses, all data for parotid saliva cAMP and cGMP from all patients in whom salivary cAMP and cGMP were obtained were assembled. Levels of salivary cyclic nucleotides varied widely. Initially, without external criteria, there was no way to understand these disparate data. To assist in understanding these data, each data point was independently identified with respect to which patient from whom it was obtained and categorized as to whether or not that patient demonstrated improvement or lack of improvement in both subjective smell function and in objective measurements of smell function (DT, RT, ME, H) on theophylline treatment. On this basis, 20 patients were identified as having improved smell function and 11 did not improve. In a similar manner, measurements of each patient's plasma theophylline level at each dosage of theophylline (at 200mg, 400mg, 600mg of theophylline) were categorized. The two patient groups were also analyzed post hoc with respect to type of smell loss and etiology of smell loss.

Differences in the characteristics between these two patient groups were analyzed with respect to each measurement obtained (Mean±SEM). Differences between mean±SEM were analyzed using Student t test and X2; differences of p≤5% were considered significant.

### RESULTS

Post hoc analysis demonstrated that initial values of subjective smell function and measurements of smell function (DT, RT, ME, H) from these two patient groups prior to theophylline treatment did not differ. At baseline, there were also no significant differences in saliva cAMP and cGMP between improved and unimproved patients (Table XI). Levels of saliva cAMP prior to treatment were consistent with mean values obtained for saliva cAMP in all 312 patients included in the original study. After treatment with 200mg of theophylline, cAMP levels in the improved patients increased 10% above baseline, albeit not significantly; there was essentially no change among the unimproved patients (Table I). After treatment with 400mg of theophylline, however, although values were not statistically significant compared to before treatment, cAMP levels increased 40% over baseline in the improved group, whereas there was essentially no change among the unimproved patients. After treatment with 600mg, cAMP levels increased significantly to 67% above initial cAMP values in the improved patients, but there was essentially no change among unimproved patients. After treatment with 600mg, levels of cAMP in the improved patients were still below the mean of saliva cAMP reported in normal subjects.

**TABLE XI: RESULTS FOR PATIENTS WITH IMPROVED SMELL FUNCTION**

| BEFORE | THEOPHYLLINE TREATMENT | | | BEFORE | THEOPHYLLINE TREATMENT | | |
|---|---|---|---|---|---|---|---|
| TREATMENT | cAMP (pmol/ml) | | | TREATMENT | cGMP (pmol/ml) | | |
| (pmol/ml) | 200mg | 400mg | 600mg | (pmol/ml) | 200mg | 400mg | 600mg |
| 0.87±0.14* | 0.96±0.13 | 1.22±0.33 | 1.45^{b}±0.37 | 0.08±0.016 | 0.08±0.016 | 0.22^{a}±0.07 | 0.27^{a}±0.09 |
| (20) | (16) | (12) | (9) | (20) | (15) | (12) | (10) |
| | | | | | | | |

| | PLASMA THEOPHYLLINE (mg/dl) | | | | | | |
|---|---|---|---|---|---|---|---|
| | | | | | | | |
| | 3.5±0.4 | 6.4±1.2 | 12.4^{a}±1.8 | | | | |
| | (13) | (12) | (9) | | | | |

| | | | | | | | |
|---|---|---|---|---|---|---|---|
| * Mean±SEM ( ) Patient number With respect to no improvement in smell function a p<0.05 b p<0.025 | | | | | | | |

At baseline, there were no significant differences between cGMP levels for the two groups of patients (Table XII). Mean values obtained in each patient group were consistent with values obtained in the total group of 312 patients prior to initiation of the treatment (see Example 1). After treatment with 200mg of theophylline, there was little difference in saliva cGMP levels between improved and unimproved patients or compared to pretreatment values. After treatment with 400mg of theophylline, saliva cGMP levels in the improved patients increased significantly to 275% over initial values, whereas there was little change among the unimproved patients. After treatment with 600 mg of theophylline, cGMP levels improved significantly to 338% over initial values, whereas there was no increase among the unimproved patients. After treatment with 400mg or 600mg of theophylline, cGMP mean levels in the improved patients were similar to those obtained in normal subjects.

**TABLE XII: RESULTS FOR PATIENTS WITH UNIMPROVED SMELL FUNCTION**

| BEFORE | THEOPHYLLINE TREATMENT | | | BEFORE | THEOPHYLLINE TREATMENT | | |
|---|---|---|---|---|---|---|---|
| TREATMENT | cAMP (pmol/ml) | | | TREATMENT | cGMP (pmol/ml) | | |
| (pmol/ml) | 200mg | 400mg | 600mg | (pmol/ml) | 200mg | 400mg | 600mg |
| 1.03±0.24* | 0.78±0.26 | 0.95±0.26 | 0.56±0.21 | 0.08±0.016 | 0.07±0.018 | 0.05±0.014 | 0.04±0.07 |
| (11) | (9) | (5) | (9) | (11) | (9) | (4) | (5) |
| | | | | | | | |

| | PLASMA THEOPHYLLINE (mg/dl) | | | | | | |
|---|---|---|---|---|---|---|---|
| | | | | | | | |
| | 3.7±1.0 | 8.6±1.4 | 8.7±0.6 | | | | |
| | (9) | (5) | (9) | | | | |

| | | | | | | | |
|---|---|---|---|---|---|---|---|
| * Mean±SEM ( ) Patient number | | | | | | | |

Comparison of blood plasma theophylline between the two groups indicated that there was no difference in mean values after treatment with either 200mg or 400mg of theophylline (Table XI). After treatment with 600mg, however, although plasma theophylline increased in both groups, compared to plasma levels seen following treatment with 200mg or 400mg of theophylline, mean theophylline was significantly elevated in the improved group compared to the unimproved group. (Tables XI and XII).

Classified by smell loss type, there were significant differences between the two groups (Table XIII). Among the improved patients, 95% of the patients exhibited Type II hyposmia, whereas, only one patient had Type I hyposmia. Among the unimproved patients, 57% of the patients exhibited Type I hyposmia, indicating that significantly more of the unimproved patients (with unchanged levels of cAMP and cGMP) had Type I hyposmia, the more severe type of smell loss (Table XII).

**TABLE XIII: COMPARISON OF CLINICAL CHARACTERISTICS BETWEEN IMPROVED AND UNIMPROVED PATIENTS**

| | | IMPROVED* | | | UNIMPROVED | | | |
|---|---|---|---|---|---|---|---|---|
| SMELL LOSS TYPE | | I | II | | I | | II | |
| PATIENT NUMBER | | 1 | 19^{a} | | 4 | | 7 | |
| | | | | | | | | |

| | IMPROVED* | | | | UNIMPROVED | | | |
|---|---|---|---|---|---|---|---|---|
| ETIOLOGY OF LOSS | AR | PIHH | HI | OTHER | AR | PIHH | HI | OTHER |
| PATIENT NUMBER | 8 | 6 | 3 | 3 | 8 | 0 | 3 | 0 |

| | | | | | | | | |
|---|---|---|---|---|---|---|---|---|
| * Parotid saliva cAMP and cGMP concentrations after 600mg theophylline were significantly higher in improved than in unimproved patients (see Tables I, II) Improved vs Unimproved by Smell Loss Type a X² = 5.2 (p<0.05) AR, allergic rhinitis PIHH, post influenza-like hyposmia and hypogeusia HI, head injury Other (drug reaction, idiopathic, post chemotherapy) | | | | | | | | |

Classified by diagnosis, the distribution of etiology of smell loss among the improved patients was similar to that previously reported in several publications. Among the unimproved patients, however, there were no patients who had PIHH or "other" causes of smell loss. Indeed, only two etiologies comprised the etiology of smell loss among unimproved patients, either allergic rhinitis or head injury (Table XII).

### DISCUSSION

The observed results were unexpected. These results, however, suggest that changes in cGMP may be more relevant to changes in smell function than measurement of cAMP since, among the improved patients, levels of cGMP increased into the normal range whereas for cAMP they increased, but not into the normal range.

These results also suggest that administration of theophylline at the same dose results in differences in smell improvement, in levels of saliva cAMP or cGMP and in serum theophylline levels in this group of patients. There are no prior studies that would suggest differential effects of drug response or biochemical changes related to theophylline intake. This type of change, however, has been observed previously with many drugs and is well known in several disease states. Indeed, drug resistance has been important to understanding differential drug affects and their influence on metabolic processes. Although the patient number in this study is relatively small, apparently this is the first report of drug resistance to oral administration of theophylline.

Patients with theophylline resistance appear to have specific clinical characteristics by which they can be identified prior to their treatment and development of this resistance. It was discovered that unimproved patients had head injury and allergic rhinitis. In addition these patients exhibit a preponderance of a severe type of smell loss (Type I hyposmia), one in which they cannot recognize the character of any odor.

The data obtained also suggest that this resistance is present irrespective of drug dose, although it is more obvious at higher doses of the drug. In addition, drug resistance effects appear to be more robust with respect to levels of saliva cGMP rather than to cAMP. Levels of salivary cGMP among the improved patients returned to levels previously observed in normal subjects. Saliva cAMP levels increased, significantly so at the highest dose of theophylline administered, but they did not increase to levels measured in normal subjects.

While levels of blood theophylline increased in both groups of patients as the dose of theophylline increased (200mg, 400mg, 600mg), levels of blood theophylline were significantly higher in the improved group compared to the unimproved group after 600mg of the drug. The blood theophylline level in the improved group was 12.4mg/dl whereas it was 8.7mg in the unimproved group (Tables XI and XII). While the level of theophylline in the improved patients is in the normal range for therapeutic effects of the drug (10-20mg/dl), the level in the unimproved group was not. Previous data suggest that patients that improve with theophylline exhibit plasma levels from 2-14mg/dl, so that it would not be reasonable to imply that these differences between these two groups are attributable only to the differences obtained in serum theophylline. While this is undoubtedly one factor related to drug resistance, as is well known from many other studies, this difference may not account for all the differences between these two groups.

The open label trial of theophylline in Example 1 demonstrates the usefulness of theophylline in restoring the sense of smell in patients with smell loss. The present studies indicate that multiple factors influence successful treatment of hyposmic patients and that a thorough work up is required if treatment is to be successful. These factors include testing as to the type and degree of smell loss, discovering the etiology of the patient's smell loss, analysis of theophylline plasma levels, and analysis of parotid gland secretion of cAMP and cGMP. In particular, parotid saliva cGMP level stands out as predictive of clinical response given the unexpected finding that cGMP levels correlate with recovery of the sense of smell. This observation allows for the development of a testing regiment to select for the appropriate therapy. A patient can be administered a standard challenge dose of theophylline or other PDE inhibitor and then the level of parotid saliva cGMP is determined. Patients that achieve a threshold level of cGMP following the challenge can then be prescribed the appropriate dose that will achieve the targeted steady state plasma level of the PDE inhibitor. Patients whom parotid saliva cGMP levels fail to achieve the threshold level can be challenged with a higher dose of the PDE inhibitor, have another PDE inhibitor or other active agent added to the dosage of the original PDE inhibitor, or switched to another PDE inhibitor as is clinically warranted. In this way, a physician can determine the optimum PDE inhibitor dosage for a patient without undertaking the long dose escalation titration required in the original study detailed in Example 1.

In some cases, a method is provided for screening patients for PDE inhibitor therapy for anosmia or hyposmia comprising: administering to a patient a challenge dose of a PDE inhibitor; determining the salivary level of cGMP; and comparing the patient's salivary cGMP level to a threshold value, wherein patients who have a cGMP salivary level equal to or greater than the threshold value are candidates for PDE inhibitor therapy to treat anosmia or hyposmia. In some cases, the challenge dose is 200 mg, 400 mg, 600 mg or 800 mg of theophylline. In some cases, the threshold value is at least 0.08, 0.10, 0.12, 0.14, 0.16, 0.18, 0.20, 0.22, 0.24, 0.26, or 0.28 pmol/ml.

In some cases, the threshold value is equal to or substantially similar to the mean cGMP value seen in normal individuals following the same challenge dose. In other cases, the threshold value is not less than 10%, 20%, 30%, 40%, or 50% of the mean salivary cGMP value seen in normal individuals following the same challenge dose.

### EXAMPLE 3

Hyposmic patients for whom the etiology of smell loss is not associated with head injury or allergic rhinitis are enrolled in an open label trial of inhalable theophylline. Patients are started at an initial dose of not more than 500 µg formulated as a liquid spray or dry powder to be delivered as a metered dose. Initially only local patients are enrolled so that quicker dose titration is achieved. Groups of 5 patients are started on not more than 500 µg of theophylline and continued for 1 month.

Patients are tested for improvement in smell acuity using the standard forced choice technique using four odors as described in Example 1. Additionally, subjective measurements of smell function are obtained using a scale from 0-100 as described in Example 1. Blood samples are also drawn to ascertain blood theophylline level to gauge its relationship to the expected recovery of smell.

Nasal administration of theophylline provides for high, initial concentrations of theophylline being deposited on the olfactory epithelium, thereby exposing olfactory neurons and their sensory cilia to higher concentrations of theophylline than are achieved through oral administration through the avoidance of first pass metabolism. Nasal administration provides for at least an equivalent recovery of smell acuity comparable to that achieved with orally administered theophylline while avoiding or at least reducing the side effects caused by higher theophylline plasma levels seen with oral administration.

### EXAMPLE 4

Hyposmic patients for whom the etiology of smell loss is not associated with head injury or allergic rhinitis are enrolled in an open label trial of an inhalable PDE1 inhibitor. More preferably, a PDE1C2 inhibitor such as eburnamenine-14-carboxylic acid ethyl ester (vinpocetine), 8-methoxymethyl-1-methyl-3-(2-methylpropyl) xanthine (8MM-IBMX), zaprinast (M&B 22948), 4-[3-(cyclopentyloxy)-4-methoxyphenyl]-2-pyrrolidinone (rolipram), 4-(3-butoxy-4-methoxybenzyl)-2-imidazolidinone (RO20-1724), 1,6-dihydro-2-methyl-6-oxo-(3,4'-bipyridine)-5-carbonitrile (milrinone), trequinsin (HL 725), and/or combinations thereof because of the high expression level of PDE1C2 in the olfactory epithelium. Patients are started at an initial dose of 500 µg formulated as a liquid spray or dry powder delivered as a metered dose. Initially only local patients are enrolled so that quicker dose titration is achieved. Groups of 5 patients are started on 500 µg of the PDE1 inhibitor or PDE1C2 inhibitor and continued for 1 month.

Patients are tested for improvement in smell acuity using the standard forced choice technique using four odors as described in Example 1. Additionally, subjective measurements of smell function are obtained using a scale from 0-100 as described in Example 1. Blood samples are also drawn to ascertain blood levels of the PDE1 inhibitor or PDE1C2 inhibitor and its relationship to the observed patient response.

Nasal administration of the PDE1 inhibitor or PDE1C2 inhibitor provides higher exposure of the PDE1 inhibitor or PDE1C2 inhibitor to nasal olfactory neurons than can be achieved through oral administration. This provides for at least an equivalent recovery of smell acuity comparable to that achieved with orally administered PDE1 inhibitor or PDE1C2 inhibitor, avoiding or reducing the side effects caused by higher PDE1 inhibitor or PDE1C2 inhibitor plasma levels seen with oral administration.

### REFERENCE EXAMPLE 5

For over 60 years, theophylline has been used as a bronchodilator in the treatment of asthma and COPD and remains one of the most widely prescribed drugs for the treatment of airway diseases worldwide. Theophylline directly relaxes human airways smooth muscle in vitro and, like beta₂-agonists, acts as a functional antagonist, preventing and reversing the effects of all bronchoconstrictor agonists. Bronchodilatation by theophylline is achieved through PDE inhibition, resulting in an increase in cAMP by inhibition of PDE3 and PDE4 and in cyclic guanosine 3',5'-- monophosphate by inhibition of PDES. The bronchodilator effect of theophylline in human airways is reduced by charybdotoxin, which selectively inhibits large conductance Ca²⁺ activated K⁺ channels (maxi-K channels), suggesting that theophylline opens these maxi-K channels via an increase in cAMP.

In asthma therapy, theophylline has an increasing acute bronchodilator response above plasma concentrations of 10 mg/L (55 µM), however, the upper recommended plasma concentration is 20 mg/L due to unacceptable side effects above this level including nausea and headaches. The therapeutic range for plasma concentrations is therefore established at 10 to 20 mg/L, and doses are adjusted in individual patients to achieve this range.

Theophylline has an additional effect on mucociliary clearance through a stimulatory effect on ciliary beat frequency and water transport across the airway epithelium. Relatively high doses of theophylline are needed, as this effect is likely to be due to an increase in cAMP as a result of PDE inhibition.

Theophylline also has anti-inflammatory effects in asthma. In allergen challenge studies in patients with asthma, intravenous theophylline inhibits the late response to allergen.. A similar finding with allergen challenge was reported after chronic oral treatment with theophylline. Oral theophylline also inhibits the late response to toluene diisocyanate in toluene diisocyanate-sensitive individuals with asthma. This is interpreted as an effect on the chronic inflammatory response, and this is supported by a reduced infiltration of eosinophils and CD4⁺ lymphocytes into the airways after allergen challenge subsequent to low doses of theophylline. In patients with nocturnal asthma, low-dose theophylline inhibits the influx of neutrophils and, to a lesser extent, eosinophils in the early morning. In patients with mild asthma, low doses of theophylline (mean plasma concentration ∼ 5 mg/L) reduce the numbers of eosinophils in bronchial biopsies, bronchoalveolar lavage, and induced sputum, whereas in severe asthma, withdrawal of theophylline results in increased numbers of activated CD4⁺ cells and eosinophils in bronchial biopsies.

In patients with COPD, theophylline reduces the total number and proportion of neutrophils in induced sputum, the concentration of interleukin-8, and neutrophil chemotactic responses, suggesting an anti-inflammatory effect. This is in sharp contrast to the lack of effect of high doses of inhaled corticosteroids in a similar population of patients.

These anti-inflammatory effects of theophylline in asthma and COPD are seen at concentrations that are usually less than 10 mg/L, which is below the dose where significant clinically useful bronchodilatation is evident.

Theophylline is a weak and nonselective inhibitor of PDEs. In vitro, theophylline relaxes airway smooth muscle by inhibition of PDE activity (PDE3, PDE4, and PDES), but relatively high concentrations are needed for maximal relaxation. The degree of PDE inhibition is very small at concentrations of theophylline that are therapeutically relevant with experiments with human lung extracts demonstrating only 5 to 10% inhibition of total PDE activity at therapeutic concentrations.

Inhalation therapy with nebulized solutions of theophylline and other methylxanthines was tried to widen the therapeutic index. Inhalation of methylxanthine derivatives produce an immediate increase in specific airway resistance (sGaw) that peaks in 5 minutes, but the effects were no more than half the response seen with a standard 200 µg dose of inhaled salbutamol, and dissipated by 30 minutes. Additionally, the nebulized solutions of methylxanthine derivatives have a disagreeable taste and produced a pronounced cough leading the researchers to conclude that inhalation of methylxanthines derivatives were unlikely to be of benefit in the treatment of asthma.

Applicants believe that this past failure with inhalable methylxanthine derivatives owes more to the formulation and method of delivery than to the inherent properties of the derivatives. To test this theory, asthmatic patients are enrolled in a dose escalating single blind study. On arrival to the clinic, subjects are allowed to rest for 20 minutes before baseline measurements of FEV1 (six recordings) and sGaw (five recordings) are made. Subjects receive a dry powder dispenser loaded with a dose of 2 mg theophylline or a dry powder carrier. Subjects are advised that some of the preparations may have a bitter taste, while others will not, but that this does not necessarily reflect the presence or degree of activity of the drug. After each inhalation, measurements of sGaw are made at 1, 3, 5, 10, 15, 20, 25, and 30 minutes. After 30 minutes, the sGaw measurement is still 20% above the baseline measurement, so additional sGaw measurements are taken every 15 minutes until baseline is reached at 1 hour.

On completion of the last recording, subjects inhale a 200 µg dose of salbutamol from a metered inhaler and a further measurement of sGaw is made after 15 minutes. Subjects are also monitored for coughing and are questioned about the taste of the drug. The increase in sGaw produced by the inhaled theophylline is comparable to that produced by salbutamol. Furthermore, the dry powder formulation is without the unpleasant taste or the induction of coughing associated with a liquid formulation delivered by a nebulizer.

### REFERENCE EXAMPLE 6

IBMX, (isobutylmethylxanthine), a non-specific PDE inhibitor possesses greater potency than theophylline (IC50 = 2-50 µM). Given IBMX structural similarity to theophylline, IBMX shares theophylline's bronchodilatation, anti-inflammatory, and ciliary beat frequency stimulatory effects but is expected to have a wider therapeutic index potentially allowing for the use of lower dosages, thereby reducing side effects and complaints over disagreeable taste when administered through a nebulizer.

Asthmatic patients are enrolled in a dose escalating single blind study. On arrival to the clinic, subjects are allowed to rest for 20 minutes before baseline measurements of FEV1 (six recordings) and sGaw (five recordings) are made. Subjects receive a dry powder dispenser loaded with a dose of 1 mg IBMX formulated as a dry powder or a dry powder carrier. Subjects are advised that some of the preparations may have a bitter taste, while others will not, but that this does not necessarily reflect the presence or degree of activity of the drug. After each inhalation, measurements of sGaw are made at 1, 3, 5, 10, 15, 20, 25, and 30 minutes. After 30 minutes, the sGaw measurement is still 20% above the baseline measurement, so additional sGaw measurements are taken every 15 minutes until baseline is reached at one and a half hours.

On completion of the last recording, subjects inhale a 200 µg dose of salbutamol from a metered inhaler and a further measurement of sGaw mad after 15 minutes. Subjects are also be monitored for coughing and are questioned about the taste of the drug. The increase in sGaw produced by the inhaled IBMX is comparable with that produced by salbutamol. Furthermore, the dry powder formulation is without the unpleasant taste or the induction of coughing associated with a liquid formulation delivered by a nebulizer.

### EXAMPLE 7

Ten patients with hyposmia were selected from among 400 patients with hyposmia to participate in a pilot study to determine safety and efficacy of intranasal theophylline for the treatment of hyposmia. These patients were previously treated with oral theophylline or were switched from oral theophylline to intranasal theophylline at the start of the study. Selection for inclusion in the intranasal study was based upon several criteria. 1) non-response to oral theophylline; 2) severe side effects with oral theophylline that prevented reaching a dose of sufficient strength to restore smell function; and/or 3) preference for intranasal medication over oral medication.
Through careful evaluation of plasma, saliva and nasal mucus theophylline levels in patients with hyposmia taking theophylline at doses of 200-800 mg daily, it was determined that a dose of 20 µg theophylline delivered intranasally to each naris (or 40 µg total) was sufficient to produce localized effects similar to those achieve with oral theophylline of 200-400 mg daily. Additionally, loco-regional administration of such a low dose would avoid producing the side effects seen with oral administration.

### Intranasal Preparation

A batch of theophylline for intranasal administration at a dose of 20 µg/0.4 ml of was prepared by dissolving 250 mg of methylparaben powder and 250 mg of propylparaben powder in 5 ml of propylene glycol. Next 50 mg of theophylline, anhydrous powder, was dissolved in a small amount of 0.9% sodium chloride. The dissolved parabens were added to the theophylline solution and mixed well. Sufficient 0.9% sodium chloride was added to the mixture to bring the total volume to 1000 ml. The solution was sterilized by filtering through a sterile 0.2 µm filter. 1 ml syringes were loaded with 0.4 ml of the sterile solution and then capped with a tamper evident cap. Representative samples were sent to an independent testing laboratory for pH, endotoxin, sterility, and fungal contamination testing in addition to the determination of the theophylline concentration. The test results demonstrated that the preparation had 20.716 µg of theophylline per 0.4 ml with a pH of 5.9. Further, endotoxin was below 1.0 EU/ml and the preparation was sterile and free of fungal contamination.

### Study Design

The purpose, risk and benefits of participating in the study were explained to each patient by the study's supervising physician. Prior to enrollment in the study, each patient read and signed an informed consent form. Next, each patient was instructed in the proper technique of intranasal administration.
Patients discontinued their use of oral theophylline either at time of entry into the study (eight patients) or four months prior to study entry (two patients).

Changes to smell function was determined at four specific periods:
Time 0 - at the start of the study.
Time 1 - one week after starting intranasal theophylline
Time 2 - two weeks after starting intranasal theophylline
Time 4 - four weeks after starting intranasal theophylline

After reaching Time 4, the study was discontinued and the patients were returned to their use of oral theophylline if indicated.

### Study Measurements

At each of the four time periods of the study the following battery of tests were performed and samples obtained.

### Objective Test Measurements

### Taste function tests

Taste function was measured by detection threshold (DT), recognition threshold (RT), magnitude estimation (ME) and hedonics (H) for four tastants (NaCl, sucrose, HCl, urea) by use of a standard three stimuli, forced choice staircase drop technique described in Example 1 and ref 53.

### Smell function tests

Smell function was measured for DT, RT, ME and H for four odorants (pyridine, nitrobenzene, thiophene, amyl acetate) by use of the standard three stimuli, forced choice staircase sniff technique described in Example 1 and in ref 53.

### Bodily fluids

Blood was obtained by venipuncture to collect plasma and red blood cells used to measure trace metals (Cu, Zn, Mg), various enzymes, theophylline and other chemical moieties.

Saliva was collected with a modified Lashley cup as described in Example 2 and used to measure trace metals (Cu, Zn, Mg), various enzymes (cAMP, cGMP, CA VI, etc.), theophylline and other chemical moieties.

Nasal mucus was collected by use of a standard technique and used to measure trace metals (Cu, Zn, Mg), various enzymes (cAMP, cGMP, CA VI, etc.), theophylline and other chemical moieties. Trace metals were measured by atomic absorption spectrophotometry (as previously described) using a dual beam Thermo-Jarrel Ash atomic absorption spectrophotometer. CA VI was measured by enzymatic analysis of activity of the enzyme by our modification of the method of Richli, et al. Cyclic nucleotides were measured by a sensitive 96 plate spectrophotometric sensitive ELISA assay provided by Applied Biosystems, Minneapolis, MN.

### Subjective Test Measurements

Subjective responses to treatment were obtained independent of any interaction with the clinical staff by using a scale of 0-100 to measure response of taste function and smell function to treatment with 0 indicating no response, 100 indicating return to normal function and intermediate numbers indicating an intermediate response.

### Treatment Technique

The patients inserted a plastic syringe containing 0.4 ml of fluid (20 µg theophylline) into each naris once a day. Previous investigation of the syringe injection technique for intranasal drug administration indicated that a volume of 0.4 ml delivered to each naris was sufficient to deliver the drug dose without having the liquid escape out of the nares or directly into the pharynx. The drug dose was delivered through a plastic nipple which fitted snugly onto the filled syringe which was attached directly to the nipple. One nipple was supplied for each set of two syringes in each application kit. The patient fitted the nipple on one syringe, placed the nipple securely into the lower naris and injected the contents of the syringe directly into the lower vault of one naris accompanied by a modest inhalation. This technique was then used for the second syringe used for the other naris. The patients were instructed to administer the drug either seated or standing with their head in an erect, vertical position.

Patients were supplied with 15 doses on each of two occasions with all used syringes returned to the clinic after use to insure proper and complete usage.

### RESULTS

Ten hyposmia patients were enrolled in the study; eight patients have completed all four phases of the study with the remaining two patients still in process. No side effects of intranasal use were observed, including, no nasal congestion, nasal pruritus, nasal discomfort, cough or unusual or bitter taste. No patient exhibited a further of loss of smell while in the study. This demonstrates that the patients that stopped their oral medication at the time of the start of the study, which ranged from 400-800 mg oral theophylline daily, received at least an equivalent localized dose of theophylline to the nasal olfactory epithelium as was achieved with their total oral dose.

Of the eight patients that completed the study, six reported subjective improvement in both taste and smell function (>10-20% over prior results with oral theophylline). The plasma theophylline levels for the eight patients was zero indicating that at most an undetectable fraction of intranasal theophylline was absorbed systemically. Furthermore, this data demonstrates that at the dosage level of 20 µg per naris, more theophylline is delivered locally to the nasal olfactory epithelium than is delivered by 200-800 mg of oral theophylline because the six patients that experienced increased smell acuity with the intranasal theophylline reported that their acuity diminished upon the resumption of their previous oral dosage of theophylline.

Overall, of the first eight patients, six (75%) exhibited subjective improvement in smell function in the study with the other two patients reporting maintenance of the improvement in smell acuity previously achieved with oral theophylline .

### DISCUSSION

Preliminary results of this trial of intranasal theophylline indicate that patients can administer the drug without difficulty. Further, no side effects were observed with the intranasal administration of theophylline. Additionally, all of the patients preferred intranasal administration over oral administration with six of the eight evaluable patients studied noting significant improvement in taste and smell function after use of intranasal over that achieved with oral theophylline. All ten patients reported no diminution of their smell function using intranasal administration demonstrating that they are receiving at least an equivalent dose of theophylline by intranasal administration. As the last two patients have not yet completed the study, the number of patients responding to the intranasal therapy may further increase. Furthermore, taste and smell acuity was stable or improved without producing measureable blood theophylline levels.

One of the patients that achieved an improved sense of smell did not wish to return the unused portion of the intranasal theophylline since the methodology also successfully enhanced her nasal breathing, nasal homeostasis and ability to sleep more soundly at night due to improvement in nasal function.

### EXAMPLE 8

**Objective:** To determine whether intranasal theophylline methylpropyl paraben can correct hyposmia and hypogeusia.

**Design:** We performed an open-label pilot study in patients with hyposmia and hypogeusia under the following 3 conditions: (1) before treatment, (2) after oral theophylline treatment, and (3) after intranasal theophylline treatment. Under each condition, we performed subjective evaluations of taste and smell functions, quantitative measurements of taste (gustometry) and smell (olfactometry), and measurements of serum theophylline level and body weight.

**Setting:** The Taste and Smell Clinic in Washington, DC.

**Patients:** Ten patients with hyposmia and hypogeusia clinically related to the effects of viral illness, allergic rhinitis, traumatic brain injury, congenital hyposmia, and other chronic disease processes were selected.

**Interventions:** Oral theophylline methylpropyl paraben, 200 to 800 mg/d for 2 to 12 months, was administered to each patient. This treatment was discontinued for 3 weeks to 4 months when intranasal theophylline methylpropyl paraben, 20 ug/d in each naris, was administered for 4 weeks.

**Main Outcome Measures:** At termination of each condition, taste and smell function was determined subjectively, by means of gustometry and olfactometry, with measurement of serum theophylline levels and body weight.

**Results:** Oral theophylline treatment improved taste and smell acuity in 6 patients after 2 to 12 months of treatment. Intranasal theophylline treatment improved taste and smell acuity in 8 patients after 4 weeks, with improvement greater than after oral administration. No adverse effects accompanied intranasal drug use. Body weight increased with each treatment but was greater after intranasal than after oral administration.

**Conclusions:** Intranasal theophylline treatment is safer and more effective in improving hyposmia and hypogeusia than oral theophylline treatment.

Loss of smell (hyposmia) and taste (hypogeusia) are common symptoms that affect many thousands of patients in the United States, as reported by several investigators. Effective treatment for these symptoms has been demonstrated only recently and has not been formally established.

Before effective treatment to correct loss of smell and taste can be established, a biochemical basis for the cause of these symptoms is necessary. To accomplish this, we determined that these symptoms are commonly caused by decreased secretion of several growth factors in the saliva and nasal mucus. The growth factors act on stem cells in taste buds and olfactory epithelial cells to generate the elegant repertoire of cellular components in these sensory organs. Growth factor stimulation of these sensory organs is thought to maintain normal taste and smell function. If these growth factors were diminished by any of several diseases and pathological conditions, then hyposmia and hypogeusia occur. These conditions and diseases include trace metal deficiencies; vitamin deficiencies; liver disease; diabetes mellitus's; other metabolic, otolaryngological, and neurodegenerative disorders, including multiple sclerosis, Parkinson disease, and Alzheimer disease; and other neurological disorders. Effective treatment to increase secretion of these growth factors is therefore necessary to improve hypogeusia and hyposmia and return taste and smell function to normal as demonstrated by several previous studies.

To understand more about these processes, a comprehensive study of many patients with loss of smell and taste determined that levels of the salivary and nasal mucus growth factors cyclic adenosine monophosphate (cAMP) and cyclic guanosine monophosphate (cGMP) were lower than in healthy subjects and were responsible for the onset of hyposmia and hypogeusia in many of these patients. Indeed, as hyposmia increased in severity, levels of these salivary and nasal mucus growth factors decreased in a consistent manner.

To increase salivary and nasal mucus cAMP and cGMP levels and thereby correct hypogeusia and hyposmia, we hypothesized that treatment with a phosphodiesterase inhibitor would be useful. To test this hypothesis, a previous study from our institution administered oral theophylline to 312 patients with hyposmia and hypogeusia in an open-label controlled clinical trial. Results of this study demonstrated that oral theophylline treatment successfully corrected hyposmia in more than 50% of these patients. Subsequent investigators have used other oral phosphodiesterase inhibitors to correct hyposmia. An open-label study also demonstrated that, as nasal mucus cAMP and cGMP levels increased, hyposmia was corrected, whereas in patients in whom these moieties did not increase, hyposmia was not corrected. These results suggested that some patients may be resistant to treatment with oral theophylline.

However, successful treatment with oral theophylline that increased nasal mucus levels of cAMP and cGMP required increased theophylline doses, sometimes prolonged treatment duration, and endurance of adverse effects, including restlessness, gastrointestinal tract discomfort, sleep difficulties, tachycardia, and other unwanted symptoms. Theophylline treatment also required regular determinations of blood theophylline levels to ensure adequate drug absorption and lack of toxic effects. These efforts limited use of this orally administered drug.

Because of these adverse effects, we wished to learn more about the pharmacology of theophylline administration. After treatment with oral theophylline, the drug was found in blood, nasal mucus, and saliva in a dose dependent manner. These results were consistent with improvement in smell function as demonstrated in patients with hyposmia in the prior clinical trial. Results of these studies and efforts to improve therapeutic efficacy and reduce adverse effects of oral theophylline administration made it logical to administer the drug intranasally. In this manner, the drug could affect olfactory receptors more directly without causing the systemic adverse effects associated with oral therapy.

To accomplish this, with assistance of an established medical device company, an intranasal delivery device was developed. With assistance of an established pharmaceutical company, the drug was packaged for sterile, intranasal delivery. Using this device, an open-label, single source, controlled pilot study in 10 patients with hyposmia and hypogeusia and with levels of parotid saliva and nasal mucus cAMP and cGMP below the reference range was performed to determine safety and to compare smell and taste responses after intranasal theophylline treatment, with patient responses before any treatment and after oral theophylline treatment.

### METHODS

### Patients

We selected 10 patients with hyposmia and hypogeusia from the 312 patients who participated in the prior open-label controlled clinical trial at The Taste and Smell Clinic for this pilot study. Each patient had undergone previous evaluation before any drug treatment, followed by treatment with oral theophylline. These patients had hyposmia and hypogeusia and exhibited levels of cAMP and cGMP lower than their respective reference ranges in the saliva and nasal mucus before theophylline treatment. These 10 patients were selected from the group undergoing previous evaluation and treatment for the intranasal trial because (1) their response to oral theophylline was subjectively submaximal; (2) they developed adverse effects after attempts to increase the drug dose to obtain a more maximal clinical response, thus limiting the administered drug dose; and (3) they resided in an area in close proximity to The Clinic, which made their frequent return visits to The Clinic more practical for any additional clinical trial.

These 10 patients included 7 men, aged 37 to 77 (mean [SEM] age, 64 [6]) years, and 3 women, aged 47 to 77 (62 [1 1]) years. Patients had 1 of the following 5 different clinical causes of sensory dysfunction: allergic rhinitis (n=3), postinfluenzalike hyposmia and hypogeusia (n =3), head injury (n=2), congenital hyposmia49 (n =1), and other disorders (n=1). Patients served as their own control throughout each condition of this study. The conditions included no treatment (before entry into the oral theophylline study), oral theophylline treatment, and intranasal theophylline treatment.

### Procedures

Subjective changes in smell and taste function under each study condition were measured by questionnaire before measurements of smell or taste function. Responses were graded on a scale from 0 to 100, with 0 reflecting no subjective response in overall sensory function; 100, return to normal sensory function; and values between 0 and 100 intermediate responses. Overall sensory function was defined as the ability to smell all odors and identify all tastants, although response intensity varied.

Smell and taste functions under each study condition were measured by standardized psychophysical sensory testing techniques. Measurements included determination of detection thresholds (DTs), recognition thresholds (RTs), magnitude estimation (ME), and hedonic response (HR) for 4 odors (*e*.*g*., pyridine [dead fish], nitrobenzene [bitter almond], thiophene [petroleum], and amyl acetate [banana oil]) (olfactometry) and for 4 tastants (*e*.*g*., sodium chloride [salt], sucrose [sweet], hydrochloride [sour], and urea [bitter]) (gustometry). These techniques have been previously described with olfactometry confirmed in a prior controlled double-blind clinical trial. Each measurement was performed independent of any prior knowledge of response.

Serum theophylline levels were measured by fluorescence polarization at each treatment condition. Body weight was measured with a calibrated clinical scale during each study condition and reported at the final measurement in each study condition.

### Study Protocol

The patients each underwent initial clinical evaluation at The Clinic to establish the cause, degree, and character of hyposmia and hypogeusia exhibited. Measurements in blood, urine, erythrocytes, saliva, and nasal mucus determined before their entry into the open trial of oral theophylline established the biochemical cause of their hyposmia and hypogeusia to be related to their levels of saliva and nasal mucus cAMP and cGMP being lower than the reference range. These 10 patients were then selected for this study on the basis of the laboratory and clinical criteria noted previously.

The 10 patients in this intranasal pilot study entered into the previous oral theophylline study according to a protocol approved by the institutional review board of the Georgetown University Medical Center. In this prior trial, oral theophylline was administered daily in 2 divided doses (at breakfast and lunch) of 200, 400, 600, or 800 mg for 2 to 12 months of treatment. Treatment was divided into 2- to 4-month periods, at which time patients returned to The Clinic for measurements of subjective sensory responses, olfactometry, gustometry, serum theophylline level, and body weight. If oral theophylline treatment failed to correct hyposmia at a given dose, the theophylline dose was increased by 200 mg, and the patient underwent reevaluation at 2- to 4-month intervals to a dose of 800 mg. As noted previously, study patients did not obtain a maximal clinical response to oral theophylline' or, while taking oral theophylline at a given dose, demonstrated some clinical improvement but experienced significant adverse effects that limited increasing the oral dose as necessary to achieve maximum clinical benefit. In the 10 patients selected for the intranasal pilot study, oral theophylline treatment was discontinued 3 weeks to 4 months before initiation of the intranasal drug trial. At that time, the mean (SEM) serum theophylline level was unmeasurable in any patient (0 [0] mg/L).

A pilot study of intranasal theophylline treatment was then initiated among these 10 patients. This trial was an investigator initiated phase 1, open-label, single-source, controlled pilot study. Intranasal drug therapy reflected a compassionate trial of a potentially more useful therapeutic method to improve hyposmia (and hypogeusia) than oral theophylline. Before the intranasal trial, risks and benefits were explained and the patients signed an informed consent.

The intranasal administration device was a calibrated 1 mL syringe fitted with a nozzle that fit comfortably into the anterior naris (Wolfe Tory Medical, Inc) and loaded under sterile conditions with 20 µg of theophylline methylpropyl paraben in a 0.4-mL saline solution (Foundation Care). Patients were instructed to direct the spray superiorly into the nasal cavity but not posteriorly into the nasopharynx. This technique was practiced before study initiation with sterile saline. Each patient used the technique easily and as demonstrated before drug administration.

Each patient delivered the theophylline dose in each naris once daily throughout the study. Patients underwent evaluation 1, 2, and 4 weeks during drug use with the same measurements used for the oral study.

Values for the oral trial were taken from the last measurements made before discontinuation of oral drug treatment and before initiation of the intranasal trial. This period varied from 2 to 12 months after oral treatment initiation and reflected the maximal improvement in sensory function each patient experienced. Values for the intranasal pilot study were taken from measurements obtained after completion of 4 weeks of intranasal treatment.

The mean and standard error of the mean for all values obtained at each study condition were compared. Differences were considered significant if P < .05 by the unpaired t test. Paired comparison tests were also used with differences considered significant if P < .05 by the t test.

### RESULTS

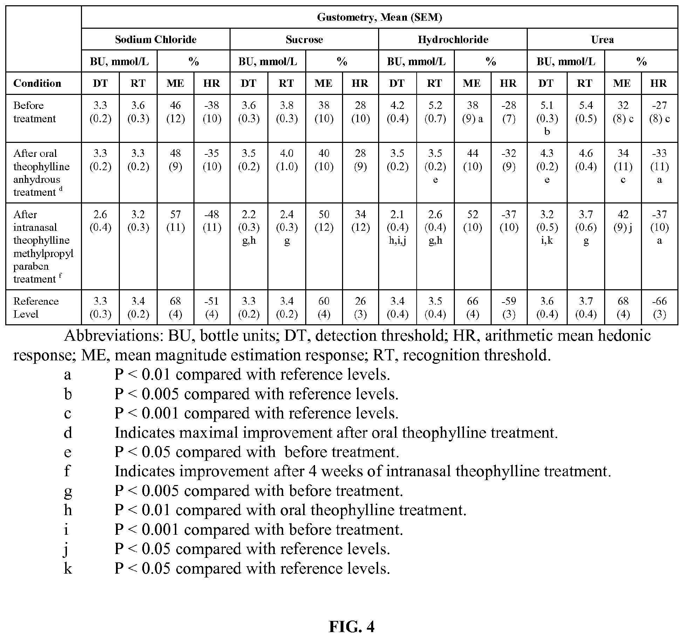

With oral theophylline administration, hypogeusia improved after 2 to 12 months of treatment, but hypogeusia improved further within 1 to 4 weeks of intranasal treatment (Figure 4). Results of gustometry after oral and intranasal theophylline are shown in Figure 4. Before treatment, DTs for sucrose, hydrochloride, and urea (less sensitive) and RTs for all tastants were elevated (less sensitive) above the reference levels. Magnitude estimations for all tastants were lower (less sensitive) than the reference level. Hedonic responses for sodium chloride, hydrochloride, and urea were lower (less unpleasant) than the reference levels. After oral theophylline treatment, DTs for sucrose and hydrochloride and RTs for sodium chloride, hydrochloride, and urea decreased (more sensitive). Magnitude estimations for all tastants increased (more sensitive) and HR for hydrochloride and urea increased (more unpleasant) as previously reported. After intranasal theophylline treatment, DTs and RTs for all tastants were lower (more sensitive) than before treatment or after oral theophylline treatment. Magnitude estimations for all tastants after intranasal theophylline treatment were higher (more intense) than before any treatment or after oral theophylline treatment. Hedonic responses for sodium chloride, hydrochloride, and urea were more negative (more unpleasant), whereas HRs for sucrose were more positive (more pleasant) than before any treatment or after oral theophylline treatment.

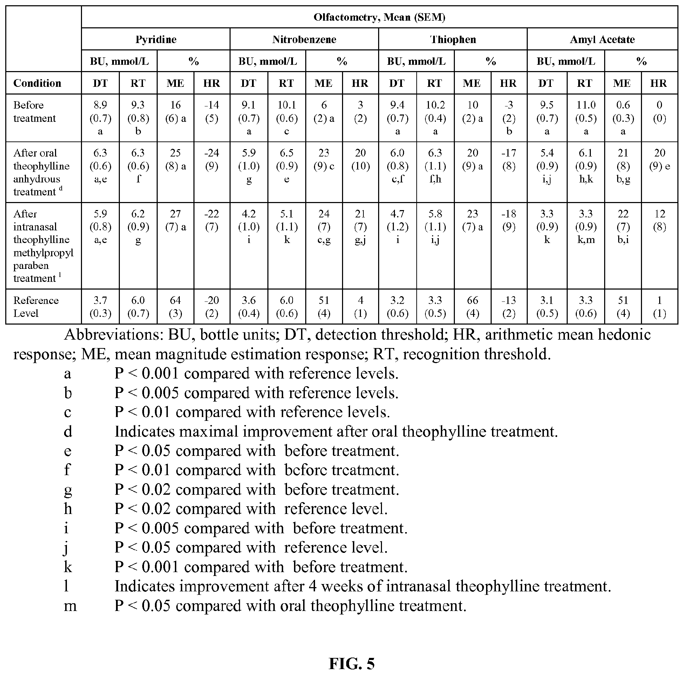

After oral theophylline treatment, hyposmia improved with 2 to 12 months of treatment but improved more with intranasal theophylline after 1 to 4 weeks of treatment (Figure 5). Olfactometry comparisons of oral and intranasal theophylline treatment are shown in Figure 5. Before treatment, compared with reference levels, DTs and RTs for all odorants were elevated (less sensitive); MEs for all odorants were decreased (less sensitive); HRs for pyridine and thiophene were decreased (less unpleasant); and HRs for nitrobenzene and amyl acetate were decreased (less pleasant). After oral theophylline treatment, DTs and RTs for all odorants were decreased (more sensitive), MEs for all odorants were increased (more sensitive), and HRs for all odorants increased (for pyridine and thiophene, more unpleasant; for nitrobenzene and amyl acetate, more pleasant) as previously reported. After intranasal theophylline treatment, DTs and RTs for each odor were lower (more sensitive) than before treatment or after oral theophylline treatment. Magnitude estimations for each odor were higher (more intense) than before treatment or after oral theophylline treatment. Hedonic responses to thiophene were more negative (more unpleasant) and to nitrobenzene were more positive (more pleasant) than before treatment or after oral theophylline treatment.

Smell and taste acuity were reported to be subjectively improved with oral theophylline treatment, but greater improvement was reported after 4 weeks of intranasal theophylline treatment. After oral theophylline treatment, 6 patients reported overall increased taste and smell function, whereas 4 reported no improvement. After intranasal theophylline treatment, 8 of the 10 patients reported overall improvement in taste and smell functions, whereas 2 reported no improvement. This response frequency is higher than that previously reported among patients with hyposmia and treated with oral theophylline, in which slightly more than 50% reported improvement.

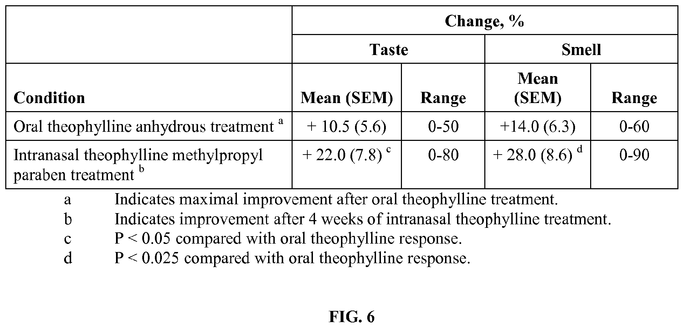

Taste and smell acuity were measured as subjectively improved after oral theophylline treatment, but this improvement was measured as increased after 4 weeks of intranasal theophylline treatment (Figure 6). After intranasal theophylline treatment, a 2-fold improvement was measured for taste and smell functions compared with oral treatment. Paired t test results showed that responses after intranasal theophylline were significantly greater than after oral theophylline treatment (taste, P < .05; smell, P < .025).

Body weight increased from pretreatment levels after oral theophylline treatment, but weight increased more after intranasal theophylline treatment. After oral theophylline treatment, mean (SEM) weight increased by 1.5 (0.4) kg from pretreatment values, whereas after intranasal theophylline treatment, weight increased by 2.5 (0.5) kg from pretreatment values. Patients related this change to increased food flavor obtained by improved smell function after intranasal theophylline treatment, which increased appetite and food enjoyment, resulting in subsequent weight gain. These changes were measured in each patient group despite no sensory improvement in 4 patients after oral theophylline treatment and none in 2 after intranasal theophylline treatment.

During oral theophylline treatment, the mean (SEM) serum theophylline level at the time of maximum improvement for these 10 patients was 6.4 (2.0) mg/L (to convert to micromoles per liter, multiply by 5.55). During intranasal theophylline treatment, the mean serum theophylline level was 0.0 (0.0). Discontinuation of intranasal theophylline treatment resulted in loss of smell and taste function within 1 week in 2 patients and after 6 weeks in 2. Four patients reported some persistence of improvement after 10 weeks.

### COMMENT

Results of this open-label, single-source, controlled pilot trial demonstrate that oral theophylline effectively improved hyposmia, as previously reported. The earliest this improvement was measured was after 2 months of treatment, but maximal improvement varied from 4 to 12 months. These results also demonstrate that oral theophylline was effective in improving hypogeusia in the same time frame as improvement in smell acuity.

In addition, intranasal theophylline was shown to be safe and more effective than oral theophylline in correcting hyposmia and hypogeusia. This improvement was measured as early as 1 week after starting treatment, but maximal improvement varied from 1 to 4 weeks.

Mechanisms by which intranasal theophylline was more effective than oral theophylline are not clearly defined. Intranasal drug delivery avoids the first-pass hepatic effect of an oral drug, bypassing initial cytochrome P450 metabolism and decreasing metabolism of the orally administered drug, thereby allowing for lower intranasally administered drug doses to be clinically efficacious. This lowering of the drug dose from a range of 200 to 800 mg orally to 40 µg intranasally was sufficient and specific enough to also avoid production of systemic adverse effects. This delivery mechanism may also avoid development of drug resistance that has occurred with oral theophylline. In addition, because more drug presumably contacts the olfactory epithelium with intranasal than with oral theophylline, direct nasal administration may activate more olfactory receptors than does oral administration.

However, additional actions of intranasal theophylline might enhance its therapeutic efficacy. Theophylline has been shown to inhibit symptoms of allergic rhinitis, which affected 3 patients in the intranasal trial. Many of the diseases and conditions that caused hyposmia and hypogeusia have an associated inflammatory component that may be suppressed by the anti-inflammatory effects of a phosphodiesterase inhibitor. In addition, drugs introduced intranasally can be delivered into the brain (1) directly by absorption through the cribriform plate along the olfactory bulb, (2) indirectly by absorption through blood-brain barrier receptors, or (3) through combinations of both methods. Although studies of theophylline absorption from nasal mucus into the brain have not been performed, studies of insulin, nerve growth factor, several neurotransmitters, and other moieties indicate uptake of these intranasally introduced moieties into the brain.

Whatever its mechanism of action, intranasal theophylline in this pilot study corrected hyposmia and hypogeusia relatively rapidly in 8 of 10 patients with several clinical diagnoses. The 2 patients who did not experience improvement were men, one with allergic rhinitis and the other with the effects of viral illness.

These results are consistent with prior studies in which several intranasal drugs were more effective than oral drugs. Inhaled adrenocorticosteroids were more effective with fewer adverse effects for asthma treatment than oral adrenocorticosteroids, and inhaled adrenocorticosteroids were more efficacious in asthma treatment than oral prednisolone acetate. Intranasal zolmitriptan achieved faster control of migraine headaches with fewer effects than the orally administered drug. Nasal administration of chicken type II collagen suppressed adjuvant arthritis in rats more effectively than oral administration.

However, intranasally administered drugs have also been reported to be only as effective as these same drugs given orally. Intranasal estradiol valerate was as effective as oral administration in alleviating postmenopausal symptoms but produced less frequent mastalgia and uterine bleeding. Intranasal desmopressin acetate was as effective for nocturnal enuresis as the oral drug but at a dose one-tenth that of the oral drug. Intranasal desmopressin is the preferred route for management of central diabetes insipidus.

At present, no generally clinically accepted method of treatment for hyposmia and hypogeusia exists. This pilot study suggests a simple, direct, and safe method to improve hyposmia and hypogeusia in a varied group of patients with both dysfunctions. In conclusion, intranasal theophylline treatment was safe and effective in improving hyposmia and hypogeusia and was more efficacious than oral theophylline treatment.

### Example 10

In this example, subject with taste and or smell loss are administered intranasal formulations containing theophylline and papaverine. The subjects are administered effective amounts once per day in each naris and experience an improvement in taste or smell function. The improvements in taste and smell function are evidenced by a decrease in the detection threshold for at least one odorant, a decrease in the recognition threshold for at least one odorant, and/or an increase in a magnitude estimation for at least one odorant. The benefits of the intranasal formulation are realized more rapidly than for orally administered combinations.

### Example 11

In this example, subject with taste and or smell loss are administered intranasal formulations containing theophylline and roflumilast. The subjects are administered effective amounts once per day in each naris and experience an improvement in taste or smell function. The improvements in taste and smell function are evidenced by a decrease in the detection threshold for at least one odorant, a decrease in the recognition threshold for at least one odorant, and/or an increase in a magnitude estimation for at least one odorant. The benefits of the intranasal formulation are realized more rapidly than for orally administered combinations.

### Example 12

In this example, subject with taste and or smell loss are administered intranasal formulations containing theophylline and cilostazol. The subjects are administered effective amounts once per day in each naris and experience an improvement in taste or smell function. The improvements in taste and smell function are evidenced by a decrease in the detection threshold for at least one odorant, a decrease in the recognition threshold for at least one odorant, and/or an increase in a magnitude estimation for at least one odorant. The benefits of the intranasal formulation are realized more rapidly than for orally administered combinations.

### Example 13

In this example, subject with taste and or smell loss are administered intranasal formulations containing theophylline, cilostizol, and roflumilast. The subjects are administered effective amounts once per day in each naris and experience an improvement in taste or smell function. The improvements in taste and smell function are evidenced by a decrease in the detection threshold for at least one odorant, a decrease in the recognition threshold for at least one odorant, and/or an increase in a magnitude estimation for at least one odorant. The benefits of the intranasal formulation are realized more rapidly than for orally administered combinations.

The following claims define the scope of the invention.

## Claims

1. A multi-dose nasal spray device for delivery of theophylline to a subject's nasal epithelium for use in the treatment of a taste or smell disorder in said subject, that delivers a dosage unit in a plume upon actuation, wherein the dosage unit comprises from 10 µg to 200 µg of theophylline in a pharmaceutically acceptable carrier comprising one or more excipients; and wherein the plume is **characterized by**:
(a) a total volume of from 25 µL to 200 µL; and
(b) a droplet size distribution **characterized by** :
(i) less than 5% of the droplets in the plume having a size of less than 10 µm,
(ii) a D₁₀ of greater than 12.5 µm, wherein 10% of the droplets in the plume have a size less than the D₁₀,
(iii) a D₅₀ of from 30 to 70 µm, wherein 50% of the droplets in the plume have a size less than the D₅₀,
(iv) a D₉₀ of less than 200 µm, wherein 90% of the droplets in the plume have a size less than the D₉₀, and
(v) a span of from 1 to 6, wherein the span is calculated according to: (D₉₀ - D₁₀)/D₅₀.

2. The device for delivery of theophylline for use according to claim 1, wherein the droplet size distribution is **characterized by** the D₁₀ that is from 12.5 µm to 30 µm.

3. The device for delivery of theophylline for use according to any one of claims 1-2, wherein the droplet size distribution is **characterized by** the D₁₀ that is from 15 µm to 25 µm.

4. The device for delivery of theophylline for use according to any one of claims 1-3, wherein the droplet size distribution is **characterized by** the D₅₀ that is from 30 µm to 50 µm.

5. The device for delivery of theophylline for use according to any one of claims 1-4, wherein the droplet size distribution is **characterized by** the D₅₀ that is from 30 µm to 40 µm.

6. The device for delivery of theophylline for use according to any one of claims 1-5, wherein the droplet size distribution is **characterized by** the D₉₀ that is less than 100 µm.

7. The device for delivery of theophylline for use according to any one of claims 1-6, wherein the droplet size distribution is **characterized by** the D₉₀ that is from 75 µm to 100 µm.

8. The device for delivery of theophylline for use according to any one of claims 1-7, wherein the droplet size distribution is **characterized by** the span that is from 1 to 2.

9. The device for delivery of theophylline for use according to any one of claims 1-8, wherein the plume is further **characterized by** having an ovality of about 1.

10. The device for delivery of theophylline for use according to any one of claims 1-9, wherein the plume is further **characterized by** having a geometry of from 45° to 75°.

11. The device for delivery of theophylline for use according to any one of claims 1-10, for use in the treatment of the smell disorder.

12. The device for delivery of theophylline for use according to any one of claims 1-10, for use in the treatment of the taste disorder.

13. The device for delivery of theophylline for use according to any one of claims 1-11, wherein the theophylline is administered once or twice daily to each naris.

14. The device for delivery of theophylline for use according to any one of claims 11-13, wherein the subject experiences a clinically detectable improvement in taste or smell function within 1-4 weeks of starting treatment.

15. The device for delivery of theophylline for use according to any one of claims 11-13, wherein the subject experiences a decrease in a detection threshold (DT) score, a decrease in a recognition threshold (RT) score, an increase in a magnitude estimation (ME) score, or a change in a hedonic (H) score as measured with a forced-choice, three-stimuli, stepwise-staircase technique using one or more odorants or tastant testing compounds after administering the theophylline to the subject.

## Patentansprüche

1. Eine Mehrfachdosis-Nasensprayvorrichtung für die Lieferung eines Theophyllin an das nasale Epithel eines Subjekts für die Verwendung bei der Behandlung einer Geschmacks- oder Geruchsstörung bei diesem Subjekt, die bei Betätigung eine Dosiereinheit in einer Wolke liefert, wobei die Dosiereinheit 10 µg bis 200 µg Theophyllin in einem pharmazeutisch zulässigen Trägerstoff umfasst, der wiederum einen oder mehrere Hilfsstoffe beinhaltet; und wobei die Wolke **gekennzeichnet ist durch**:
(a) ein Gesamtvolumen von 25 µl bis 200 µl; und
(b) eine Tröpfchengrößenverteilung **gekennzeichnet durch** Folgendes:
(i) weniger als 5 % der Tröpfchen in der Wolke haben eine Größe unter 10 µm,
(ii) ein D₁₀ von über 12,5 µm, wobei 10 % der Tröpfchen in der Wolke eine Größe unter der von D₁₀ haben,
(iii) ein D₅₀ von 30 bis 70 µm, wobei 50 % der Tröpfchen in der Wolke eine Größe unter der von D₅₀ haben,
(iv) ein D₉₀ von weniger als 200 µm, wobei 90 % der Tröpfchen in der Wolke eine Größe unter der von D₉₀ haben, und
(v) eine Spanne von 1 bis 6, wobei die Spanne berechnet wird gemäß: (D₉₀-D₁₀)/D₅₀.

2. Die Vorrichtung für die Lieferung von Theophyllin zur Verwendung nach Anspruch 1, wobei die Tröpfchengrößenverteilung **gekennzeichnet ist durch** D₁₀, das zwischen 12,5 µm und 30 µm liegt.

3. Die Vorrichtung für die Lieferung von Theophyllin zur Verwendung nach einem der Ansprüche 1-2, wobei die Tröpfchengrößenverteilung **gekennzeichnet ist durch** D₁₀, das zwischen 15 µm und 25 µm liegt.

4. Die Vorrichtung für die Lieferung von Theophyllin zur Verwendung nach einem der Ansprüche 1-3, wobei die Tröpfchengrößenverteilung **gekennzeichnet ist durch** D₅₀, das zwischen 30 µm und 50 µm liegt.

5. Die Vorrichtung für die Lieferung von Theophyllin zur Verwendung nach einem der Ansprüche 1-4, wobei die Tröpfchengrößenverteilung **gekennzeichnet ist durch** D₅₀, das zwischen 30 µm und 40 µm liegt.

6. Die Vorrichtung für die Lieferung von Theophyllin zur Verwendung nach einem der Ansprüche 1-5, wobei die Tröpfchengrößenverteilung **gekennzeichnet ist durch** D₉₀, das unter 100 µm liegt

7. Die Vorrichtung für die Lieferung von Theophyllin zur Verwendung nach einem der Ansprüche 1-6, wobei die Tröpfchengrößenverteilung **gekennzeichnet ist durch** D₉₀, das zwischen 75 µm und 100 µm liegt.

8. Die Vorrichtung für die Lieferung von Theophyllin zur Verwendung nach einem der Ansprüche 1-7, wobei die Tröpfchengrößenverteilung **gekennzeichnet ist durch** die Spanne, die zwischen 1 und 2 liegt.

9. Die Vorrichtung für die Lieferung von Theophyllin zur Verwendung nach einem der Ansprüche 1-8, wobei die Wolke ferner **dadurch gekennzeichnet ist, dass** sie eine Ovalität von 1 hat.

10. Die Vorrichtung für die Lieferung von Theophyllin zur Verwendung nach einem der Ansprüche 1-9, wobei die Wolke ferner **dadurch gekennzeichnet ist, dass** sie eine Geometrie zwischen 45° und 75° umfasst.

11. Die Vorrichtung für die Lieferung von Theophyllin zur Verwendung nach einem der Ansprüche 1-10, für die Verwendung bei der Behandlung der Geruchsstörung.

12. Die Vorrichtung für die Lieferung von Theophyllin zur Verwendung nach einem der Ansprüche 1-10, für die Verwendung bei der Behandlung der Geschmacksstörung.

13. Die Vorrichtung für die Lieferung von Theophyllin zur Verwendung nach einem der Ansprüche 1-11, wobei das Theophyllin einmal oder zweimal täglich in jede Naris verabreicht wird.

14. Die Vorrichtung für die Lieferung von Theophyllin zur Verwendung nach einem der Ansprüche 11-13, wobei das Subjekt eine klinisch erkennbare Verbesserung in der Geschmacks- oder Geruchsfunktion innerhalb von 1 - 4 Wochen nach Behandlungsbeginn erfährt.

15. Die Vorrichtung für die Lieferung von Theophyllin für die Verwendung nach einem der Ansprüche 11-13, wobei das Subjekt einen Rückgang eines Wahrnehmungsschwellenwerts (DT), einen Rückgang eines Erkennungsschwellenwerts (RT), eine Zunahme eines Größen-Schätzwerts (ME) oder eine Änderung eines hedonischen Werts (H) erfährt, die anhand eines schrittweisen Forced-Choice-Staircase-Verfahrens mit drei Stimuli, unter Verwendung eines oder mehrerer Geruchsstoff- oder Geschmacksstoff-Testpräparate, nach der Verabreichung des Theophyllin an ein Subjekt, gemessen werden.

## Revendications

1. Dispositif de pulvérisation nasale à multiples doses, destiné à l'administration de la théophylline dans l'épithélium nasal d'un sujet, destinée à être utilisée pour le traitement d'un trouble du goût ou de l'odorat chez un sujet, qui administre une unité posologique dans un panache après son déclenchement, dans lequel l'unité posologique comprend de 10 µg à 200 µg de théophylline dans un support pharmaceutiquement acceptable comprenant un ou plusieurs excipients ; dans lequel le panache est **caractérisé par** :
(a) un volume total de 25 µl à 200 µl ; et
(b) une distribution de la taille des gouttelettes **caractérisée par** :
(i) moins de 5 % des gouttelettes dans le panache présentant une taille inférieure à 10 µm,
(ii) une valeur D₁₀ supérieure à 12,5 µm, 10 % des gouttelettes dans le panache présentant une taille inférieure à la valeur D₁₀,
(iii) une valeur D₅₀ de 30 à 70 µm, 50 % des gouttelettes dans le panache présentant une taille inférieure à la valeur D₅₀,
(iv) une valeur D₉₀ inférieure à 200 µm, 90 % des gouttelettes dans le panache présentant une taille inférieure à la valeur D₉₀ et
(v) une portée de 1 à 6, la portée étant calculée selon : (D₉₀ - D₁₀)/D₅₀.

2. Dispositif destiné à l'administration de la théophylline destinée à être utilisée selon la revendication 1, dans lequel la distribution de la taille des gouttelettes est **caractérisée par** la valeur D₁₀ qui va de 12,5 µm à 30 µm.

3. Dispositif destiné à l'administration de la théophylline destinée à être utilisée selon l'une quelconque des revendications 1 à 2, dans lequel la distribution de la taille des gouttelettes est **caractérisée par** la valeur D₁₀ qui va de 15 µm à 25 µm.

4. Dispositif destiné à l'administration de la théophylline destinée à être utilisée selon l'une quelconque des revendications 1 à 3, dans lequel la distribution de la taille des gouttelettes est **caractérisée par** la valeur D₅₀ qui va de 30 µm à 50 µm.

5. Dispositif destiné à l'administration de la théophylline destinée à être utilisée selon l'une quelconque des revendications 1 à 4, dans lequel la distribution de la taille des gouttelettes est **caractérisée par** la valeur D₅₀ qui va de 30 µm à 40 µm.

6. Dispositif destiné à l'administration de la théophylline destinée à être utilisée selon l'une quelconque des revendications 1 à 5, dans lequel la distribution de la taille des gouttelettes est **caractérisée par** la valeur D₉₀ qui est inférieure à 100 µm

7. Dispositif destiné à l'administration de la théophylline destinée à être utilisée selon l'une quelconque des revendications 1 à 6, dans lequel la distribution de la taille des gouttelettes est **caractérisée par** la valeur D₉₀ qui va de 75 µm à 100 µm.

8. Dispositif destiné à l'administration de la théophylline destinée à être utilisée selon l'une quelconque des revendications 1 à 7, dans lequel la distribution de la taille des gouttelettes est **caractérisée par** la portée qui va de 1 à 2.

9. Dispositif destiné à l'administration de la théophylline destinée à être utilisée selon l'une quelconque des revendications 1 à 8, dans lequel le panache est **caractérisé en outre par** la présentation d'une ovalisation d'environ 1.

10. Dispositif destiné à l'administration de la théophylline destinée à être utilisée selon l'une quelconque des revendications 1 à 9, dans lequel le panache est **caractérisé en outre par** la présentation d'une géométrie de 45° à 75°.

11. Dispositif destiné à l'administration de la théophylline destinée à être utilisée selon l'une quelconque des revendications 1 à 10, destiné à être utilisé dans le traitement du trouble de l'odorat.

12. Dispositif destiné à l'administration de la théophylline destinée à être utilisée selon l'une quelconque des revendications 1 à 10, destiné à être utilisé dans le traitement du trouble du goût.

13. Dispositif destiné à l'administration de la théophylline destinée à être utilisée selon l'une quelconque des revendications 1 à 11, dans lequel la théophylline est administrée une ou deux fois par jour à chaque narine.

14. Dispositif destiné à l'administration de la théophylline destinée à être utilisée selon l'une quelconque des revendications 11 à 13, dans lequel le sujet éprouve une amélioration de la fonction du goût ou de l'odorat cliniquement perceptible dans les 1 à 4 semaines suivant le début du traitement.

15. Dispositif destiné à l'administration de la théophylline destinée à être utilisée selon l'une quelconque des revendications 11 à 13, dans lequel le sujet éprouve une diminution dans un score du seuil de détection (DT), une diminution dans un score du seuil de reconnaissance (RT), une augmentation dans un score d'estimation de l'amplitude (ME), ou un changement dans un score hédoniste (H) tel que mesuré par une technique d'escalier progressif à choix forcé, à trois stimuli à l'aide d'un ou plusieurs composés d'essais de substances odorantes ou d'exhausteur de goût après l'administration de la théophylline chez le sujet.
